(19) Europäisches Patentamt
European Patent Office
Office européen des brevets

(11) Veröffentlichungsnummer: **0 418 695 A1**

# (12) EUROPÄISCHE PATENTANMELDUNG

(21) Anmeldenummer: **90117389.8**

(22) Anmeldetag: **10.09.90**

(51) Int. Cl.[5]: **C12N 15/82**, C12N 15/56, C12N 1/21, C12N 15/10, A01H 5/00, C12Q 1/68, A01N 65/00

(30) Priorität: **13.09.89 CH 3334/89**

(43) Veröffentlichungstag der Anmeldung: **27.03.91 Patentblatt 91/13**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Meins, Jr., Frederick, Prof. Dr.**
**Leimgrubenweg 52**
**CH-4125 Riehen(CH)**
Erfinder: **Shinshi, Hideaki, Dr.**
**Ottominami 2-9-24**
**Tsuchiura City, Ibaraki 300(JP)**
Erfinder: **Neuhaus, Jean-Marc, Dr.**
**Göschenenstrasse 28**
**CH-4054 Basel(CH)**

(74) Vertreter: **Zumstein, Fritz, Dr. et al**
**Bräuhausstrasse 4**
**W-8000 München 2(DE)**

## (54) Regulatorische DNA-Sequenz.

(57) Die vorliegende Erfindung betrifft eine neue, im wesentlichen reine, regulatorische DNA Sequenz, welche aus der 5′-Region eines pflanzlichen Chitinasegens erhältlich ist und in operabler Verknüpfung mit einer beliebigen exprimierbaren DNA zu stark erhöhten Expressionsraten in transformierten Pflanzen führt.

Ein weiterer Gegenstand der vorliegenden Erfindung betrifft rekombinante DNA Moleküle, welche die erfindungsgemässe DNA Sequenz in operabler Verknüpfung mit einer exprimierbaren DNA enthalten sowie die davon abgeleiteten Vektoren, desweiteren Wirtszellen und/oder Wirtsorganismen, einschliesslich transgener Pflanzen, die besagte rekombinante DNA bzw. die davon abgeleiteten Vektoren enthalten.

Ebenso umfasst von der vorliegenden Erfindung ist ein neues Chitinasegen, das aus *Nicotiana tabacum* L. c.v. Havana 425 Pflanzen erhältlich ist und das in seiner 5′-nicht-translatierten Region die erfindungsgemässe, regulatorische DNA Sequenz enthält.

EP 0 418 695 A1

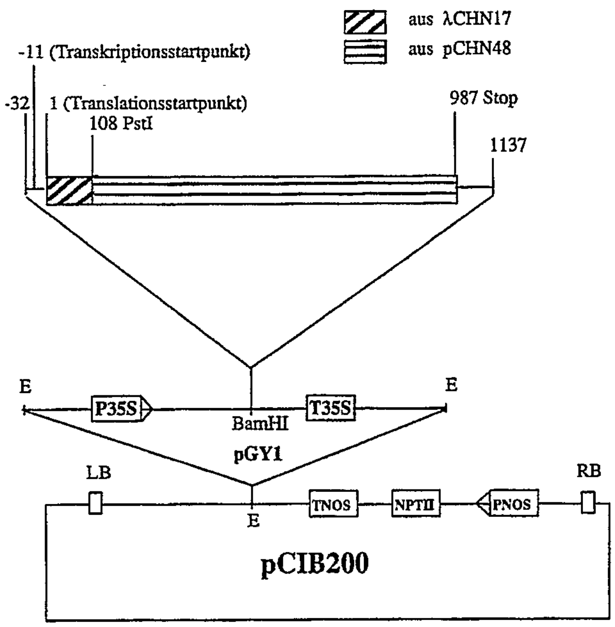


## 5'-Ende der relevanten Sequenz

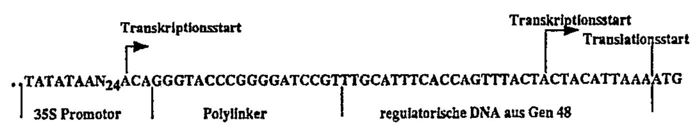


**FIG. 1 Genkarte von pSCH12**

# REGULATORISCHE DNA-SEQUENZ

Die vorliegende Erfindung betrifft eine neue, im Wesentlichen reine, regulatorische DNA Sequenz, welche aus der 5′-Region eines pflanzlichen Chitinasegens erhältlich ist und in operabler Verknüpfung mit einer beliebigen exprimierbaren DNA zu stark erhöhten Expressionsraten in transformierten Pflanzen führt. Ein weiterer Gegenstand der vorliegenden Erfindung betrifft rekombinante DNA Moleküle, welche die erfindungsgemässe DNA Sequenz in operabler Verknüpfung mit einer exprimierbaren DNA enthalten sowie die davon abgeleiteten Vektoren. Ebenso umfasst sind Wirtszellen und/oder Wirtsorganismen, einschliesslich transgener Pflanzen, die besagte rekombinante DNA bzw. die davon abgeleiteten Vektoren enthalten.

Ebenso umfasst von der vorliegenden Erfindung ist ein neues Chitinasegen, das aus *Nicotiana tabacum* L. c.v. Havana 425 Pflanzen erhältlich ist und das in seiner 5′-nicht-translatierten Region die erfindungsgemässe, regulatorische DNA Sequenz enthält.

Die Verfahren zur Herstellung der erfindungsgemässen DNA Sequenz sowie der diese DNA Sequenz enthaltenden rekombinanten DNA Moleküle und Vektoren bilden einen weiteren Bestandteil der vorliegenden Erfindung, ebenso deren Verwendung zur Herstellung transgener Pflanzen.

Einer der wesentlichen limitierenden Faktoren bei der Herstellung transgener Pflanzen mit neuen und nützlichen Eigenschaften ist die z.T. nur geringe Expressionsrate der eingeschleusten Fremdgene. Bedingt durch diese geringe Expression des Fremdgens und die damit verbundenen geringen Proteinkonzentrationen in der Pflanze treten die gewünschten Effekte oft in nur ungenügendem Masse oder aber überhaupt nicht zu Tage.

Bei den intensiv untersuchten bakteriellen Expressionssystemen stehen mittlerweile verschiedene Möglichkeiten zur Verfügung um die Transkriptionsrate eines einklonierten Strukturgens zu erhöhen. So kann beispielsweise ein Strukturgen hinter einen starken Promotor eines Replikons inseriert werden, was in vielen Fällen zu einer Steigerung der Genexpressionsrate führt. Weiterhin besteht die prinzipielle Möglichkeit die gesamte Operon-Einheit eines Gens, dh sowohl Promotor als auch Operator und Terminator, in ein Multicopy-Plasmid einzuspleissen. Aufgrund der gesteigerten Genexpression bzw. im zweiten Fall aufgrund der erhöhten Gendosis lässt sich somit unter Umständen eine Steigerung der Proteinkonzentration in der Zelle erreichen.

In den weit weniger gut untersuchten pflanzlichen Systemen dagegen, bei denen die Mechanismen der Regulation von Transkription und Translation noch weitgehend im Dunkeln liegen, sind die zur Zeit verfügbaren Möglichkeiten zur Erhöhung der Transkriptionsrate noch sehr begrenzt und wo vorhanden oft nur mit geringem Erfolg anwendbar.

Eine der Hauptaufgaben der vorliegenden Erfindung bestand somit darin, eine regulatorische Sequenz zur Verfügung zu stellen, die in der Lage ist, die Expression eines in operabler Weise assoziierten Fremdgens oder sonstiger nützlicher DNA-Sequenzen, wie z.B. 'anti-sense' DNA, in einer Weise zu erhöhen, dass die gewünschten phänotypischen Effekte, wie z.B. die Ausbildung einer Resistenz gegenüber bestimmten Pathogenen oder Chemikalien, deutlich sichtbar werden und die betreffenden Pflanzen somit gegenüber diesen schädlichen Einflüssen effektiv geschützt sind.

Als ein weiterer Anwendungsbereich der erfindungsgemässen, regulatorischen DNA Sequenz sei an dieser Stelle die Steigerung der Syntheseleistungen transgener Pflanzen genannt, die für die Produktion nützlicher und wertvoller Verbindungen vorgesehen sind.

Die zuvor formulierte Aufgabe konnten jetzt im Rahmen dieser Erfindung durch die Anwendung zum Teil bekannter Massnahmen überraschenderweise gelöst werden.

Im einzelnen betrifft die vorliegende Erfindung eine regulatorische DNA Sequenz, die aus der 5′- nicht-translatierten Region eines pflanzlichen Chitinasegens, vorzugsweise aus der 5′- nicht-translatierten Region eines Chitinasegens von Tabak, erhältlich ist und die, in operabler Verknüpfung mit geeigneten, in pflanzlichen Zellen aktiven Expressionssignalen sowie einem Strukturgen oder sonstigen exprimierbaren DNA-Sequenzen, zu einer signifikanten Erhöhung der Expressionsrate des jeweils operabel assoziierten Strukturgens oder sonstiger operabel assoziierter DNA Sequenzen, wie z.B. einer 'anti-sense' DNA, in pflanzlichem Material führt.

Insbesondere betrifft die vorliegende Erfindung eine regulatorische DNA Sequenz, vorzugsweise in im wesentlichen reiner Form, die aus der 5′- nicht-translatierten Region eines basischen Chitinasegens von *Nicotiana tabacum* L. cv. Havana 425 Pflanzen erhältlich ist und die im wesentlichen die folgende DNA-Sequenz aufweist:

5′- **TTGCATTTCACCAGTTTACTACTACATTAAA** -3′

Ebenso umfasst sind alle Derivate dieser DNA-Sequenz, die sich auf die Mutation einer oder mehrerer Basen stützen, die aber obiger Sequenz nach wie vor im wesentlichen homolog sind und noch die erfindungswesentlichen Eigenschaften aufweisen.

Unter einer Mutation sollen dabei sowohl die Deletion bzw. Insertion von einer oder mehreren Basen als auch insbesondere der Austausch von einer oder mehreren Basen verstanden werden.

Im Rahmen dieser Erfindung ist eine DNA Sequenz einer zweiten DNA Sequenz dann im wesentlichen homolog, wenn wenigstens 60%, vorzugsweise wenigstens 80% und ganz besonders bevorzugt wenigstens 90% der aktiven Abschnitte der DNA Sequenzen einander homolog sind.

Ebenso umfasst von der vorliegenden Erfindung sind Fragmente oder Teilsequenzen, die aus der oben näher bezeichneten DNA Sequenz oder aus Derivaten dieser DNA Sequenz erhältlich sind und die noch die spezifischen Eigenschaften der Ausgangssequenz aufweisen.

Besonders bevorzugt ist eine Teilsequenz, welche die folgende DNA-Sequenz aufweist:

**5′ ACTACTACATTAAA-3′**

einschliesslich aller Derivate dieser DNA-Sequenz, die sich auf die Mutation einer oder mehrerer Basen stützen, die aber obiger Sequenz nach wie vor im wesentlichen homolog sind und die noch die spezifischen, regulatorischen, expressionssteigernden Eigenschaften der Ausgangssequenz aufweisen.

Die vorliegende Erfindung betrifft weiterhin rekombinante DNA Moleküle, die eine chimäre genetische Konstruktion enthalten, worin die erfindungsgemässe, regulatorische DNA-Sequenz in operabler Verknüpfung mit einer exprimierbaren DNA sowie weiteren, in pflanzlichen Zellen aktiven Expressionssignalen, wie Promotor- und Terminationssequenzen, vorliegt, und die bei Transformation in einen pflanzlichen Wirt zu einer signifikanten Erhöhung der Expressionsrate der operabel assoziierten, exprimierbaren DNA führen.

Besonders bevorzugt ist eine rekominantes DNA Molekül, das die obige erfindungsgemässe DNA-Sequenz in operabler Verknüpfung mit einem 35S Promotor aus Cauliflower Mosaik Virus (CaMV) sowie einem Chitinase- oder Glucanase-Strukturgen enthält.

Weiterhin umfasst von der vorliegeden Erfindung sind Klonierungs-, Transformations- und Expressionsvektoren, die besagtes erfindungsgemässes rekombinantens DNA-Molekül enthalten sowie die mit besagten Vektoren transformierten Wirtsorganismen.

Einen weiteren Gegenstand dieser Erfindung bilden sog. 'Shuttle'-Vektoren, die besagtes erfindungsgemässes rekombinantens DNA-Molekül enthalten und die in der Lage sind sowohl in *E. coli* als auch in *A. tumefaciens* stabil zu replizieren.

Unter den Wirtsorganismen sind insbesondere pflanzliche Wirte ausgewählt aus der Gruppe bestehend aus pflanzlichen Protoplasten, Zellen, Kalli, Geweben, Organen, Zygoten, Embryonen, Pollen und/oder Samen bevorzugt sowie insbesondere auch ganze, vorzugsweise fertile Pflanzen, die mit besagter rekombinanter DNA transformiert sind. Ganze Pflanzen können dabei entweder direkt als solche mil dem erfindungsgemässen, rekombinanten DNA Molekül transformiert oder aber aus zuvor transformierten Protoplasten, Zellen und/oder Geweben *via* Regeneration gewonnen werden.

Ganz besonders bevorzugt sind transgene Pflanzen, insbesondere aber transgene fertile Pflanzen, die eine gegenüber dem Wildtyp signifikant erhöhten Chitinase-und/oder Glucanasegehalt aufweisen.

Ebenso umfasst von der vorliegender Erfindung ist jegliches Vermehrungsgut einer transgenen Pflanze, wobei besagte transgene Pflanze entweder durch direkte Transformation mit dem erfindungsgemässen, rekombinanten DNA Molekül entstanden sein kann oder aber aus zuvor transformierten Protoplasten, Zellen, Kalli, Geweben, Organen, Zygoten, Embryonen, Pollen und/oder Samen *via* Regeneration gewonnen wird, ohne jedoch darauf beschränkt zu sein.

Unter Vermehrungsgut soll im Rahmen dieser Erfindung jegliches pflanzliche Material verstanden werden, das sich sexuell oder asexuell oder aber *in vitro* oder *in vivo* vermehren lässt, wobei Protoplasten, Zellen, Kalli, Gewebe, Organe, Eizellen, Zygoten, Embryonen, Pollen oder Samen, die von einer erfindungsgemässen transgenen Pflanze erhalten werden können, bevorzugt sind. Einen weiteren Gegenstand dieser Erfindung bildet die Nachkommenschaft besagter Pflanzen, sowie Mutanten und Varianten davon, einschliesslich derjenigen, die sich von Pflanzen ableiten, welche durch somatische Zellfusion, genetische Modifikation oder Mutantenselektion erhalten werden.

Weitere Gegenstände der vorliegenden Erfindung bilden Verfahren

(a) zur Herstellung der erfindungsgemässen regulatorischen DNA-Sequenz;

(b) zur Herstellung der erfindungsgemässen rekombinanten DNA-Moleküle, welche die obige erfindungsgemässe DNA-Sequenz in operabler Verknüpfung mit einem geeigneten Promotor sowie einem Strukturgen enthalten;

(c) zur Herstellung von Klonierungs-, Transformations-und/oder Expressionsvektoren, welche besagtes erfindungsgemässes rekombinantes DNA-Molekül enthalten;

(d) zur Herstellung transformierter Wirtsorganismen, insbesondere pflanzlicher Wirte ausgewählt aus der Gruppe bestehend aus pflanzlichen Protoplasten, Zellen, Kalli, Geweben, Organen, Zygoten, Embryonen, Pollen und/oder samen sowie insbesondere auch ganze, vorzugsweise fertile Pflanzen;

(e) zur Herstellung von Vermehrungsgut ausgehend von transformiertem Pflanzenmaterial, insbesondere aber zur Herstellung von sexueller und asexueller Nachkommenschaft; und

(f) zum Schutz von Pflanzen vor chitinhaltigen Pathogenen, insbesondere aber vor pathogenen Pilzen und Insekten.

Das Verfahren zur Herstellung der erfindungsgemässen regulatorischen DNA-Sequenz ist im wesentlichen dadurch gekennzeichnet, dass man besagte Sequenz oder eines ihrer Derivate

(a) aus der 5′-nicht-translatierten Region eines pflanzlichen Chitinasegens, vorzugsweise aus der 5′-nicht-translatierten Region eines basischen Chitinasegens aus Tabak, unter Anwendung bekannter Massnahmen isoliert; oder

(b) mit Hilfe chemischer Verfahren synthetisiert.

Im einzelnen umfasst Verfahrensschritt (a) die folgenden, an sich bekannten Massnahmen:

(a) Extrahieren und Reinigen von genomischer DNA aus Geweben, die in der Lage sind Chitinase zu exprimieren;

(b) Zerlegen der extrahierten und gereinigten DNA Präparationen in Fragmente einer für die nachfolgende Insertion in einen Klonierungs-Vektor geeigneten Grösse;

(c) Einklonieren der fragmentierten DNA in einen Klonierungsvektor und Herstellen einer Genbibliothek;

(d) Auslesen von Klonen, die das Chitinasegen oder Teile davon enthalten mit Hilfe von Probenmolekülen;

(e) Isolierung derjenigen Klone, die mit dem Probenmolekül ein starkes Hybridisierungssignal zeigen;

(f) Charakterisierung der in (e) isolierten Klone mit Hilfe biochemischer Verfahren; und

(g) Identifizierung und Isolierung der für die Expressionssteigerung verantwortlichen regulatorischen DNA Sequenz.

Das Verfahren zur Herstellung der erfindungsgemässen rekombinanten DNA Moleküle ist imwesentlichen dadurch gekennzeichnet, dass man eine der erfindungsgemässen, regulatorischen DNA Sequenzen unmittelbar vor dem Startkodon einer gewünschten exprimierbaren DNA inseriert und diese Konstruktion mit in pflanzlichen Zellen aktiven Expressionssignalen operabel verknüpft.

Besonders bevorzugt im Rahmen der vorliegenden Erfindung ist dabei die Herstellung eines rekombinanten DNA Moleküls, worin ein Strukturgen, das den transformierten pflanzlichen Zellen sowie den daraus sich entwickelnden Geweben und insbesondere aber den Pflanzen einen Schutzeffekt gegenüber Pathogenen, Chemikalien sowie nachteiligen Umwelteinflüssen verleiht, mit der erfindungsgemässen, regulatorischen DNA Sequenz assoziierten ist.

Das Verfahren zur Herstellung eines Expressionsvektors, welcher eine in pflanzlichen Zellen exprimierbare DNA in operabler Verknüpfung mit einer der erfindungsgemässen, regulatorischen DNA Sequenzen enthält, ist im wesentlichen dadurch gekennzeichnet, dass man

(a) besagte regulatorische DNA Sequenz unmittelbar vor dem Startkodon einer gewünschten exprimierbaren DNA inseriert; und

(b) dieses Konstrukt in einen bekarnten Pflanzenexpressionsvektor, der gegebenenfalls ein für die Transformantenselektion geeignetes Markergen enthalten kann, zwischen in pflanzlichen Zellen aktiven Expressionssignalen operabel einspleisst.

Das Verfahren zur Herstellung von pflanzlichem Material ausgewählt aus der Gruppe bestehend aus Protoplasten, Zellen, Kalli, Geweben, Organen, Samen, Embryonen, Pollen, Eizellen, Zygoten, etc., ist im wesentlichen dadurch gekennzeichnet, dass man besagtes pflanzliches Material unter Verwendung an sich bekannter Verfahren mit einem der zuvor charakterisierten, rekombinaten DNA Moleküle transformiert.

Das Verfahren zur Herstellung einer transgenen Pflanze mit gegenüber dem Wildtyp erhöhtem Proteingehalt in den transformierten Zellen und/oder Geweben ist im wesentlichen dadurch gekennzeichnet, dass man besagte Pflanze unter Verwendung an sich bekannter Verfahren mit einem der zuvor charakterisierten, rekombinaten DNA Moleküle transformiert.

Besonders bevorzugt ist ein Verfahren zur Herstellung einer transgenen Pflanze mit einem gegenüber dem Wildtyp signifikant erhöhten Glucanase- oder Chitinasegehalt.

Die Transformation des pflanzlichen Material wird vorzugsweise mit Hilfe bekannter Verfahren, ausgewählt aus der Gruppe bestehend aus Polyethylenglykolbehandlung, Hitzeschockbehandlung und Elektroporation pflanzlicher Protoplasten; Mikroinjektion in pflanzliche Protoplasten, Zellen oder Embryonen; Beschuss von Zellen oder Geweben mit Mikroprojktilen; *Agrobacterium*-vermittelter Transformation pflanzlicher Protoplasten, Zellen, Gewebe oder ganzer Pflanzen; sowie CaMV-vermittelter Transformation pflanzlicher Protoplasten, Zellen, Gewebe oder ganzer Pflanzen, durchgeführt.

Das Verfahren zur Herstellung von transgenem Saatgut ist im wesentlichen dadurch gekennzeichnet, dass man die zuvor näher charakterisierten, transgenen Pflarzen sexuell vermehrt und die sich entwickelnden Samen, die noch das eingeschleuste genetische Material enthalten, mit Hilfe bekannter Verfahren gewinnt.

Das Verfahren zur Herstellung transformierter, lebensfähiger Teile von transgenen Pflanzen, ist im wesentlichen dadurch gekennzeichnet, dass man ausgehend von den zuvor näher charakterisierten, transgenen Pflarzen mit Hilfe bekannter Methoden transformierte Pflanzenteile, ausgewählt aus der Gruppe bestehend aus Protoplasten, Zellen, Zellklonen, Zellagglomeraten, Kallus- und/oder Gewebekulturen, Samen, Pollen, Eizellen, Zygoten oder Embryonen gewinnt und diejenigen selektioniert, die noch das eingeschleuste genetische Material enthalten.

Das Verfahren zur Herstellung von Kreuzungs- und Fusionsprodukten unter Verwendung des zuvor näher charakterisierten, transgenen Pflanzenmaterials ist im wesentlichen dadurch gekennzeichnet, dass man besagtes Pflanzenmaterial untereinander oder mit Fremdmaterial kreuzt oder fusioniert und diejenigen Kreuzungs- und Fusionsprodukte selektioniert, die noch das eingeschleuste genetische Material enthalten und nach wie vor die erfindungswesentlichen Eigenschaften aufweisen.

Das Verfahren zur Herstellung von Varianten und Mutanten von den zuvor näher charakterisierten, transgenen Pflanzen ist im wesentlichen dadurch gekennzeichnet, dass man transgene Pflanzen oder lebensfähige Teile davon sowie deren sexuelle und asexuelle Nachkommenschaft mit Hilfe bekannter Methoden mutiert und anschliessend diejenigen Individuen selektioniert, die noch das eingeschleuste genetische Material enthalten und die erfindugswesentlichen Eigenschaften aufweisen.

Das Verfahren zur Herstellung transgener Pflanzenteile ausgewählt aus der Gruppe bestehend aus. Blüten, Stengeln, Früchten, Blättern und Wurzeln, ist im wesentlichen dadurch gekennzeichnet, dass man besagte Pflanzenteile isoliert und diejenigen selektioniert, die noch in einem Grossteil ihrer Zellen das eingeschleuste genetische Material enthalten

Ebenfalls umfasst von dieser Erfindung ist ein Verfahren zum Schutz von Pflanzen vor chitinhaltigen Pathogenen, das dadurch gekennzeichnet ist, dass man besagte Pflanzen mit einem rekombinanten DNA Molekül transformiert, das eine chimäre genetische Konstruktion enthält, worin die erfindungsgemässe, regulatorische DNA-Sequenz mit einen Strukturgen das Chitinase kodiert sowie mit weiteren, in pflanzlichen Zellen aktiven Expressionssignalen operabel verknüpft ist, und dass man das eingeschleuste Chitinasegen in einer Menge exprimiert, die ausreicht, die Pathogene abzutöten oder aber diese unter Kontrolle zu halten.

Desweiteren betrifft die vorliegende Erfindung ein Verfahren zur Bekämpfung chitinhaltiger Pathogene, das dadurch gekennzeichnet ist, dass man besagte Pathogene mit einer transgenen Pflanze oder Teilen einer solchen Pflanze in Kontakt bringt, die mit einem rekombinanten DNA Molekül transformiert sind, das eine chimäre genetische Konstruktion enthält, worin die erfindungsgemässe regulatorische DNA Sequenz mit einem Strukturgen das Chitinase kodiert sowie mit weiteren, in pflanzlichen Zellen aktiven Expressionssignalen operabel verknüpft ist, sodass das eingeschleuste Chitinasegen in einer transgenen Pflanze oder in Teilen einer solchen Pflanze in einer Menge exprimiert wird, die ausreicht, die Pathogene abzutöten oder aber diese unter Kontrolle zu halten.

Ein weiterer Gegenstand der vorliegenden Erfindung betrifft die Verwendung der erfindungsgemässen, regulatorischen DNA Sequenz zu Steigerung der Genexpression in pflanzlichem Material sowie zum Aufspüren homologer DNA Sequenzen gleicher Funktion.

## Figuren

Bevor die vorliegende Erfindung in der Folge im Detail beschrieben wird, soll an dieser Stelle kurz auf die Abbildungen eingegangen werden.

Figur 1 (Fig.1): zeigt eine Genkarte von PSCH12, welche die charakteristischen Regionen dieses Plasmids wiedergibt sowie deren Herkunft. (P35S→CaMV 35S Promotor; T35S→CaMV Terminationssequenz; TNOS→Terminationssequenz des Neomycinphosphotransferasegens; PNOS→Promotor des Neomycinphosphotransferasegens; LB, RB→Linke bzw. rechte Bordersequenz aus dem Ti-Plasmid von *Agrobacterium tumefaciens*.

Der relevante Teil der 5'- Region der chimären, Chitinasekodierenden Genkonstruktion ist im Detail anhand der Nukleotidsequenz angegeben.

Figur 2 (Fig.2): zeigt eine Restriktionskarte von PCIB200

Figur 3 (Fig.3): gibt einen schematischen Überblick über die Konstruktion von Plasmid PSCH12. Details sind in Beispiel 8 enthalten.

Figur 4 (Fig.4): gibt einen schematischen Überblick über die Konstruktion von Plasmid pSGL7. Details sind in Beispiel 9 enthalten.

Figur 5 (Fig.5): gibt einen Überblick über die Chitinasekonzentration in den Blättern transformierter *N. sylvestris* S1 pflanzen ($Km^R/Km^R$) im Vergleich zu den Kontrollen ($Km^S/Km^S$). Die Nummerierung der Blätter erfolgt ansteigend von unten nach oben.

SEQ ID NO 1 [Sequenzprotokoll, Abschnitt IX]: zeigt die Nukleotidsequenz von Gen 48, einem basischen Chitinasegen aus *N. tabacum*.

## Definitionen

In der folgenden Beschreibung werden eine Reihe von Ausdrücken verwendet, die in der rekombinanten DNA Technologie, sowie in der Pflanzengenetik gebräuchlich sind. Um ein klares und einheitliches Verständnis der Beschreibung und der Ansprüche sowie des Umfangs, der den besagten Ausdrücken zukommen soll, zu gewährleisten, werden die folgenden Definitionen aufgestellt.

Pflanzliches Material: In Kultur oder als solches lebensfähige pflanzliche Teile wie Protoplasten, Zellen, Kallus, Gewebe, Embryonen, Pflanzenorgane, Knospen, Samen, u.a. sowie ganze Pflanzen.

Pflanzenzelle: Strukturelle und physiologische Einheit der Pflanze, bestehend aus einem Protoplasten und einer Zellwand.

Protoplast: Aus Pflanzenzellen oder -geweben isolierte zellwandlose "nackte" Pflanzenzelle mit der Potenz zu einem Zellklon oder einer ganzen Pflanze zu zegenerieren.

Pflanzengewebe: Gruppe von Pflanzenzellen, die in Form einer strukturellen und funktionellen Eirheit organisiert sind.

Pflanzenorgan: Strukturelle und funktionelle Einheit aus mehreren Geweben, wie beispielsweise Wurzel, Stamm, Blatt oder Embryo.

Phänotypisches Merkmal: Erkenn- oder zumindest nachweisbares Merkmal, das auf der Expression eines oder mehrerer Gene beruht. Ein phänotypisches Merkmal kann jedoch auch durch Unterdrückung einer Genexpression, z.B. beim Einsatz von 'anti-sense' DNA, zum Ausdruck kommen.

Heterologe(s) Gen(e) oder DNA: Eine DNA Sequenz, die ein spezifisches Produkt oder Produkte kodiert oder eine biologische Funktion erfüllt und die von einer anderen Spezies stammt als derjenigen, in welche das besagte Gen eingeschleust wird; besagte DNA Sequenz wird auch als Fremdgen oder Fremd-DNA bezeichnet.

Homologe(s) Gen(e) oder DNA: Eine DNA Sequenz, die ein spezifisches Produkt oder Produkte kodiert oder eine biologische Funktion erfüllt und die aus der gleichen Spezies stammt, in welche das besagte Gen eingeschleust wird.

Synthetische(s) Gen(e) oder DNA: Eine DNA-Sequenz, die ein spezifisches Produkt oder Produkte kodiert oder eine biologische Funktion erfüllt und die auf synthetischem Wege hergestellt sind.

Pflanzen-Promotor: Eine Kontrollsequenz der DNA Expression, die die Transkription jeder beliebigen homologen oder heterologen DNA Gensequenz in einer Pflanze gewährleistet, sofern besagte Gensequenz in operabler Weise mit einem solchen Promotor verknüpft ist.

Terminations-Sequenz: DNA-Sequenz am Ende einer Transkriptions-Einheit, die das Ende des Transkriptionsvorganges signalisiert.

Ueberproduzierender Pflanzen-Promotor (OPP): Pflanzen-Promotor, der in der Lage ist, in einer transgenen Pflanzenzelle die Expression einer jeden in operabler Weise verknüpften funktionalen Gensequenz(en) in einem Ausmass (ge messen in Form der RNA oder der Polypeptidmenge) zu bewirken, das deutlich höher liegt, als dies natürlicherweise in Wirtszellen, die nicht mit besagtem OPP transformiert sind, beobachtet wird.

3′/5′ nicht-translatierte Region: Stromabwärts/stromaufwärts der kodierenden Region gelegene DNA-Abschnitte, die zwar in mRNA transkribiert, nicht aber in ein Polypeptid übersetzt werden. Diese Region enthält regulatorische Sequenzen, wie z.B. die Ribosomenbindungsstelle (5′) oder das Polyadeny lierungssignal (3′).

DNA-Klonierungsvektor: Klonierungs-Vehikel, wie z.B. ein Plasmid oder ein Bakteriophage, das alle Signal-Sequenzen enthält, die für die Klonierung einer inserierten DNA in einer geeigneten Wirtszelle notwendig sind.

DNA-Expressions-Vektor: Klonierungs-Vehikel, wie z.B. ein Plasmid oder ein Bakteriophage, das alle Signal-Sequenzen enthält, die für die Expression einer inserierten DNA in einer geeigneten Wirtszelle notwendig sind.

DNA-Transfer-Vektor: Transfer-Vehikel, wie z.B. ein Ti-Plasmid oder ein Virus, das die Einschleusung von genetischem Material in eine geeignete Wirtszelle ermöglicht.

Mutanten, Varianten transgener Pflanzen: Spontan oder aber artifiziell, unter Anwendung bekannter Verfahrensmassnahmen, wie z.B. UV-Behandlung, Behandlung mit mutagenen Agentien etc. entstandener Abkömmling einer transgenen Pflanze, der noch die erfindungswesentlichen Merkmale und Eigenschaften der Ausgangspflanze aufweist.

Im wesentliche reine DNA Sequenz: Eine DNA Sequenz, die in im wesentlichen reiner Form aus einer natürlichen oder nichtnatürlichen Quelle isoliert wird. Eine solche Sequenz kann in einem natürlichen System vorliegen, wie beispielweise in Bakterien, Viren oder in pflanzlichen oder tierischen Zellen oder sie kann alternativ dazu in Form synthetischer DNA oder von cDNA zur Verfügung gestellt werden.
Im wesentlichen reine DNA wird in der Regel in Form eines Vektors isoliert, welcher die besagte DNA als Insert enthält. Im wesentlichen rein heisst, dass andere DNA Sequenzen nur in vernachlässigbarem Umfang vorliegen und beispielsweise weniger als 5%, vorzugsweise weniger als 1% und ganz besonders bevorzugt weniger als 0.1% ausmachen. Solche Sequenzen und die diese Sequenzen enthaltenden Vektoren liegen im allgemeinen in wässriger Lösung vor und zwar in einer Pufferlösung oder in einem der üblicherweise verwendeten Kulturmedien.

Der Hauptgegenstand der vorliegenden Erfindung betrifft eine neue, regulatorische, aus der 5'- nicht-translatierten Region eines pflanzlichen Chitinasegens erhältliche DNA Sequenz, die, sofern sie mit exprimierbarer DNA sowie in pflanzlichen Zellen aktiven Expressionssignalen operabel verknüpft ist, bei Transformation in einen pflanzlichen Wirt zu einer signifikanten Erhöhung der Expressionsrate der jeweils operabel assoziierten exprimierbaren DNA führt.

Insbesondere betrifft die vorliegende Erfindung eine neue, im wesentlichen reine, regulatorische DNA Sequenz, welche aus der 5'- nicht-translatierten Region eines pflanzlichen Chitinasegens erhältlich ist und in operabler Verknüpfung mit einem beliebigen Strukturgen zu einer starken Erhöhung der Expressionsraten des assoziierten Strukturgens in trans formiertem pflanzlichemmaterial führt.

Besonders bevorzugt im Rahmen dieser Erfindung ist eine DNA-Sequenz, die in der 5'- nicht-translatierten Region eines basischen Chitinasegens aus Tabak vorliegt und aus dieser mit Hilfe an sich bekannter Techniken gewonnen werden kann.

Ebenfalls umfasst von der vorliegenden Erfindung ist ein basisches Chitinasegen aus Tabak, welches als Ausgangsmaterial für die Gewinnung der erfindungsgemässen, regulatorischen DNA Sequenz fungieren kann und in seiner 5'-nicht-translatierten Region die folgenden strukturellen Besonderheiten aufweist:

(a) einen Transkriptionsstart innerhalb der Sequenz CTACT in Position 1967 bis 1971;
(b) ein erstes mögliches Startkodon in Position 1980, 11 Bp stromabwärts der Transkriptionsstartstelle;
(c) eine TAAATA-Sequenz ('TATA box') stromaufwärts der Transkriptionsstartstelle in Position -28 bis -23 der 5'-flankierenden Region;
(d) eine CCAATT-Sequenz in Position -114;
(e) eine 6 Bp umfassende unvollkommen invertierte Wiederholungssequenz (GCCGAATTCGAGC) in Position -140;
(f) eine 10 Bp umfassende vollkommene Wiederholungssequenz (ATGTCCAAAC) in Position -152 und -228;
(g) eine 20 Bp umfassende unvollkommene direkte Wiederholungssequenz (TTTTAACTAAATCTATGTCC) in Position -166 und -569;
(h) eine 25 Bp umfassende unvollkommene direkte Wiederholungssequenz (CAACTTTCAAAAATTATTTTTTAAA) in Position -191 und -217;
(i) ein 20 Bp umfassendes Palindrom (TAAAATATGA|TCATGTTTTA) in Position -289;
(j) eine 11 Bp umfassende vollkommene direkte Wiederholungssequenz (TAAGAGCCGCC) in Position -435 und -480;
(k) eine 15 Bp umfassende unvollkommene direkte Wiederholungssequenz (TAAAATACACGTCGA) in Position -514 und -644;
(l) zwei AATAAA Sequenzen in Position 52 und 120, stomabwärts der Translationsstop Sequenz TAA in der 3'-flankierenden Region.

Besonders bevorzugt im Rahmen dieser Erfindung ist ein basisches Chitinasegen, das aus *Nicotiana tabacum* L. c.v. Havana 425 Pflanzen erhältlich ist und die in Figur 6 wiedergegebe DNA sequenz aufweist.

Bei der Isolierung eines geeigneten Chitinasegens geht man vorzugsweise von genomischen oder cDNA Genbibliotheken aus, die nach gängigen, dem Fachman auf diesem Gebiet bestens bekannten Routineverfahren hergestellt werden können. Die grundsätzlichen Verfahren zur Herstellung von genomischen oder cDNA Genbibliotheken sind beispielsweise bei Maniatis *et al* (1982) im Detail beschrieben, während die Umsetzung und Anwendung dieser Verfahren auf pflanzliche Systeme z.B. bei Mohnen (1979) wiedergegeben ist.

Genomische DNA sowie cDNA kann auf unterschiedliche Weise gewonnen werden. Genomische DNA beispielsweise kann mit Hilfe bekannter Verfahren aus geeigneten Zellen extrahiert und gereinigt werden. Bei der Herstellung von cDNA geht man in der Regel von mRNA aus, die aus ausgesuchten Zellen oder Geweben isoliert werden kann, insbesondere aber aus Zellen oder Geweben die bekanntermassen hohe Chitinkonzentrationen aufweisen. Die isolierte mRNA kann dann im Rahmen einer reversen Transkription als Matritze für die Herstellung einer korrespondierenden cDNA verwendet werden. Besonders bevorzugt als Ausgangsmaterial für die Herstellung von cDNA sind pflanzliche Zellen oder Gewebe oder sonstiges geeignetes Pflanzenmaterial, das zuvor mit Hilfe geeigneter Massnahmen zur Produktion hoher Chitinasespiegel angeregt wurde. Dies lässt sich beispielsweise dadruch erreichen, dass man kultivierte Zellen oder Gewebe oder sonstiges geeignetes Pflanzenmaterial auf ein Hormon-freies Medium überimpft und dort über einen geeigneten Zeitraum hinweg kultiviert, der für die Induktion hoher Chitinasespiegel ausreichend ist. Besonders bevorzugt im Rahmen dieser Erfindung ist ein Basismedium, das die von Linsmaier und Skoog (1965) vorgeschlagene Salz- und Thiamin-HCl Konzentration aufweist (LS-Medium).

Die Verfahren zur Isolierung von Poly ($A^+$) RNA und zur Herstellung von cDNA sind dem Fachmann bekannt und werden in der Folge im Rahmen der Ausführungsbeispiele im Detail beschrieben.

Die extrahierten und gereinigten DNA Präparationen werden anschliessend für die nachfolgende Klonierung in Fragmente zerlegt. Die Fragmentierung der zu klonierenden genomischen DNA oder der cDNA auf eine für die Insertion in einen Klonierungs-Vektor geeignete Grösse kann entweder durch mechanisches Scheren oder aber vorzugsweise durch Schneiden mit geeigneten Restriktionsenzymen erfolgen. Geeignete Klonierungsvektoren, die bereits routinemässig für die Herstellung von genomischen und/oder cDNA Genbibliotheken verwendet werden umfassen beispielsweise Phagenvektoren, wie die λ-Charon Phagen oder aber bakterielle Vektoren, wie das *E. coli* Plasmid pBR322. Weitere geeignete Klonierungsvektoren sind dem Fachmann bekannt.

Aus den auf diese Weise hergestellten Genbibliotheken können dann im Rahmen eines Screening-Programmes geeignete Klone, die das Chitinasegen oder Teile davon enthalten, z.B. mit Hilfe geeigneter Oligonukleotidproben (Probenmolekül) aufgespürt und anschliessend isoliert werden. Zum Aufspüren geeigneter Klone stehen verschiedene Methoden zur Verfügung wie z.B. die differentielle Koloniehybridisierung oder die Plaquehybridisierung. Ebenso anwendbar sind immunologische Nachweismethoden, die auf einer Identifizierung der spezifischen Translationsprodukte beruhen.

Als Probenmolekül kann beispielsweise ein zuvor bereits isoliertes DNA Fragment aus dem gleichen oder aber einem strukturell verwandten Chitinasegen verwendet werden, das in der Lage ist mit dem korrespondierenden Sequenzabschnitt innerhalb des erfindungsgemässen Chitinasegens zu hybridisieren.

Sofern die Aminosäuresequenz des zu isolierenden Gens oder aber zumindest Teile dieser Sequenz bekannt sind, kann aufgrund dieser Sequenzinformation eine entsprechende korrespondierende DNA Sequenz entworfen werden. Da der genetische Kode bekanntlich degeneriert ist, können in der Mehrzahl der Fälle für ein und dieselbe Aminosäure verschiedene Kodons verwendet werden. Dies führt dazu, dass, abgesehen von wenigen Ausnahmefällen, in der Regel eine bestimmte Aminosäuresequenz von einer ganzen Reihe einander ähnlicher Oligonukleotide kodiert werden kann. Dabei ist jedoch zu beachten, dass nur ein Mitglied aus dieser Reihe von Oligonukleotiden tatsächlich mit der entsprechenden Sequenz innerhalb des gesuchten Gens übereinstimmt. Um von vorneherein die Anzahl möglicher Oligonukleotide zu begrenzen kann beispielsweise auf die von Lathe R *et al* (1985) aufgestellten Regeln für die Verwendung von Kondons zurückgegriffen werden, welche die Häufigkeit, mit der ein bestimmtes Kodon in eukaryontischen Zellen tatsächlich verwendet wird, berücksichtigen.

Aufgrund dieser Informationen lassen sich somit Oligonukleotidmoleküle entwerfen, die als Protenmoleküle für die Identifizierung und Isolierung geeigneter Klone verwendet werden können, indem man besagte Probenmoleküle in einem der zuvor beschriebenen Verfahren mit genomischer DNA oder cDNA hybridisiert.

Um die Nachweisbarkeit des gewünschten Chitinase-kodierenden Gens zu erleichtern, kann das zuvor beschriebene DNA-Probenmolekül mit einer geeigneten, leicht nachweisbaren Gruppe markiert werden. Unter einer nachweisbaren Gruppe soll im Rahmen dieser Erfindung jedes Material verstanden werden, das eine bestimmte leicht identifizierbare physikalische oder chemische Eigenschaft aufweist. Solche Materialien finden insbesondere auf dem Gebiet der Immunoassays bereits eine breite Anwendung und sind in der Mehrzahl der Fälle auch in der vorliegenden Anmeldung verwendbar. Insbesondere seien an dieser Stelle enzymatisch aktive Gruppierungen genannt, wie z.B. Enzyme, Enzymsubstrate, Coenzyme und Enzyminhibitoren, desweiteren fluoreszierende und lumineszierende Agentien, Chromophore sowie Radioisotope, wie z.B. $^{3}H$, $^{35}S$, $^{32}P$, $^{125}I$ und $^{14}C$. Die leichte Nachweisbarkeit dieser Marker beruht einerseits auf ihren inhärent vorhandenen physikalischen Eigenschaften (z.B. Fluoreszenzmarker, Chromophore, Radioisotope), andererseits auf ihren Reaktions- und Bindungseigenschaften (z.B. Enzyme, Substrate, Coenzyme, Inhibitoren).

Weiterhin geeignet als Probenmolekül ist eine einzelsträngige cDNA, die sich von einer poly(A)+ RNA ableitet, welche ihrerseits aus einem zur Produktion hoher Chitinasespiegel induzierten Gewebe oder einer Zelle isoliert wird. Besonders bevorzugt im Rahmen dieser Erfindung ist der Chitinase cDNA Klon pCHN 50, der zwar die Protein-kodierende DNA Sequenz, nicht aber die das N-terminale Signalpeptid kodierende DNA Sequenz enthält und der bei Shinshi *et al* (1987) beschrieben ist.

Allgemeine Verfahren betreffend Hybridisierung sind beispielsweise bei Maniatis T *et al* (1982) und bei Haymes BT *et al* (1985) beschrieben.

Diejenigen Klone innerhalb der zuvor beschriebenen Genbibliotheken, die in der Lage sind mit einem Probenmolekül zu hybridiseren und die sich mit Hilfe eines der oben erwähnten Nachweisverfahren identifizieren lassen, können dann weiter analysiert werden um Ausmass und Natur der Chitinasekodierenden Sequenz im einzelnen zu bestimmten.

Ein alternatives Verfahren zur Klonierung von Chitinasegenen beruht auf der Konstruktion einer Genbibliothek, die sich aus Expressionsvektoren aufbaut. Dabei wird in Analogie zu den zuvor bereits beschriebenen Verfahren zunächst genomische DNA, vorzugsweise aber cDNA, aus einerzelle oder einem Gewebe isoliert, das in der Lage ist Chitinase zu exprimieren und anschliessend in einen geeigneten Expressionsvektor eingespleisst. Die auf diese Weise hergestellten Genbibliotheken können dann mit Hilfe geeigneter Massnahmen, vorzugsweise unter Verwendung von anti-Chitinase Antikörpern, gescreent und diejenigen Klone ausgelesen werden, die das gewünschte Gen oder aber zumindest einenen Teil dieses Gens als Insert enthalten.

Mit Hilfe der zuvor beschriebenen Verfahren ist es somit möglich ein pflanzliches Chitinasegen, insbesondere aber ein basisches Chitinasegen aus Tabak, zu isolieren, das in seiner 5′-Region eine regulatorische DNA-Sequenz besitzt, die in operabler Verknüpfung mit einem beliebigen Strukturgen zu stark erhöhten Expressionsraten in transformiertem pflanzlichem Material führt.

Zur weiteren Charakterisierung dieses Chitinasegens werden die auf die zuvor beschriebene Weise gereinigten und isolierten DNA Sequenzen einer Sequenzanalyse unterzogen. Dabei wird das zuvor isolierte Chitinasegen zunächst mit Hilfe geeigneter Restriktionsenzyme in Fragmente zerlegt und anschliessend in geeignete Klonierungsvektoren, wie z.B. die M13 Vektoren mp18 und mp19 einkloniert. Die Sequenzierung wird in 5′ → 3′ Richtung durchgeführt, wobei vorzugsweise die Dideoxynucleotid-Kettenterminationsmethode nach Sanger [Sanger *et al*, 1977] oder das Verfahren nach Maxam and Gilbert [Maxam and Gilbert, 1980] Anwendung findet. Um Fehler bei der Sequenzierung zu vermeiden ist es vorteilhaft, wenn man beide DNA Stränge parallel sequenziert. Die Analyse der Nukleotid-und der korrespondierenden Aminosäuresequenz wird vorteilhafterweise Computer-unterstützt, unter Verwendung einer geeigneten Computersoftware, durchgeführt.

In einer bevorzugten Ausführungsform der vorliegenden Erfindung, die in den nachfolgenden Ausführungsbeispielen im Detail beschrieben ist, wird zunächst eine genomische Genbibliothek chromosomaler Tabak DNA hergestellt und mit Hilfe der Plaquehybridiserung gescreent, unter Verwendung eines zuvor isolierten cDNA Klons (pCHN 50, beschrieben bei Shinshi *et al*, 1987) als Probenmolekül. Nach Überprüfung von insgesamt 5 x $10^5$ Plaques erhält man 10 Rekombinanten, die zunächst gereinigt und dann mit Hilfe der Southern Blot Analyse teilweise charakterisiert werden. Zwei dieser Klone (λCHN14 und λCHN17), die sich aus unterschiedlichen Bereichen innerhalb des Genoms ableiten, enthalten das komplette Chitinasegen. Für die weitere Charakterisierungwird der KlonλCHN17 ausgewählt, da dieser mit den cDNA Klonen pCHN50 und pCHN48 (beschrieben in Beispiel 4.4), die als Probenmoleküle fungieren, ein sehr starkes Hybridisierungssignal erzeugt. Nach Verdauung mit dem Restriktionsenzym HindIII erhält man ein 5.4 Kb umfassendes DNA Fragment, welches das komplette Chitinasegen enthält und mit einem Fragment gleicher Grösse aus Tabak DNA korrelliert. Ein Teil dieses Fragments, das 3850 Bp umfasst und die gesamte kodierende Sequenz beinhaltet wird anschliessend sequenziert. Die komplette DNA Sequenz des basischen Chitinasegens aus Tabak ist in Abbildung 6 wiedergegeben.

Die 5′- und 3′- flankierenden Regionen des basischen Chitinasegens aus Tabak lassen diverse Bereiche erkennen, die in Analogie zu anderen eukaryontischen Genen in einen direkten Zusammenhang mit regulatorischen Funktionen gebracht werden können. So kann beispielsweise mit Hilfe der S1 Nuklease-Kartierung die Transkriptionsstartstelle der mRNA ermittelt werden. Man findet insgesamt zwei Banden, was die Vermutung nahe legt, das mehrere Startstellen auf dem Chitinasegen existieren. Geht man von der Intensität der betreffenden Banden aus, dann ist die Haupt-Transkriptionsstartstelle identisch mit dem A inposition 1969 innerhalb der Sequenz CT **A**CT. Das erste mögliche Initiationssignal befindet sich in Position 1980, 11 Bp stromabwärts des Transkriptionsstarts, innerhalb einer Basenfolge TAAA **ATG**AG, die in Übereinstimmung mit anderen Translationsstartstellen pflanzlicher Gene als Consensus-Sequenz bezeichnet wird.

Die 5′-flankierende Region ist darüberhinaus durch folgenden Basenfolgen charakterisiert:

(a) eine TAAATA-Sequenz ('TATA box') stromaufwärts der Transkriptionsstartstelle in Position -28 bis -23 der 5′-flankierenden Region;

(b) eine CCAATT-Sequenz in Position -114, ähnlich der CAAT Box, die zuweilen in tierischen Genen, stromaufwärts der TATA Box, gefunden wird ;

(c) eine 6 Bp umfassende unvollkommen invertierte Wiederholungssequenz ( GCCGAATTCG A GC ) in Position -140, die dem regulatorischen Hitzeschock-Consensuselement tierischer Gene ähnelt;

(d) eine 10 Bp umfassende vollkommene Wiederholungssequenz (ATGTCCAAAC) in Position -152 und -228;

(e) eine 20 Bp umfassende unvollkommene direkte Wiederholungssequenz ( TTTTAA C TAA-ATCTATGTCC ) in Position -166 und -569;

(f) eine 25 Bp umfassende unvollkommene direkte Wiederholungssequenz ( CAACTTT C AAAA A TT A TTTTTTAAA ) in Position -191 und -217;

(g) ein 20 Bp umfassendes Palindrom ( TAAAATATGA | TCAT G TTTTA ) in Position -289;

(h) eine 11 Bp umfassende vollkommene direkte Wiederholungssequenz (TAAGAGCCGCC) in Position -435 und -480;

(i) eine 15 Bp umfassende unvollkommene direkte Wiederholungssequenz ( TAAAATACA C GTCGA ) ir Position -514 und -644;

Die 3′-flankierende Region ist durch zwei AATAAA Sequenzen in Position 52 und 120, stomabwärts der Translationsstop Sequenz TAA gekennzeichnet.

Die erfindungsgemässe, regulatorische DNA Sequenz kann mit Hilfe geeigneter und dem Fachmann bestens bekannter Verfahren aus der 5′-Region des zuvor isolierten Chitinasegens gewonnen werden. Besonders bevorzugt im Rahmen dieser Erfindung ist die regulatorische DNA Sequenz aus der 5′-Region eines basischen Chitinasegens von *Nicotiana tabacum*. Bei diesem Sequenzabschnitt handelt es sich um eine A/T reiche Region, die zu wenigstens 55%, vorzugweise zu wenigstents 60% und ganz besonders bevorzugt zu wenigstens 70% aus A/T besteht und wenigstens 100, vorzugsweise aber wenigstens 40 und ganz besonders bevorzugt wenigstens 31 Basenpaare aufweist.

Im Falle des basischen Chitinasegens, isoliert aus einer klonierten Linie parenchymatischen Markgewebes von *Nicotiana tabacum* L. c.v. Havana 425 pflanzen [Eichholz *et al*, 1983], erstreckt sich die besagte Sequenz von Position -31 bis zur Position -1, dem Beginn des Translationsstartpunktes. Anhand der Sequenzanalyse lässt sich folgende Nukleotidsequenz für diesen Sequenzabschnitt ermitteln:

5′- **TTGCATTTCACCAGTTTACTACTACATTAAA** -3′

Diese nunmehr in ihrer Basenabfolge bekannte, regulatorische DNA Segeuenz muss nicht jedesmal wieder neu aus einem geeigneten Chitinasegen isoliert werden, sondern kann selbstverständlich jederzeit sehr einfach mit Hilfe chemischer Verfahren synthetisiert werden. Geeignete Verfahren zur Synthese kurzer DNA Oligonukleotide, wie z.B. die Phosphotriester- oder die Phosphitmethode sind dem Fachman bekannt. Heute sind die Oligonucleotidsynthesen zum grösstenteil mechanisiert und automatisiert, sodass kurze DNA-Fragmente innerhalb kurzer Zeit herstellbar sind.

Durch Deletion, Insertion oder Austausch einer oder mehrerer Basenpaare können daher sehr einfach Varianten oder Mutanten der oben näher spezifizierten DNA-Sequenz hergestellt und auf ihre Tauglichkeit überprüft werden.

Im einzelnen kann man dabei so vorgehen, dass man zunächst ein Gen identifiziert und isoliert, dass eine der erfindungsgemässen, regulatorischen DNA-Sequenzen enthält. Dieses Gen, insbesondere aber die 5′-gelegene regulatorische Sequenz, kann dann nach Einspleissen in einen geeigneten Klonierungsvektor mit Hilfe bekannter Verfahrensmassnahmen modifiziert werden. Besonders geeignet zur Herstellung spezifischer Mutanten ist die sog. Oligonukleotid-vermittelte Mutagenese. Dabei werden kurze Oligonukleotid-Fragmente synthetisiert, die der Wildtyp-Sequenz zwar im wesentlichen homolog sind, sich jedoch in einzelnen Nukleotiden von dieser unterscheiden. Bei besagten Unterschieden kann es sich um Insertionen, Deletionen oder um einen Austausch eines oder mehrerer Nukleotide handeln. Diese mutierten Fragmente werden dann im Rahmen allgemein bekannter Verfahren gegen die homolgen Gegenstücke auf dem Wildtypgen ausgetauscht. Das fertige Konstrukt kann dann, wie im folgenden im Betail beschrieben, in einen geeigneten Pflanzenexpressionsvektor einkloniert und in eine Pflanze transformiert werden.

Die vorliegende Erfindung beschränkt sich daher nicht auf die oben näher spezifizierte Basenabfolge, sondern umfasst darüberhinaus auch alle Mutanten und/oder Varianten dieser DNA Sequenz, die durch Deletion bzw. Insertion von einer oder mehreren Basen oder aber insbesondere durch Austausch von einer oder mehreren Basen entstanden sind und die noch die spezifischen, regulatorischen, expressionssteigernden Eigenschaften der Ausgangssequenz aufweisen.

Ebenso umfasst von der vorliegenden Erfindung sind Fragmente oder Teilsequenzen, die aus der oben näher bezeichneten DNA Sequenz oder aus Derivaten dieser DNA Sequenz erhältlich sind und die noch die spezifischen, regulatorischen, expressionssteigernden Eigenschaften der Ausgangssequenz aufweisen.

Besonders bevorzugt ist eine Teilsequenz, welche die folgende DNA-Sequenz aufweist:

**ACTACTACATTAAA**

Die auf die zuvor beschriebene Weise isolierbare, regulatorische DNA Sequenz oder Teilsequenzen davon können jetzt dazu verwendet werden um homologe DNA Sequenzen gleicher Funktion aufzusprüren, indem man beispielsweise zunächst genomische Genbibliotheken herstellt und diese in der zuvor beschriebenen Weise, unter Verwendung einer der erfindungsgemässen, regulatorischen DNA Sequenzen als Probenmolekül, auf das Vorhandensein homologer DNA Sequenzen untersucht, die in der Lage sind mit dieser zu hybridisieren.

Diese Verfahren zum Aufspüren homologer DNA Sequenzen gleicher Funktion unter verwendung einer der erfindungsgemässen DNA Sequenzen bilden ebenfalls einen Bestandteil der vorliegenden Erfindung.

Ein weiterer Gegengstand der vorliegenden Erfindung betrifft die Konstruktion von rekombinanten DNA Molekülen, die eine chimäre genetische Konstruktion enthalten, worin die erfindungsgemässe, regulatorische DNA-Sequenz in operabler Verknüpfung mit einem Strukturgen sowie weiteren, in pflanzlichen Zellen aktiven Expressionssignalen, wie Promotor- und Terminationssequenzen, vorliegt.

Dabei ist es oft vorteilhalt, zwischen der Promotorsequenz und der sich anschliessenden erfindungsgemässen DNA-Seqeuenz eine 'Spacer'-Sequenz einzubauen, wobei die Länge der 'Spacer'-Sequenz so gewählt wird, dass der Abstand zwischen dem Promotor und der erfindungsgemässen, regulatorischen DNA-Sequenz für die Expression des jeweils assoziierten Strukturgens optimal ist. Besonders bevorzugt im Rahmen dieser Erfindung ist eine 'Spacer'-Sequenz, die zwischen 1 Bp und 100 Bp, vorzugsweise aber zwischen 2 Bp und 46 Bp umfasst.

Die erfindungsgemässe, regulatorische DNA-Sequenz führt in operabler Verknüpfung mit einem Strukturgen sowie weiteren, in pflanzlichen Zellen aktiven Expressionssignalen, wie Promotor- und Terminationssequenzen, zu einer signifikanten Erhöhung der Expressionsrate, die eine Steigerung bis zum 400 fachen und mehr erfahren kann. Somit wird es im Rahmen der vorliegenden Erfindung nunmehr erstmals möglich die phänotypische Ausprägung von bestimmten Merkmalen in Pflanzen, wie z.B. einer Resistenz gegenüber bestimmten Pathogenen oder chemischen Verbindungen, die bisher aufgrund einer zu geringen Genexpression und einer damit verbundenen geringen Proteinkonzentration in der Zelle nicht oder aber nur in ungenügendem Ausmass zu erreichen war, signifikant zu verbessern. Dies ist insbesondere in den Fällen von Bedeutung, wo die Merkmalsausprägung direkt mit der Protein konzentration in der Zelle korreliert ist.

Eine Resistenz gegenüber Cytotoxinen beispielsweise kann durch Übertragung eines Gens erreicht werden, das ein Enzym kodiert und in der pflanzlichen Zelle exprimiert, welches das Cytotoxin detoxifiziert, wie z.B. die Neomycinphosphotransferase Typ II oder die Aminoglycosidphosphotransferase Typ IV, die zu einer Detoxifikation von Kanamycin, Hygromycin und anderen Aminoglykosidantibiotika beitragen oder eine Glutathion-S-Transferase, Cytochrom P-450 oder andere katabolisch wirksame Enzyme, von denen bekannt ist, dass sie Triazine, Sulfonylharnstoffe oder andere Herbizide detoxifizieren. Eine Resistenz gegenüber Cytotoxinen kann auch durch ein Gen vermittelt werden, das in einer Pflanze eine bestimmte Form eines 'Zielenzyms' (Angriffspunkt der Cytotoxinwirkung) exprimiert, die resistent ist gegen die Aktivität des Cytotoxins, wie z.B. eine Variante der Acetohydroxysäuresynthase, die gegenüber der inhibitorischen Wirkung von Sulfonylharnstoffen, Imidazolinonen oder anderen Herbiziden, die mit diesem spezifischen Stoffwechselschritt interagieren, insensitiv ist; oder eine Variante der EPSP Synthase, die sich gegenüber der Hemmwirkung von Glyphosate als insensitv erweist. Es kann sich als vorteilhaft erweisen, diese veränderten Zielenzyme in einer Form zu exprimieren, die ihren Transport in das korrekte zelluläre Kompartiment, wie z.B. im obigen Fall in die Chloroplasten, erlaubt.

In bestimmten Fällen kann es darüberhinaus von Vorteil sein, die Genprodukte in die Mitochondrien, die Vakuolen, das endoplasmatische Retikulum oder in andere Zellregionen, möglicherweise sogar in die interzellulären Räume (Apoplasten) zu dirigieren.

Eine Resitenz gegenüber bestimmten Pilzklassen kann beispielsweise durch die Einschleusung eines Gens erreicht werden, das Chitinase in den pflanzlichen Geweben exprimiert. Zahlreiche pflanzenpathogene Pilze enthalten Chitin als integralen Bestandteil ihrer Hyphen- und Sporenstrukturen, so z.B. die Basidiomyceten (Brand- und Rostpilze), Ascomyceten und *Fungi Imperfecti* (einschliesslich *Alternaria* und *Bipolaris*, *Exerophilum turcicum*, *Colletotricum*, *Gleocerco spora* und *Cercospora*). Chitinase ist in der Lage das Mycelwachstum bestimmter Pathogene *in-vitro* zu hemmen. Ein pflanzliches Blatt oder eine Wurzel, die Chitinase konstitutiv oder aber als Antwort auf das Eindringen eines Pathogens exprimiert, ist gegen den Angriff einer grossen Zahl verschiedener Pilze geschützt. Je nach Situation kann eine konstitutive Expression vorteilhaft sein im Vergleich zu einer induzierbaren Expression, die bei zahlreichen Pflanzen als normale Reaktion auf einen Pathogenbefall auftritt, da die Chitinase unmittelbar in hoher Konzentration vorliegt, ohne dass erst eine lag-Phase für die Neusynthese abgewartet werden muss.

Besonders bevorzugt im Rahmen dieser Erfindung ist ein basisches Chitinasegen, das aus Tabak erhältlich ist, insbesondere aber ein basisches Chitinasegen, das aus einer klonierten Linie parenchymatischen Markgewebes von *Nicotiana tabacum* L. c.v. Havana 425 Pflanzen [Eichholz *et al*, 1983] erhältlich ist und die in Figur 6 wiedergegebene Sequenz enthält.

Eine Resistenz gegenüber Insekten kann beispielsweise durch ein Gen übertragen werden, das ein Polypeptid kodiert, welches toxisch ist für Insekten und/oder deren Larven, wie z.B. das kristalline Protein von *Bacillus thuringiensis*. Eine zweite Klasse von Proteinen, die eine Insektenresistenz vermitteln, sind die Proteaseinhibitoren. Proteaseinhibitoren bilden einen gewöhnlichen Bestandteil von pflanzlichen Speicherstrukturen. Es konnte nachgewiesen werden, dass ein aus Sojabohnen isolierter und gereinigter Bowman-Birk Proteaseinhibitor die Darmprotease von *Tenebrio* Larven hemmmt [Birk *et al* (1963)]. Das Gen, welches den Trypsininhibitor aus der Kuherbse kodiert, ist bei Hilder *et al* (1987) beschrieben.

Ein Gen, das einen Proteaseinhibitor kodiert, kann in einem geeigneten Vektor unter die Kontrolle eines Pflanzenpromotors, insbesondere eines konstitutiven Promotors, wie z.B. des CaMV 35S Promotors, gebracht werden. Das Gen, beispielsweise die kodierende Sequenz des Bowman-Birk Proteaseinhibitors aus der Sojabohne, kann mit Hilfe der cDNA Klonierungsmethode erhalten werden. Eine weitere Möglichkeit zur Herstellung eines Proteaseinhibitors besteht in dessen synthetischer Herstellung, sofern dieser weniger als 100 Aminosäuren aufweist, wie z.B. der Trypsininhibitor der Limabohne. Die kodierende Sequenz lässt sich durch Zurückübersetzen der Aminosäuresequenz voraussagen. Zusätzlich werden an beiden Enden Restriktionsschnittstellen eingebaut, die für den jeweils gewünschten Vektor geeignet sind. Das synthetische Gen wird durch Synthese überlappender Oligonukleotidfragmente von 30 bis 60 Basenpaaren hergestellt, indem diese zunächst einer Kinasereaktion unterworfen, dann miteinander verknüpft [Maniatis *et al* (1982) und schliesslich in einem passenden Vektor kloniert werden. Mit Hilfe der DNA Sequenzierung kann dann ein Klon, der das Insert in einer korrekten Orientierung aufweist, identifiziert werden. Für die Einschleusung in die Protoplasten kann isolierte Plasmid-DNA verwendet werden.

Die Mehrzahl der Insekten besitzt ein Cuticularskelett, in dem lamellenförmig geschichtete Micelle aus Chitin in einer Grundsubstanz eingebettet sind. Weiterhin denkbar zur Erzeugung einer Resistenz oder zumindest einer Toleranz gegenüber pathogenen Insekten ist daher die Verwendung eines Gens, das ein Chitinaseenzym kodiert und nach Einschleusung in die Pflanze dort exprimiert. Besonders bevorzugt im Rahmen dieser Erfindung ist ein basisches Chitinasegen, das aus Tabak erhältlich ist, insbesondere aber ein basisches Chitinasegen, das aus einer klonierten Linie parenchymatischen Markgewebes von *Nicotiana tabacum* L. c.v. Havana 425 pflanzen [Eichholz *et al*, 1983] erhältlich ist und die in Figur 6 wiedergegebene Sequenz enthält.,

Ein weiteres Enzym, das im Abwehrmechanismus der Pflanze gegenüber Pathogenen vermutlich eine zentrale Rolle spielt, ist die $\beta$-1.3-Glucanase, die im Rahmen dieser Erfindung daher ebenfalls bevorzugt ist.

In all diesen Fällen ist zu erwarten, dass der Schutzeffekt für die Pflanze mit steigender Expression der eingeschleusten Gene und als Folge davon mit steigender Protein- (Enzym-)konzentration zunimmt.

Besagte Steigerung der Expressionsrate von eingeschleusten Fremdgenen konnte jetzt im Rahmen der vorliegenden Erfindung überraschenderweise dadurch erreicht werden, dass man ein Strukturgen, welches bei Expression in der Pflanze zu einem Schutzeffekt für die Pflanze führt, in operabler Weise mit der erfindungsgemässen, regulatorischen DNA Sequenz sowie gegebenenfalls mit weiteren für die Expression sowie die Regulation von Transkription und Translation benötigten Sequenzabschnitten, die im Bezug auf die Zielpflanze sowohl homologen als auch herterolognen Ursprungs sein können, verknüpft und die resultierende chimäre genetische Konstruktion mit Hilfe an sich bekannter Massnahmen in die Zielpflanze einschleust.

Für die Verwendung in dem erfindungsgemässen Verfahren sind somit in erster Linie all diejenigen Gene geeignet, die bei den transformierten pflanzlichen Zellen sowie den daraus sich entwickelnden Geweben und insbesondere aber den Pflanzen zu einem Schutzeffekt führen, wie z.B. zu einer erhöhten Resistenz gegenüber Pathogenen (beispielsweise gegenüber phytopathogenen Pilzen, Bakterien, Viren, etc.); einer Resistenz gegenüber Chemikalien [beispielsweise gegenüber Herbiziden (wie z.B. Triazinen, Sulfonylharnstoffen, Imidazolinonen, Triazolpyrimidinen, Bialaphos, Glyphosate, etc,), Insektiziden oder anderen Bioziden]; einer Resistenz gegenüber nachteiligen Umwelteinflüssen (beispielsweise gebenüber Hitze, Kälte, Wind, ungünstigen Bodenverhältnissen, Feuchtigkeit, Trockenheit, etc.).

Auch für eine etwaige Produktionssteigerung gewünschter und nützlicher Verbindungen in der pflanzlichen Zelle als solcher oder als Bestandteil einer höher organisierten Einheit, wie z.B. einem Gewebe, Kallus, Organ, Embryo oder einer ganzen Pflanze, lässt sich die erfindungsgemässe DNA-Seqeunz in idealer Weise verwenden. Gene, die im Rahmen der vorliegenden Erfindung Verwendung finden können umfassen beispielsweise solche, die zu einer erhöhten Bildung von Reserve- oder Speicherstoffen in Blättern, Samen, Knollen, Wurzel, Stengeln, etc. führen. Zu den wünschenswerten Substanzen, die von transgenen Pflanzen produziert werden können, zählen beispielsweise Proteine, Stärken, Zucker, Aminosäuren, Vitamine, Alkaloide, Flavine, Riech- und Farbstoffe, Fette, etc..

Ebenso können Strukturgene mit der erfindungsgemässen, regulatorischen DNA-Sequenz assoziiert werden, die pharmazeutisch akzeptable Wirksubstanzen, wie z.B. bestimmte Alkaloide, Steroide, Hormone, Immunmodulatoren und andere physiologisch aktive Substanzen kodieren.

Zu den Genen, die im Rahmen dieser Erfindung in Betracht gezogen werden können, gehören daher bekannte Gene, ohne jedoch darauf beschränkt zu sein, beispielsweise pflanzenspezifische Gene wie das Zeingen aus Mais, das Aveningen aus Hafer, das Glutelingen aus Reis, etc., Säuger-spezifische Gene wie das Insulingen, das Somatostatingen, die Interleucingene, das t-PA Gen, etc., oder aber Gene mikrobiellen Ursprungs wie das NPT II Gen, etc. sowie synthetische Gene wie das Insulingen, etc.

Neben natürlicherweise vorkommenden Strukturgenen, die eine nützliche und wünschenswerte Eigenschaft kodieren, können im Rahmen dieser Erfindung auch Gene verwendet werden, die zuvor mit Hilfe chemischer oder gentechnologischer Methoden gezielt modifiert wurden.

Weiterhin sind von dem breiten Konzept der vorliegenden Erfindung auch solche Gene umfasst, die vollständig durch chemische Synthese hergestellt werden. Als Gene oder DNA Sequenzen die im Rahmen der vorliegenden Erfindung Verwendung finden können, kommen somit sowohl homologe als auch heterologe Gen(e) oder DNA sowie synthetische Gen(e) oder DNA gemäss der im Rahmen der vorliegenden Erfindung gemachten Definition in Betracht. Als Beispiel für ein synthetisches Gen sei an dieser Stelle das Insulingen genannt.

Weiterhin kann im Rahmen der vorliegenden Erfindung auch sog. 'anti-sense' DNA verwendet werden, die in operabler Verknüpfung mit in pflanzlichen Zellen aktiven Expressionssignalen zur Produktion eines RNA Moleküls führt, das zumindest einem Teil einer von einer 'sense' DNA kodierten mRNA komplementär ist und diese daher zu binden vermag. Auf diese Weise kann die Translation einer bestimmten mRNA in das korrespondierende Protein verhindert oder aber zumindest eingeschränkt werden, sodass man hiermit ein Instrumentarium in Händen hat mit dessen Hilfe eine effektive Kontrolle der Genexpression von ausgesuchten Genen in der Pflanze möglich wird.

Die im Rahmen der vorliegenden Erfindung verwendbaren DNA Sequenz können ausschliesslich aus genomischer, aus cDNA bzw. synthetischer DNA konstruiert sein. Eine andere Möglichkeit besteht in der Konstruktion einer hybriden DNA-Sequenz bestehend sowohl aus cDNA als auch aus genomischer DNA und/oder synthetischer DNA.

In diesem Fall kann die cDNA aus demselben Gen stammen wie die genomische DNA oder aber, sowohl die cDNA als auch die genomische DNA können aus verschiedenen Genen stammen. In jedem Fall können aber sowohl die genomische DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die verschiedenen Stämmen oder Varietäten derselben Art oder verschiedenen Spezies derselben Gattung angehören, oder aber von Organismen, die mehr als einer Gattung derselben oder einer anderen taxonomischen Einheit (Reich) angehören, abstammen.

Um die Expression besagter Strukturgene in der pflanzlichen Zelle zu gewährleisten, ist es vorteilhaft, wenn die kodierenden Gensequenzen zunächst in operabler Weise mit in pflanzlichen Zellen funktionsfähigen Expressions-Sequenzen verknüpft werden.

Die hybriden Genkonstruktionen im Rahmen der vorliegenden Erfindung enthalten somit neben der erfindungsgemässen, regulatorischen DNA-Sequenz ein oder mehrere Strukturgen(e) sowie Expressions-Signale, die sowohl Promotor- und Terminator-Sequenzen als auch weitere regulatorische Sequenzen der 3′ und 5′ nicht-translatierten Regionen miteinschliessen.

Jeder Promotor und jeder Terminator, der in der Lage ist, eine Induktion der Expression einer kodierenden DNA-Sequenz (Strukturgen) zu bewirken, kann als Bestandteil der hybriden Gensequenz verwendet werden. Besonders geeignet sind Expressionssignale, die aus Genen von Pflanzen oder Pflanzenviren stammen. Beispiele geeigneter Promotoren und Terminatoren sind z.B. solche der Cauliflower Mosaik Virus Gene (CaMV) oder aber homologe DNA-Sequenzen, die noch die charakteristischen Eigenschaften der genannten Expressionssignale aufweisen. Ebenfalls geeignet sind bakterielle Expressionssignale, insbesondere aber die Expressionssignale der Nopalin-Synthase-Gene (nos) oder deroctopin-Synthase-Gene (ocs) aus den Ti-Plasmiden von *Agrobacterium tumefaciens*.

Bevorzugt im Rahmen dieser Erfindung sind die 35S und 19S Expressions-Signale des CaMV Genoms oder deren Homologe, die mit Hilfe molekularbiologischer Methoden, wie sie z.B. bei Maniatis *et al*,(1982) beschrieben sind, aus besagtem Genom isoliert und mit der kodierenden DNA-Sequenz verknüpft werden können.

Unter Homologen der 35S und 19S Expressionssignale sind im Rahmen dieser Erfindung Sequenzen zu verstehen, die trotz geringer Sequenzunterschiede den Ausgangssequenzen im wesent lichen homolog sind und die nach wie vor die gleiche Funktion wie diese erfüllen.

Als Ausgangsmaterial für die 35S-Transkriptionskontroll-Sequenzen kann erfindungsgemäss z.B. das ScaI-Fragment des CaMV Stammes "S", das die Nukleotide 6808-7632 der Genkarte umfasst [Frank G *et al* (1980)], verwendet werden.

Die 19S Promotor- und 5′ nicht-translatierte Region befindet sich auf einem Genomfragment zwischen der PstI Stelle (Position 5386) und der HindIII-Stelle (Position 5850) der CaMV Genkarte [Hohn *et al* (1982)-]. Die entsprechende Terminator- und 3′ nichttranslatierte Region liegt auf einem EcoRV/BglII-Fragment zwischen Position 7342 und 7643 des CaMV Genoms.

Weiterhin bevorzugt im Rahmen dieser Erfindung sind die Expressionssignale des CaMV Stammes CM 1841, dessen komplette Nukleotidsequenz bei Gardner RC *et al* (1981)beschrieben ist.

Ein weiterer wirksamer Vertreter eines Pflanzenpromotors, der Verwendung finden kann, ist ein überproduzierender Pflanzenpromotor. Diese Art von Promotoren sollte, sofern sie in operabler Weise mit der Gensequenz, welche ein gewünschtes Genprodukt kodiert, verknüpft ist, in der Lage sein, die Expression besagter Gensequenz zu vermitteln.

Ueberproduzierende Pflanzenpromotoren, die im Rahmen der vorliegenden Erfindung zur Anwendung kommen können, schliessen den Promotor der kleinen Untereinheit (small subunit; ss) der Ribulose-1,5-bisphosphat-Carboxylase aus Sojabohnen sowie den Promotor des Chlorophyll-a/b- Bindungsproteins ein. Diese beiden Promotoren sind dafür bekannt, dass sie in eukaryontischen Pflanzenzellen durch Licht induziert werden [siehe z.B. Genetic Engineering of Plants, an Agricultural Perspective, Cashmore A(1983)].

Ein weiterer Gegenstand der vorliegenden Erfindung betrifft somit rekombinante DNA Moleküle, die eine chimäre genetische Konstruktion enthalten, worin die erfindungsgemässe, regula torische, expressionssteigernde DNA-Sequenz mit einer exprimierbaren DNA sowie mit weiteren, in pflanzlichen Zellen aktiven Expressionssignalen, operabel verknüpft ist, sodass es bei Transformation in einen pflanzlichen Wirt zu einer signifikanten Erhöhung der Expressionsrate der operabel assoziierten exprimierbaren DNA kommt.

Besonders bevorzugt sind rekombinante DNA Moleküle die eine chimäre genetische Konstruktion enthalten, worin die erfindungsgemässe, regulatorische, expressionssteigernde DNA-Sequenz mit einem Strukturgen operabel assoziiert ist, das den transformierten pflanzlichen Zellen sowie den daraus sich entwickelnden Geweben undinsbesondere aber den Pflanzen einen Schutzeffekt gegenüberpathogenen, Chemikalien sowie nachteiligen (endaphischen oder atmosphärischen) Umwelteinflüssen verleiht.

Ganz besonders bevorzugt im Rahmen dieser Erfindung sind rekombinante DNA Moleküle, die eine chimäre genetische Konstruktion enthalten, worin die erfindungsgemässe, regulatorische, expressionssteigernde DNA-Sequenz mit einem Strukturgen operabel assoziiert ist, das Chitinase oder Glucanase in pflanzlichen Zellen exprimiert.

Ebenfalls umfasst von der vorliegenden Erfindung sind rekombinante DNA Moleküle, die eine chimäre genetische Konstruk tion enthalten, worin die erfindungsgemässe, regulatorische, expressionssteigernde DNA-Sequenz mit einem Strukturgen operabel assoziiert ist, das bei Expression in der transformierten pflanzlichen Zelle als solcher oder als Bestandteil einer höher organisierten Einheit ausgewählt aus der Gruppe bestehend aus einem Gewebe, Organ, Kallus, Embryo oder einer ganzen Pflanze, zu einer Produkitonssteigerung gewünschter und nützlicher Verbindungen führt.

Weitere regulatorische DNA-Sequenzen, die für die Konstruktion chimärer Gene Verwendung finden können umfassen beispielsweise solche Sequenzen, die in der Lage sind die Transkription einer assoziierten DNA-Sequenz in pflanzlichen Geweben im Sinne einer Induktion oder Repression zu regulieren.

So gibt es beispielsweise einzelne Pflanzengene, von denen bekannt ist, dass sie durch verschiedene interne und externe Faktoren wie Pflanzenhormone, Hitzeschock, Chemikalien, Pathogene, Sauerstoffmangel, Licht, etc. induziert werden.

Als ein Beispiel für eine Genregulation durch ein Pflanzenhormon soll hier die Abszissinsäure (ABS) erwähnt werden, von der bekannt ist, dass sie den während der späten Embryonalphase auftretenden Überschuss an mRNAs in Baumwolle induziert. Ein weiteres Beispiel liefert die Gibberellinsäure (GA3), die in Rizinussamen Malatsynthasetranskripte sowie in den Aleuronschichten von Gerste Isoenzyme der α-Amylase induziert.

Die Aktivität von Glucanase und Chitinase in Bohnenblättern kann durch Behandlung mit dem Stresshormon Ethylen markant erhöht werden. Für Chitinase wird dieser Induktionseffekt über den Promotor des Chitinasegens gesteuert, was durch Reportergen versuche unter Verwendung eines Promotors aus dem Chitinasegen von Bohnen (*Phaseolus vulgaris*) gezeigt werden konnte

Die Regulation von Hitzeschock empfindlichen Proteingenen der Sojabohne wurde im Detail untersucht. Eine mehrstündige Behandlung der Pflanzen bei einer Temperatur von 40° C resultiert in der *de novo* Synthese von sogenannten Hitzeschock-Proteinen. Zahlreiche dieser Gene wurden inzwischen isoliert und ihre Regulation im einzelnen analysiert. Die Expression dieser Gene wird in erster Linie auf der Ebene der Transkription kontrolliert. Fusioniert man beispielsweise den Promotor des hps70 Gens mit dem Neomycinphosphotransferase II (NPT II) Gen, so kann das auf diese Weise entstandene chimäre Gen durch einen Hitzeschock induziert werden (Spena et al, 1985).

Eine andere Klasse von in Pflanzen induzierbaren Genen beinhaltet die Licht-regulierten Gene, insbesondere das nukleär kodierte Gen der kleinen Untereinheit der Ribulose-1,5-bisphosphatcarboxylase (RUBISCO). Morelli *et al* (1985) haben nachgewiesen, dass die 5′-flankierende Sequenz eines RUBISCO-Gens aus der Erbse in der Lage ist, die Lichtinduzierbarkeit auf ein Reportergen zu übertragen, sofern dies in chimärer Form mit dieser Sequenz verknüpft ist. Diese Beobachtung konnte auch auf andere Licht-induzierte Gene ausgedeht werden, wie z.B. das Chlorophyll a/b -Bindungsprotein.

Die Alkoholdehydrogenase-Gene (adh-Gene) des Mais waren Gegenstand intensiver Untersuchungen. Das adh1-s Gen aus Mais wurde isoliert und es wurde nachgewiesen, dass ein Teil der 5′-flankierenden DNA in der Lage ist, die Expression eines chimären Reportergens (z.B. Chloramphenicolacetyltransferase; CAT ) zu induzieren, wenn das vorübergehend transformierte Gewebe anaeroben Bedingungen ausgesetzt wurde [Howard *et al* (1987)]

Eine weitere Gruppe regulierbarer DNA-Sequenzen betrifft chemisch regulierbare Sequenzen, die z.B. in den PR-("pathogenesis related proteine")Proteingenen des Tabaks vorliegen und mit Hilfe chemischer Regulatoren induzierbar sind.

Die zuvor beispielhaft genannten regulierbaren DNA-Sequenzen können sowohl natürlichen als auch synthetischen Ursprungs sein oder aber aus einem Gemisch von natürlichen und synthetischen DNA-Sequenzen bestehen.

Ein weiterer Gegenstand der vorliegenden Erfindung umfasst somit chimäre genetische Konstruktionen, die neben der in operabler Verknüpfung mit einem Strukturgen vorliegenden erfindungsgemässen regulatorischen DNA Sequenz weitere regulatorische DNA Sequenzabschnitte enthalten, die z.B. eine gezielte Induktion oder Repression der Genexpression ermöglichen.

Die verschiedenen Sequenzabschnitte können mit Hilfe an sich bekannter Methoden miteinander zu einer vollständigen, in pflanzlichen Zellen exprimierbaren DNA-Sequenz verknüpft werden. Geeignete Methoden schliessen beispielsweise die *in vivo* Rekombination von DNA-Sequenzen, die homologe Abschnitte aufweisen sowie die *in vitro* Verknüpfung von Restriktionsfragmenten mit ein.

Als Klonierungsvektoren verwendet man im allgemeinen Plasmid-oder Virus-(Bakteriophage)-Vektoren mit Replikations- und Kontrollsequenzen, die von Spezies stammen, die mit der Wirtszelle kompatibel sind.

Der Klonierungsvektor trägt in der Regel einen Replikationsursprung, ausserdem spezifische Gene, die zu phaenotypischen Selektionsmerkmalen in der transformierten Wirtszelle führen, insbesondere zu Resistenzen gegenüber Antibiotika oder gegenüber bestimmten Herbiziden. Die transformierten Vektoren können anhand dieser phaenotypischen Marker nach einer Transformation in einer Wirtszelle selektiert werden.

Selektierbare phaenotypische Marker, die im Rahmen dieser Erfindung Anwendung finden können, umfassen beispielsweise Resistenzen gegen Ampicillin, Tetracyclin, Hygromycin, Kanamycin, Methotrexat, G418 und Neomycin, ohne das diese beispielhafte Aufzählung eine Limitierung des Erfindungsgegenstandes darstellt.

Als Wirtszellen kommen im Rahmen dieser Erfindung Prokaryonten in Frage, einschliesslich bakterieller Wirte, wie z.B. *A.tumefaciens*, *E.coli*, *S.typhimurium* und *Serratia marcescens*, sowie Cyanobakterien. Ferner können auch eukaryontische Wirte wie Hefen, mycelbildende Pilze und Pflanzenzellen im Rahmen dieser Erfindung verwendet werden.

Das Einspleissen der erfindungsgemässen hybriden Genkonstruktion in einen geeigneten Klonierungsvektor erfolgt mit Hilfe von Standardmethoden wie sie z.B. bei Maniatis *et al* (1982) beschrieben sind.

Dabei wird in der Regel der Vektor und die einzuspleissende DNA Sequenz zunächst mit geeigneten Restriktions-Enzymen geschnitten. Geeignete Restriktionsenzyme sind z.B. solche, die Fragmente mit glatten Enden liefern, wie z.B. SmaI, HpaI und EcoRV, oder aber Enzyme, die kohäsive Enden bilden, wie z.B. EcoRI, SacI und BamHI.

Sowohl Fragmente mit glatten Enden wie auch solche mit kohäsiven Enden, die einander komplementär sind, können mit Hilfe geeigneter DNA-Ligasen wieder zu einem einheitlichen durchgehenden DNA-Molekül verknüpft werden.

Glatte Enden können auch durch Behandlung von DNA-Fragmenten, die überhängende kohäsive Enden aufweisen, mit dem Klenow-Fragment der *E.coli* DNA-Polymerase durch Auffüllen der Lücken mit den entsprechenden komplementären Nukleotiden hergestellt werden.

Anderseits lassen sich auch kohäsive Enden künstlich herstellen, z.B. durch Anfügen komplementärer homopolymerer Schwänze an die Enden einer gewünschten DNA-Sequenz sowie des geschnittenen Vektormoleküls unter Verwendung einer terminalen Desoxynukleotidyl-Transferase oder aber durch Anfügen synthetischer Oligonukleotid-Sequenzen (Linker), die eine Restriktionsschnittstelle tragen und anschliessendes Schneiden mit dem entsprechenden Enzym.

Der Klonierungsvektor und die mit diesem Vektor transformierte Wirtszelle werden in der Regel dazu verwendet, die Kopienzahl des Vektors zu erhöhen. Mit einer erhöhten Kopienzahl ist es möglich, den Vektor, der die hybride Genkonstruktion trägt, zu isolieren und z.B. für die Einschleusung der chimären Gensequenz in die pflanzliche Zelle vorzubereiten.

In einem weiteren Verfahrensschritt werden diese Plasmide dazu verwendet, die ein gewünschtes Genprodukt kodierenden Strukturgene oder aber nicht- kodierende DNA-Sequenzen mit regulatorischer Funktion, wie z.B. 'anti-sense' DNA, in die pflanzliche Zelle einzuschleusen und gegebenenfalls in das Pflanzengenom zu integrieren.

Ein weiterer Gegenstand der vorliegenden Erfindung betrifft daher die Herstellung von pflanzlichen Rezipienten-Zellen, welche besagtes Strukturgen oder sonstige wünschenswerte Gene bzw. Genfragmente oder sonstige nützliche DNA-Sequenzen in ihr Genom eingebaut enthalten.

Die Einschleusung der chimären genetischen Konstruktion erfolgt vorzugsweise in pflanzliche Protoplasten mit Hilfe bekannter Gen-Transfer-Verfahren.

Es existieren mittlerweile eine Reihe von sehr effizienten Verfahren für die Einführung von DNA in Pflanzenzellen, basierend auf der Verwendung von Gentransfer-Vektoren oder auf direkten Gentransfer-Verfahren.

Eine Möglichkeit besteht beispielsweise darin, dass man Pflanzenzellen mit Viren oder mit *Agrobacterium* in Kontakt bringt. Dies kann durch Infektion sensitiver Pflanzenzellen oder durch Co-Cultivierung von Protoplasten, die sich aus Pflanzenzellen ableiten, bewerkstelligt werden. Das Cauliflower Mosaik Virus (CaMV) kann im Rahmen dieser Erfindung ebenso als Vektor für die Einschleusung der erfindungsgemässen chimären genetischen Konstruktion in die Pflanze verwendet werden. Das gesamte virale DNA-Genom von CaMV wird dabei in ein bakterielles Elternplasmid integriert unter Ausbildung eines rekombinanten DNA Moleküls, das in Bakterien vermehrt werden kann. Nach erfolgter Klonierung wird das rekombinante Plasmid mit Hilfe von Restriktionsenzymen entweder zufällig oder an ganz spezifischen, nicht essentiellen Stellen innerhalb des viralen Teils des rekombinanten Plasmids gespalten, z.B. innerhalb des Gens, das für die Uebertragbarkeit des Virus durch Blattläuse kodiert, und die hybride Genkonstruktion wird in diese Schnittstelle einkloniert.

Ein kleines Oligonukleotid, ein sog. Linker, der eine einzige, spezifische Restriktionserkennungsstelle besitzt, kann ebenfalls integriert werden. Das so modifizierte rekombinante Plasmid wird erneut kloniert und durch Einspleissen der hybriden Genkonstruktion in eine nur einmal vorkommende Restriktionsstelle weiter modifiziert.

Der modifizierte virale Anteil des rekombinanten Plasmids kann dann aus dem bakteriellen Eltern-Plasmid ausgeschnitten und für die Inokulation der Pflanzenzellen oder der gesamten Pflanzen verwendet werden.

Eine andere Methode zur Einschleusung der chimären Genkonstruktion in die Zelle bedient sich der Infektion der Pflanzenzelle mit *Agrobacterium tumefaciens* und/oder *Agrobacterium rhizogenes*, welches mit besagter Genkonstruktion transformiert ist. Die transgenen Pflanzenzellen werden anschliessend unter geeigneten, dem Fachmann bekannten Kultivierungsbedingungen kultiviert, so dass sie Schösslinge und Wurzeln ausbilden und letztlich ganze Pflanzen resultieren.

Eine weitere Möglichkeit zur Transformation pflanzlichen Materials besteht in einer gemischten Infektion unter Verwendung von sowohl *Agrobacterium rhizogenes* als auch transformiertem *Agrobacterium tumefaciens* wie es von Petit *et al* (1986) für die Transformation von Karotten beschrieben ist. Das Mischungsverhältnis muss dabei so aufeinander abgestimmt werden, dass die aufgrund der *A. rhizogenes* Trans formation gebildeten Würzelchenkolonien auch einen hohen Anteil an *A. tumefaciens* Ti-Plasmiden enthalten. Dies kann z.B. dadurch erreicht werden, dass man *A. rhizogenes* und *A. tumefaciens* in einem Mischungsverhältnis von 1:1 bis 1:100, vorzugsweise aber in einem Mischungsverhältnis von 1:10 in bekannter Weise gemeinsam auf das Pflanzenmaterial appliziert. Die transgenen Pflanzen werden anschliessend unter geeigneten, dem Fachmann bekannten Kultivierungsbedingungen kultiviert, sodass sie Schösslinge und Wurzel ausbilden und letztlich ganze Pflanzen resultieren. Das Vermischen der beiden *Agrobacterium* Spezies erfolgt vorteilhafterweise erst kurz vor der eigentlichen Inokulation des zu transformierenden pflanzlichen Materials.

Die erfindungsgemässe chimäre Genkonstruktion lässt sich somit beispielsweise mit Hilfe des Ti-Plasmids von *Agrobacterium tumefaciens* oder des Ri-Plasmids von *Agrobacterium rhizogenes* in geeignete Pflanzenzellen überführen. Das Ti-Plasmid bzw. das Ri-Plasmid wird im Verlaufe der Infektion durch *Agrobacterium* auf die Pflanze übertragen und stabil ins Pflanzengenom integriert.

Sowohl Ti-Plasmide als auch Ri-Plasmide besitzen zwei Regionen, die für die Herstellung transformierter Zellen essentiell sind. Eine davon, die Transfer-DNA (T-DNA) Region, wird auf die Pflanze übertragen und führt zur Induktion von Tumoren. Die andere, die virulenzverleihende (vir) Region, ist nur für die Ausbildung, nicht aber für die Aufrechterhaltung der Tumoren essentiell. Die Transfer-DNA Region kann in ihren Dimensionen durch Einbau der chimären Genkonstruktion vergrössert werden, ohne dass die Uebertragungsfähigkeit beeinträchtigt wird. Durch Entfernen der tumorverursachenden Gene und durch Einbau eines selektionierbaren Markers kann das modifizierte Ti- bzw. Ri-Plasmid als Vektor für den Transfer der erfindungsgemässen Genkonstruktion in eine geeignete Pflanzenzelle verwendet werden.

Die vir-Region bewirkt den Transfer der T-DNA Region von *Agrobacterium* auf das Genom der Pflanzenzelle, unabhängig davon, ob die T-DNA-Region und die vir-Region auf demselben Vektor oder auf verschiedenen Vektoren innerhalb derselben *Agrobacterium*-Zelle vorliegen. Eine vir-Region auf einem Chromosom induziert ebenso den Transfer der T-DNA von einem Vektor in eine Pflanzenzelle.

Im Rahmen dieser Erfindung wird daher bevorzugt ein System zur Uebertragung einer T-DNA-Region von *Agrobacterium* in Pflanzenzellen verwendet, bei dem die vir-Region und die T-DNA-Region auf verschiedenen Vektoren liegen. Ein solches System ist unter dem Begriff "binäres Vektor-System" bekannt und der die T-DNA enthaltende Vektor wird entsprechend als ein "binärer Vektor" bezeichnet.

Jeder T-DNA enthaltende Vektor, der in Pflanzenzellen übertragbar ist und der eine Selektion der transformierten Zellen erlaubt, ist für die Verwendung im Rahmen dieser Erfindung geeignet.

Besonders bevorzugt im Rahmen dieser Erfindung ist ein 'Shuttle'-Vektor, der die erfindungsgemässe chimäre genetische Konstruktion zwischen der Linken (LB) und Rechten Bordersequenz (RB) einkloniert enthält und der in der Lage ist sowohl in *E. coli* als auch in *A. tumefaciens* stabil zu replizieren.

Durch Anwendung neu entwickelter Transformationstechniken lassen sich mittlerweile *in vitro* auch solche Pflanzenspezies transformieren, die keine natürlichen Wirtspflanzen für *Agrobacterium* sind. So sind beispielsweise monokotyle Pflanzen, insbesondere aber die Getreidearten sowie verschiedene Gräser, keine natürlichen Wirte für *Agrobacterium.*

Es gibt in der Zwischenzeit aber vermehrt Hinweise darauf, dass sich auch Monokotyledonen mit *Agrobacterium* transformieren lassen, so dass unter Verwendung von neuen experimentellen Ansätzen, die jetzt verfügbar werden, auch Getreide und Gräser-Spezies einer Transformation zugänglich sind [Grimsley NH *et al* (1987)].

Bevorzugt im Rahmen dieser Erfindung ist die sog. Blattscheiben-Transformation ("Leaf Disk Transformation") unter Verwendung von *Agrobacterium* [Horsch *et al* (1985)]. Sterile Blattscheiben einer geeigneten Zielpflanze werden dabei mit *Agrobacterium* Zellen, die eine der erfindungsgemässen chimären Genkonstruktionen enthalten, inkubiert und anschliessend in oder auf ein geeignetes Nährmedium überführt. Besonders geeignet und daher im Rahmen dieser Erfindung bevorzugt sind LS Medien, die durch Zusatz von Agar verfestigt sind und die mit einem oder mehreren der üblicherweise verwendeten pflanzlichen Wachstumsregulatoren, insbesondere aber solchen ausgewählt aus der Gruppe der Auxine bestehend aus α-Naphthylessigsäure, Picloram, 2,4,5-Trichlorphenoxyessigsäure, 2,4-Dichlorphenoxyessigsäure, Indol-3-buttersäure, Indol-3-milchsäure, Indol-3-bernsteinsäure, Indol-3-essigsäure, p-Chlorphenoxyessigsäure sowie aus der Gruppe der Cytokinine bestehend aus Kinetin, 6-Benzyladenin, 2-Isopentenyladenin und Zeatin, angereichert sind. Die bevorzugte Konzentration der Auxine und Cytokinine liegt in einem Bereich von 0.1 mg/l und 10 mg/l.

Nach mehrtägiger Inkubation, vorzugsweise aber nach 2 bis 3tägiger Inkubation bei einer Temperatur von 20 °C bis 40 °C, vorzugsweise von 23 °C bis 35 °C und ganz besonders bevorzugt von 25 °C und diffusem Licht werden die Blattscheiben zur Sprossinduktion auf ein geeignetes Medium tranferriert. Besonders bevorzugt im Rahmen dieser Erfindung ist ein LS Medium, das kein Auxin enthält, zur Selektion der Transformanten aber mit einer Selektivsubstanz versetzt ist. Die Kulturen werden im Licht gehalten und in geeigneten Abständen, vorzugsweise aber in einwöchigen Intervallen auf frisches Medium übertragen. Sich entwickelnde grüne Sprosse werden ausgeschnitten und in einem Medium, das eine Bewurzelung der Sprosse induziert, weiterkultiviert. Besonders bevorzugt im Rahmen dieser Erfindung ist ein LS Medium, das kein Auxin und kein Cytokinin enthält, zur Selektion der Transformanten aber mit einer Selektivsubstanz versetzt ist.

Neben der *Agrobacterium*-vermittelten Transformation sind im Rahmen dieser Erfindung für die Einschleusung der erfindungsgemässen Genkonstruktionen in pflanzliches Material auch direkte Transformationsverfahren anwendbar.

So kann das genetische Material, das in einem Vektor enthalten ist, beispielsweise mit Hilfe rein physikalischer Massnahmen direkt in die pflanzliche Zelle eingeschleust werden, wie z.B. durch Mirkoinjektion unter Verwendung fein ausgezogener Mikropipetten [Neuhaus *et al* (1987)] oder durch Beschuss der Zellem mit Mikroprojektilen, die mit der transformierenden DNA beschichtet sind ["Microprojectile Bombardment"; Wang Y-C *et al* (1988)].

Weitere Möglichkeiten für den direkten Transfer von genetischem Material in die pflanzliche Zelle bestehen in der Behandlung der Protoplasten mit die Plasmamembran modifizierenden Verfahrensmassnahmen, wie z.B. der Polyethylenglykol Behandlung, der Hitzeschockbehandlung oder der Elektroporation sowie einer Kombination dieser Verfahrensmassnahmen [Shillito *et al* (1985)].

Bei der Elektroporations-Technik werden pflanzliche Protoplasten zusammen mit Plasmiden, die die hybride Genkonstruktion enthalten, elektrischen Impulsen hoher Feldstärke ausgesetzt. Dies führt zu einer reversiblen Permeabilitätserhöhung von Biomembranen und ermöglicht damit die Einschleusung der Plasmide. Elektroporierte pflanzliche Protoplasten erneuern ihre Zellwand, teilen sich und bilden Kallusgewebe. Die Selektion der transformierten Pflanzenzellen kann mit Hilfe der zuvor beschriebenen phaenotypischen Marker erfolgen.

Ein weiteres Verfahren für die direkte Einführung von gene tischem Material in Pflanzenzellen, das auf rein chemischen Verfahrensmassnahmen beruht und eine sehr effiziente und schnelle Durchführung der Transformation ermöglicht, ist bei Negrutiu I *et al*(1987) beschrieben.

Ebenfalls geeignet für die Transformation pflanzlichen Materials ist der direkte Gentransfer unter Verwendung der Co-Transformation (Schocher RJ *et al* 1986).

Bei der Co-Transformation handelt es sich um eine Methode, die auf der gleichzeitigen Aufnahme und Integration verschiedener DNA-Moleküle (nicht-selektionierbares und selektionierbares Gen) ins pflanzliche Genom beruht, und die daher das Auffinden von Zellen, die mit nicht selektierbaren Genen transformiert sind, ermöglicht.

Die zuvor gegebene beispielhafte Auflistung möglicher Transformationsverfahren erhebt keinen Anspruch aufVollständigkeit und soll den Erfindungsgegenstand in keiner Weise einschränken.

Diejenigen Zellklone, die die hybride Genkonstruktion in ihr Genom eingebaut enthalten werden mit Hilfe üblicher Selektions-, Screening- und Nachweismethoden ausgelesen und für die Regeneration transgener Pflanzen verwendet.

Die Regeneration von in Kultur gehaltenen Protoplasten zu ganzen Pflanzen ist z.B. bei Potrykus I und Shillito RD (1986) beschrieben.

Die Regenerationsverfahren unterscheiden sich von Pflanzenspezies zu Pflanzenspezies. Im allgemeinen aber werden die Protoplasten in einem der bekannten Kulturmedien zur Teilung und Zellwandbildung angeregt. Es entstehen schliesslich Kalluskulturen, welche durch Behandlung mit bestimmten Wirkstoffen, wie z.B. Auxinen und Cytokininen zur Wurzel- bzw. Sprossbildung induziert werden können.

Die so gewonnenen Pflänzchen können anschliessend in Erde übertragen und in gleicher Weise wie normale Sämlinge weiterkultiviert werden.

Eine effiziente Regeneration hängt in erster Linie vom Medium, vom Genotyp und von der Vorgeschichte der Kultur ab. Werden diese drei Variablen hinreichend kontrolliert, dann ist die Regeneration vollständig reproduzier- und wieder holbar.

Die regenerierten transgenen Pflanzen, welche ein in der pflanzlichen Zelle exprimierbares Strukturgen der zuvor beschriebenen hybriden Genkonstruktion als integralen Bestandteil des pflanzlichen Genoms enthalten, kann vegetativ vermehrt werden, vorzugsweise in Form steriler Spross-Kulturen.

Die stabile Integration eines funktionsfähigen exprimierbaren Gens ins pflanzliche Genom der regenerierten transgenen Pflanzen wird anhand der mitotischen Stabilität des integrierten Gens sowie aufgrund des Verhaltens als Mendelsches Merkmal während der Meiose und unter Verwendung der "Southern blot" Analyse (Southern EM, 1975) verifiziert.

Ebenfalls umfasst vom breiten Konzept dieser Erfindung ist somit transgenes pflanzliches Material, ausgewählt aus der Gruppe bestehend aus Protoplasten, Zellen, Kalli, Geweben, Organen, Samen, Embryonen, Pollen, Eizellen, Zygoten, etc., einschliesslich ganzer Pflanzen, insbesondere aber ganzer, fertilern Pflanzen, das mit Hilfe der zuvor beschriebenen Verfahren transformiert worden ist und die erfindungsgemässe rekombinante DNA in exprimierbarer Form enthält sowie Verfahren zur Herstellung besagten transgenen pflanzlichen Materials.

Das Verfahren zur Herstellung von transformiertem pflanzlichem Material, welches zumindest in einem Teil seiner Zellen eines der erfidungsgemässen rekombinanten DNA Moleküle enthält, ist im wesentlichen dadurch charakterisiert, dass man:

(a) zunächst die für die für eine Überexpression verantwortliche DNA-Sequenz aus einer geeigneten Quelle isoliert oder diese mit Hilfe bekannter Verfahren synthetisiert;

(b) besagte DNA-Sequenz in das 5′-terminale Ende einer beliebigen exprimierbaren DNA-Sequenz operabel inseriert;

(c) das fertige Konstrukt in einen Pflanzenexpressionsvektor unter die Kontrolle von in Pflanzen aktiven Expressionssignalen einkloniert; und

(d) besagten Expressionsvektor mit Hilfe bekannter Verfahren in pflanzliches Material transformiert und dort exprimiert.

Ebenfalls umfasst vom breiten Konzept dieser Erfindung sind somit transgene Pflanzen, insbesondere aber transgene fertile Pflanzen, die mit Hilfe des zuvor beschriebenen erfindungsgemässen Verfahrens transformiert worden sind sowie deren asexuelle und/oder sexuelle Nachkommenschaft, welche noch die durch die Transformation der Mutterpflanze bedingte(n) neue(n) und wünschenswerte(n) Eigenschaft(en) aufweisen.

Unter den Begriff der asexuellen und/oder sexuellen Nachkommenschaft transgener Pflanzen fallen definitionsgemäss im Rahmen dieser Erfindung somit auch alle Mutanten und Varianten die mit Hilfe bekannter Verfahren, wie z.B. durch Zellfusionen oder Mutantenselektion gewonnen werden können, und welche noch die charakteristischen Eigenschaften der transformierten Ausgangspflanze aufweisen, sowie sämtliche Kreuzungs-und Fusionsprodukte mit dem transformierten Pflanzenmaterial.

Bevorzugt im Rahmen dieser Erfindung sind transgene Pflanzen, insbesondere fertile transgene Pflanzen, die einen gegenüber dem Wildtyp erhöhten Proteingehalt in den transformierten Zellen und/oder Geweben aufweisen sowie deren sexuelle und asexuelle Nachkommenschaft.

Besonders bevorzugt im Rahmen dieser Erfindung sind transgene Pflanzen, insbesondere fertile transgene Pflanzen, die mit einem rekombinanten DNA Molekül transformiert sind, das eine chimäre genetische Konstruktion enthält, worin die erfindungsgemässe, regulatorische DNA-Sequenz mit einem Strukturgen, das Chitinase- und/oder Glucanase kodiert, sowie mit weiteren, in pflanzlichen Zellen aktiven Expressionssignalen, operabel verknüpft ist, sodass der Chitinase und/oder Glucanasegehalt in der Pflanze aufgrund der stark erhöhten Expressionsrate des assoziierten Strukturgens gegenüber dem Wildtyp signifikant erhöht ist.

Ganz besonders bevorzugt sind transgene Pflanzen, insbesondere fertile transgene Pflanzen, die einen Chitinasegehalt zwischen 400 µg/g Frischgewicht (FG) und 1200 µg/g Frischgewicht (FG) aufweisen.
Ebenso bevorzugt sind transgene Pflanzen, insbesondere fertile transgene Pflanzen, die einen Glucanasegehalt zwischen 20 µg/g Frischgewicht (FG) und 130 µg/g Frischgewicht (FG) aufweisen.

Ein weiterer Gegenstand dieser Erfindung betrifft das Vermehrungsgut transgener Pflanzen.

Unter Vermehrungsgut transgener Pflanzen soll im Rahmen dieser Erfindung jedes beliebige pflanzliche Material verstanden werden, das sich auf sexuellem oder asexuellem Wege bzw. *in-vivo* oder *in-vitro* vermehren lässt. Als besonders bevorzugt sind im Rahmen dieser Erfindung in erster Linie Protoplasten, Zellen, Kalli, Gewebe, Organe, Samen, Embryonen, Pollen, Eizellen, Zygoten anzusehen, sowie jedes beliebige andere Vermehrungsgut das von transgenen Pflanzen erhalten werden kann.

Pflanzenteile, wie z.B. Blüten, Stengel, Früchte, Blätter, Wurzeln, die von transgenen Pflanzen oder deren Nachkommen stammen, die zuvor mit Hilfe des erfindungsgemässen Verfahrens transformiert worden sind und die somit wenigstens zum Teil aus transgenen Zellen aufgebaut sind, sind ebenfalls Gegenstand dieser Erfindung.

Das erfindungsgemässe Verfahren eignet sich für die Transformation aller Pflanzen, insbesondere solchen der systematischen Gruppen *Angiospermae* und *Gymnospermae*.

Unter den *Gymnospermae* sind von besonderem Interesse die Pflanzen der Klasse *Coniferae*.

Unter den *Angiospermae* sind neben den Laubbäumen und Sträuchern von besonderem Interesse Pflanzen der Familien *Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae, Malvaceae* oder *Gramineae* und der Ordnung *Leguminosae* und hier vor allem der Familie *Papilionaceae*. Bevorzugt sind Vertreter der Familien *Solanaceae, Cruciferae* und *Gramineae*.

Zu den Zielkulturen im Rahmen der vorliegenden Erfindung zählen beispielsweise auch jene, ausgewählt aus der Reihe: *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Gossypium, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum, Sorghum, Ipomoea, Passiflora, Cyclamen, Malus, Prunus, Rosa, Rubus, Populus, Santalum, Allium, Lilium, Narcissus, Ananas, Arachis, Phaseolus* und *Pisum*.

Aufgrund neuer Enwicklungen auf dem Gebiet der *in vitro* Kultivierung von Pflanzen, vornehmlich im Bereich der Pflanzenregeneration, ist es inzwischen möglich, auch bei Vertretern aus der Familie der *Gramineae*, ausgehend von pflanzlichen Protoplasten, ganze Pflanzen zu regenerieren. Beispiele von erfolgreich durchgeführten Regenerationsexperimenten bei Gramineen sind u.a. bei Yamada Y *et al* (1986) für Reisprotoplasten, bei Rhodes *et al* (1988) und Shillito RD *et al* (1989) für Maisprotoplasten sowie bei Horn *et al* (1988) für *Dactylis glomerata* Protoplasten beschrieben.

Es können im Rahmen der vorliegenden Erfindung daher auch die folgenden Pflanzen verwendet werden: *Lolium, Zea, Triticum, Sorghum, Saccharum, Bromus, Oryzae, Avena, Hordeum, Secale* und *Setaria*.

Die reifen Pflanzen, die aus transformierten Pflanzenzellen herangezogen wurden, werden zur Samenproduktion mit sich selbest gekreuzt. Einige der Samen enthalten die Gene, die für eine nützliche und wünschenswerte Eigenschaft kodieren in einem Verhältnis, das genau den etablierten Gesetzen der Vererbung gehorcht. Diese Samen können zur Produktion transgener Pflanzen verwendet werden.

Homozygote Linien können durch wiederholte Selbstbestäubung und Herstellung von Inzuchtlinien gewonnen werden. Diese Inzuchtlinien können dann wiederum zur Entwicklung von Hybriden verwendet werden. Bei diesem Verfahren wird eine Inzuchtlinie, die besagtes Fremdgen enthält mit einer anderen Inzuchtlinie zur Produktion gekreuzt.

Nach der allgemeinen Beschreibung der vorliegenden Erfindung soll nun zum besseren Verständnis auf spezifische Ausführungsbeispiele Bezug genommen werden, die zum Zwecke der Illustration in die Beschreibung mitaufgenommen werden, und die keinen limitierenden Charakter haben, es sei denn, es wird speziell darauf hingewiesen.

Nichtlimitierende Ausführungsbeispiele:

Allgemeine rekombinante DNA-Techniken

Da viele der in dieser Erfindung genutzten rekombinanten DNA-Techniken Routine für den Fachmann sind, ist eher hier eine kurze Beschreibung dieser allgemein gebrauchten Techniken enthalten als jedesmal dort, wo sie erscheinen. Bis darauf, wo extra darauf hingewiesen wird, sind alle diese Verfahren in der Referenz von Maniatis *et al* (1982) beschrieben.

A. Schneiden mit Restriktionsendonukleasen

Typischerweise sind etwa 50 bis 500 µg/ml DNA in dem Reaktionsansatz enthalten, in der vom Hersteller, New England Biolabs, Beverly, MA., empfohlenen Pufferlösung. 2 bis 5 Einheiten von Restriktionsendonukleasen werden für jedes ug DNA hinzugefügt und der Reaktionsansatz bei der vom Hersteller empfohlenen Temperatur für eine bis drei Stunden inkubiert.

Die Reaktion wird durch 10 minütiges Erhitzen auf 65° C oder durch Extraktion mit Phenol, gefolgt durch Präzipitation der DNA mit Ethanol, beendet. Diese Technikwird auch auf den Seiten 104 bis 106 der Maniatis *et al* (1982)-Referenz beschrieben.

B. Behandlung der DNA mit Polymerase, um glatte Enden zu erzeugen

50 bis 500 µg/ml DNA-Fragmente werden zu einem Reaktionsansatz hinzu- gefügt, in dem vom Hersteller, New England Biolabs, empfohlenen Puffer. Der Reaktionsansatz enthält alle vier Desoxynukleotidtriphosphate in Konzentrationen von 0.2 mM. Die Reaktion erfolgt während 30 Minuten bei 15° C und wird dann durch 10 minütiges Erhitzen auf 65° C beendet. Für Fragmente, die durch Schneiden mit Restriktionsendonukleasen erhalten werden, welche 5′-hervortretende Enden erzeugen, wie EcoRI und BamHI, wird das grosse Fragment, oder Klenow-Fragment, der DNA-Polymerase benutzt. Für Fragmente, die durch Endonukleasen erhalten werden, welche 3′-hervortretende Enden erzeugen, wie PstI und SacI, wird die T4-DNA-Polymerase benutzt. Die Verwendung dieser zwei Enzyme wird auf den Seiten 113 bis 121 der Maniatis *et al* (1982)-Referenz beschrieben.

C. Agarose-Gelelektrophorese und Reinigung von DNA-Fragmenten von Gelen

Die Agarose-Gelelektrophorese wird in einem horizontalen Apparat durch- geführt, wie auf den Seiten 150 bis 163 der Maniatis et al.-Referenz beschrieben. Der benutzte Puffer ist der dort beschriebene Tris-Borat- puffer. Die DNA-Fragmente werden durch 0.5 µg/ml Ethidiumbromid gefärbt, das entweder im Gel- oder Tankpuffer während der Elektrophorese vorhanden ist oder nach der Elektrophorese hinzugefügt wird. Die DNA wird durch Beleuchtung mit langwelligem Ultraviolettlicht sichtbar gemacht. Wenn die Fragmente vom Gel abgetrennt werden sollen, wird eine Agarose verwendet, die bei niedriger Temperatur geliert und von Sigma Chemical, St. Louis, Missouri, bezogen werden kann. Nach der Elektrophorese wird das gewünschte Fragment ausgeschnitten, in ein Plastikröhrchen gegeben, etwa 15 Minuten auf 65° C erhitzt, dreimal mit Phenol extrahiert und zweimal mit Ethanol gefällt. Dieses Verfahren ist gegenüber dem von Maniatis *et al* (1982) auf Seite 170 beschriebenen leicht verändert.

Als Alternative kann die DNA aus der Agarose mit Hilfe des Geneclean Kits (Bio 101 Inc., La Jolla, CA, USA) isoliert werden.

D. Hinzufügen von synthetischen Linkerfragmenten an DNA-Enden

Wenn es erwünscht ist, eine neue Endonukleaseschnittstelle an das Ende eines DNA-Moleküls anzufugen, wird das Molekül gegebenenfalls zuerst mit DNA-Polymerase behandelt, um glatte Enden zu erzeugen, wie im obigen Abschnitt beschrieben. Etwa 0.1 bis 1.0 µg dieses Fragments wird zu etwa 10 ng phosphorylierter Linker-DNA gegeben, die von New England Biolabs bezogen wurde, in einem Volumen von 20 bis 30 µl mit 2 µl T4 DNA-Ligase von New England Biolabs, und 1mM ATP in dem vom Hersteller empfohlenen Puffer. Nach einer Inkubation über Nacht bei 15° C wird die Reaktion durch 10 minütiges Erhitzen auf 65° C beendet. Der Reaktionsansatz wird auf etwa 100 µl in einem Puffer verdünnt, der richtig für die Restriktionsendonuklease ist, die die synthetische Linkersequenz schneidet. Ungefähr 50 bis 200 Einheiten dieser Endonuklease werden hinzugefügt. Die Mischung wird 2 bis 6 Stunden bei der angemessenen Temperatur inkubiert, dann wird das Fragment einer Agarose-Gelelektrophorese unterworfen und gereinigt wie oben beschrieben. Das resultierende Fragment wird nun Enden haben mit Endigungen, welche durch Schneiden mit der Restriktionsendonuklease erzeugt wurden. Diese Enden sind gewöhnlich kohäsiv, so dass das resultierende Fragment nun leicht an andere Fragmente mit den gleichen kohäsiven Enden geknüpft werden kann.

E. Entfernen von 5′-terminalen Phosphaten von DNA-Fragmenten

Während der Plasmidklonierungsschritte vermindert die Behandlung des Vektorplasmids mit Phosphatase die Rezirkularisation des Vektors (diskutiert auf Seite 13 der Maniatis *et al*-Referenz). Nach Schneiden der DNA mit der richtigen Restriktionsendonuklease wird eine Einheit alkalische Phosphatase aus dem Darm von Kälbern hinzugefügt, die von Boehringer-Mannheim, Mannheim, erworben wurde. Die DNA wirdeine Stunde bei 37° C inkubiert und anschliessend zweimal mit Phenol extrahiert und mit Ethanol gefällt.

F. Verknüpfen der DNA-Fragmente

Wenn Fragmente mit komplementären kohäsiven Enden miteinander verknüpft werden sollen, werden etwa 100 ng von jedem Fragment in einem Reaktions- gemisch von 20 bis 40 µl mit etwa 0.2 Einheiten T4 DNA-Ligase von New England Biolabs im vom Hersteller empfohlenen Puffer inkubiert. Die Inkubation wird 1 bis 20 Stunden lang bei 15° C durchgeführt. Wenn DNA-Fragmente mit glatten Enden verknüpft werden sollen, werden sie inkubiert wie oben, bis darauf, dass die Menge der T4 DNA-Ligase auf 2 bis 4 Einheiten erhöht wird.

G. Die Transformation von DNA in *E. coli*

*E. coli* Stamm HB101 wird für die meisten Experimente verwendet. DNA wird mit dem Kalziumchloridverfahren, wie es von Maniatis *et al* (1982), Seiten 250 bis 251, beschrieben wurde, in *E. coli* eingeführt.

H. Screening von *E. coli* auf Plasmide

Nach der Transformation werden die resultierenden Kolonien von *E. coli* auf das Vorhandensein des gewünschten Plasmids durch ein schnelles Plasmidisolationsverfahren geprüft. Zwei gebräuchliche Verfahren werden auf den Seiten 366 bis 369 der Maniatis *et al* (1982)-Referenz beschrieben.

I. Isolierung von Plasmid-DNA in grossem Massstab

Verfahren zur Isolierung von Plasmiden aus *E. coli* in grossem Massstab werden auf den Seiten 88 bis 94 der Maniatis *et al* (1982)-Referenz beschrieben.

J. Klonierung in M13 Phagenvektoren

Die folgende Beschreibung ist so zu verstehen, dass die doppelsträngige replikative Form der Phage M13-Abkömmlinge für Routineverfahren, wie Schneiden mit Restriktionsendonuklease, Verknüpfen etc., benutzt wird.

Enzyme können, wenn nicht extra darauf hingewiesen wird, von Boehringer, Biolabs (BRL), bezogen werden. Sie werden entsprechend den Angaben des Herstellers verwendet, es sei denn, es wird gesondert darauf hingewiesen.

K. Southern blot-Analyse

Die extrahierte DNA wird dabei zunächst mit Restriktionsenzymen behandelt, anschliessend einer Elektrophorese in einem 0,8%igen bis 1%igen Agarose-Gel unterworfen, auf eine Nitrozellulosemembran [Southern EM (1975)] transferiert und mit der nachzuweisenden DNA, welche zuvor einer Nick Translation unterworfen wurde (DNA-spezifische Aktivitäten von $5 \times 10^8$ bis $10 \times 10^8$ c.p.m./$\mu$g), hybridisiert. Im vorliegenden Fall dient ein 3′ AluI-PstI Fragment des Tabakchitinase cDNA Klons pCHN50 [Shinshi *et al* - (1987)], das unter Verwendung eines 'Random Primer' Markierungskits [Boehringer Mannheim] $^{32}$P markiert werden kann, als Hybridisierungsprobe. Die Filter werden dreimal je eine Stunde lang mit einer wässrigen Lösung von 0.03 M Natriumcitrat und 0.3 M Natriumchlorid bei 65 °C gewaschen. Die hybridisierte DNA wird durch Schwärzung eines Röntgenfilms während24 bis 48 Stunden sichtbar gemacht.

L. Western Blot Analyse

Nach einer SDS-Polyacrylamidgelelektrophorese werden die Proteine elektrophoretisch auf einen Nitrocellulose- oder Nylonfilter übertragen. Dieser Filter wird dann zunächst mit einem blockierenden Agens (z.B. 5% Magermilchpulver in PBS: Milch-PBS) vorbehandelt. Anschliessend wird der Filter mehrere Stunden mit einem Antiserum inkubiert, das mit der jeweils nachzuweisenden Verbindung (im vorliegenden Fall: Kaninchen anti-Tabak-Chitinase IgG) reagiert. Der auf diese Weise vorbehandelte Filter wird mehrmals mit Milch-PBS gewaschen und dann mit einem kommerziell erhältlichen sekundären Antikörper, der mit einem Enzym gekoppelt ist [z.B. Peroxidase gekoppelte Ziegen-anti-Kaninchen Antikörper (BIORAD), 1:2000 verdünnt in Milch-PBS], inkubiert. Der Filter wird nochmals in PBS gewaschen und anschliessend gemäss den Angaben des Herstellers [BIORAD] mit Chloronaphtol und Wasserstoffperoxid angefärbt. Weiter Details sind in Sambrook *et al* (1989) angegeben.

M. Northern Blot Analyse

Gesamt RNA kann nach der bei Logemann *et al* (1987) beschriebenen Methode durchgeführt werden, wobei jedoch vorteilhafterweise ein zusätzlicher Präzipitationsschritt mit 2 M LiCl zwischengeschaltet wird um eventuell kontaminierende DNA restlos zu beseitigen. Die RNA (0.4 $\mu$g oder 4 $\mu$g) wird anschliessend in einem 1% Agarosegel, enthaltend 6.7% Formaldehyd [Maniatis *et al* (1982)], elektrophoretisch fraktioniert. Die einzelnen Fraktionen werden auf 'Zeta-Probe' Membranen [BIORAD] übertragen und mit einem $^{32}$P-markierten PstI Insert aus dem Tabakchitinase cDNA Klon pCHN48 hybridisiert. Die Grösse der hybridisierenden DNA kann anhand des mitlaufenden Standards abgeschätzt werden.

N. Bestimmung der Enzymaktivität

Die Chitinaseaktivität wird radiometrisch unter Verwendung von $^3$H-markiertem Chitin [Spez. Aktivität $4.93 \times 10^5$ dpm/Mol N-Acetylglucosamin] als Substrat bestimmt [Boller *et al* (1983)].

Die α-Mannosidaseaktivität wird in Mikrotiterplatten unter Verwendung von p-Nitrophenol als Substrat bestimmt [Boller und Kende (1979)].
Die Peroxidaseaktivität wird unter Verwendung von Guaiacol als Substrat bestimmt [Siegel und Galston (1967)].

Zur Illustration der eher allgemeinen Beschreibung sowie zum besseren Verständnis der vorliegenden Erfindung soll nunmehr auf spezifische Ausfuhrungsbeispiele Bezug genommen werden, die keinen limitierenden Charakter haben, es sei denn, es wird speziell darauf hingewiesen. Das gleiche gilt auch für alle beispielhaften Aufzählungen, die in der vorangegangenen Beschreibung enthalten sind.

## I. Herstellung von cDNA- und genomischen Genbibliotheken

### Beispiel 1: Pflanzenmaterial

*Nicotiana tabacum* L. c.v. Havana 425 Pflanzen werden entweder im Gewächshaus oder aber ausgehend von Oberflächensterilisierten Samen herangezogen.

Die Oberflächensterilisation der Samen wird folgendermassen durchgeführt:
20 bis 30 Samen werden in ein Sieb mit einer Porengrössen von ca. 200 µm gegeben und in 10 ml einer 10%igen, kommerziell erhältlichen Bleichlösung (NaOCl) inkubiert. Die Samen werden anschliessend mehrmals mit sterilem destilliertem Wasser abgespült.

In den folgenden Ausführungsbeispielen wird vorrangig eine klonierte Linie parenchymatischen Markgewebes (N) verwendet, die gemäss Eichholz *et al* (1983) aus *Nicotiana tabacum* L. c.v. Havana 425 Pflanzen isoliert werden kann.

### Beispiel 2: Gewebekultur

Das Tabakgewebe wird auf einem Basismedium kultiviert, das eine Salz- und Thiamin-HCl-Konzentration nach Linsmeier und Skoog (1965) (LS) aufweist und durch Zusatz von 10 g/l Agar verfestigt ist. Als weiteren Zusatz enthält dieses Basis medium einen pH-Indikator, wie beispielsweise 5 mg/l Chlorphenolrot. Weitere Medien, die auf diesem LS Basismedium aufbauen und in den sich anschliessenden Ausführungsbeispielen zur Anwendung kommen, enthalten als weitere Zusätze z.B. Kinetin (1.4 µM) [Cytokinin-Medium] oder α-Naphthylessigsäure (10.7 µM) [Auxin-Medium] oder aber ein Gemisch aus Kinetin und α-Naphtylessigsäure [Auxin/Cytokinin-Medium (Medium A)].

Die Selektivmedien B und C enthalten kein α-Naphtylessigsäure, dafür aber eine Selektivsubstanz, mit deren Hilfe die Transformanten aus der grossen Anzahl nicht transformierter Zellen und Gewebe ausgelesen werden können. Die genaue Zusammensetzung dieser Selektivmedien ist in Abschnitt VII (Medien) wiedergegeben.

Die aus *Nicotiana tabacum* L. c.v. Havana 425 Pflanzen isolierte Stamm-Linie (275N) wird in Intervallen von 21 Tagen auf einem Auxin/Cytokinin-Medium (10 ml) subkultiviert, d.h. jeweils auf neues Medium (10 ml) überimpft und bei 25° C im Licht kultiviert.

Zur Induktion hoher Chitinase-Spiegel in den kultivierten Geweben werden diese von dem Auxin/Cytokinin-Medium auf ein Hormon-freies Basismedium überimpft. Weitere Einzelheiten zur Kultivierung von Pflanzengeweben und zur Induktion von Chitinase sind bei Felix und Meins (1985)beschrieben.

### Beispiel 3: Herstellung von Tabakprotoplasten

100 ml einer 2 Tage alten Tabak Zellsuspension gemäss Beispiel 1 wird mit einem gleichen Volumen einer doppelt konzentrierten Enzymlösung gemischt. Die Enzymlösung enthält die folgenden Bestandteile:

| | |
|---|---|
| Cellulase R.10 Onozuka® (Yakult Honsha, Tokyo, Japan) | 10.0 g/l |
| Macerase (Pektinase from Rhizopus sp., Behring Diagnostics, La Jolla, CA) | 2.5 g/l |
| Pectolyase Y-23® (Seishin Pharm. Co., Tokyo, Japan) | 1.0 g/l |
| $CaNO_3 . 4H_2O$ | 1.45 g/l |
| $MgSO_4 . 7H_2O$ | 0.5 g/l |
| $NH_4H_2PO_4$ | 0.23 g/l |
| $KNO_3$ | 1.2 g/l |
| D-Mannit (pH 5.70) | 73.0 g/l |

Obige Enzymlösung wird zentrifugiert und durch ein 0.2 µm Filter sterilisiert.

Der Ansatz bestehend aus Tabak-Suspensionskultur und Enzymlösung wird 5 Stunden bei Zimmertemperatur auf einer Rundschüttelmaschine (40 Upm) vorsichtig bewegt. In bestimmten Zeitabständen werden aus diesem Ansatz Proben entnommen und mikroskopisch analysiert. Die enzymatische Verdauung wird solange fortgesetzt, bis ca. 80% der Zellen in spärische Protoplasten umgewandelt sind. Die Inkubationsgefässe werden dann von der Schüttelmaschine genommen. Man lässt die Zell-/Protoplastensuspension absetzten und entfernt die obere Hälfte des Mediums, die keine Zellen oder Protoplasten enthält durch Absaugen mit einer Pipette und verwirft diese. Der Rest wird in 50 ml Zentrifugenröhrchen überführt und 10 Minuten bei 500 Upm in einer klinischen Zentrifuge (Modell HN-SII, IEC) zentrifugiert. Die Protoplastenhaltigen Pellets werden in einer Rinse I-Lösung [verlgeiche Abschnitt VII] resuspendiert und anschliessend erneut 10 Minuten bei 1'000 Upm zentrifugiert.

Die Bande mit den Protoplasten befindet sich am oberen Rand des Zentrifugenröhrchens. Die Protoplastenfraktion wird gesammelt und anschliessend erneut in Rinse I-Lösung gewaschen. Die Protoplasten werden in ein Zentrifugenröhrchen zurücküberführt und bis zur weiteren Verwendung in Eis aufbewahrt.

## Beispiel 4: Konstruktion einer cDNA Genbibliothek

Die cDNA-Genbibliotek wird ausgehend von poly(A)+ RNA hergestellt, welche aus einer klonierten Linie parenchymatischen Markgewebes von *Nicotiana tabacum* L. c.v. Havana 425 Pflanzen (siehe Beispiel 1) erhältlich ist. Das Tabakgewebe wird zuvor gemäss Beispiel 2 zur Produktion hoher Chitinasespielgel angeregt, indem man dieses auf einem hormonfreien LS-Basismedium kultiviert.

### 4.1. Isolierung von Gesamt-RNA

Die Aufbereitung von Gesamt-RNA erfolgt im wesentlichen nach dem bei Lagrimini LM *et al* (1987) beschriebenen Verfahren.

In flüssigem Stickstoff tiefgefrorenes Tabakgewebe wird zunächst in einem Mörser grob zerstossen und dann in einen geeigneten Homogenisations-Puffer [(1) 7.56 M Guanidin HCl, 0.73 M Mercaptoethanol, 18.9 mM Natriumacetat pH 5.0; oder (2) 4% (w/v) SDS, 0.1 M Tris-HCl pH 7.8 (1 Volumenteil) + 80% Phenol (v/v), 0.1% (w/v) Hydroxyquinolin, 0.1 M Tris-HCl pH 7.8 (1 Volumenteil); oder (3) gemäss Lagrimini LM *et al*, (1987)] gegeben (2.5 ml Puffer pro Gramm Gewebe). Nach Zugabe eines gleichen Volumensteils Phenol wird dieser Ansatz homogenisiert, z.B in einem Polytron Homogenisator. Anschliessend wird ein halbes Volumen Chloroform zugesetz und die Emulsion für etwa 15 Minuten vorsichtig durchmischt. Die verschiedenen Phasen werden dann über eine Zentrifugation (10,400 g für 10 Minuten) voneinander getrennt; die wässrige Phase wird verworfen. An dieser Stelle können wahlweise weitere Extraktionsschritte angeschlossen werden, z.B. in Form einer zusätzlichen Phenol-Chloroform Extraktion oder einer zweimaligen Extraktion mit einem Gemisch aus Phenol:Chloroform:Isoamylalkohol (25:24:1).

An die Extraktion schliesst sich der Präzipitationsschritt an. Dieser erfolgt durch Zugabe von 0.3 M Natriumacetat und 2.5 Volumenteilen Ethanol. Das Präzipitat wird durch Zentrifugation gesammelt (10.400 g für ca. 15 Minuten) und in 2 ml sterilem Wasser resuspendiert. Nach Zugabe von Lithiumchlorid in einer Endkonzentration von 3 M wird der gesamte Ansatz bei 4 °C über Nacht inkubiert. Das Präzipitat wird dann erneut durch Zentrifugation gesammelt und das gebildete Pellet mit eisgekühltem Ethanol gewaschen. Das Pellet wird anschliessend getrocknet und in 500 µl sterilemwasser resuspendiert. Die Konzentration der Gesamt-RNA in dieser Präparation wird spektrophotometrisch bestimmt.

Alternativ zu dem zuvor beschriebenen Verfahren kann die Gesamt-RNA auch aus Kallusgewebe isoliert werden. Auch in diesem Fall kommen die zuvor beschriebenen Verfahrensschritte zur Anwendung, wobei als Ausgangsmaterial jedoch in Würfel geschnittenes Kallusgewebe (ca. 3 mm) verwendet wird, das vor dem Homogenisations-Schritt zunächst in flüssigem Stickstoff tiefgefroren und anschliessend in einem vorgekuhlten Mörser zu einem freinen Pulver zerstossen wird.

4.2. Isolierung polyadenylierter RNA

Poly(A)+ RNA wird mit Hilfe einer Oligo-d(T) Cellulosechromatographie (Collaborative Research, Lexington, MA, USA) gemäss an sich bekannter Verfahren isoliert [siehe z.B. Mohnen (1979)].

Oligo-d(T) Cellulose wird zunächst 15 Minuten in 20 Volumenteilen 2.0 M NaCl gewaschen, anschliessend in sterilem Wasser suspendiert und auf eine Säule (1.5 cm Durchmesser und 20 cm Länge) gepackt. Die Säule wird mit einer Pufferlösung (440 mM NaCl, 0.9 mM EDTA, 9 mM Tris-HCl, pH 7.5; oder 0.5 M NaCl, 10 mM Tris-HCl, pH 7.5) gewaschen bis das Eluat einen pH-Wert zwischen 7.0 und 7.6 aufweist. Anschliessend werden RNA Lösungen mit einem RNA-Anteil zwischen 0.6 mg und 6.0 mg RNA in einem Volumen von 4.0 ml auf eine Endkonzentration von 1 mM EDTA und 10 mm Piperazin-1,4-bis(2-ethansulfonsäure), pH7.5 eingestellt. Die RNA wird durch 5 minütiges Erhitzen bei 70 °C und anschliessendes Abkühlen auf Eis denaturiert. Die gesamte Lösung wird dann mit 0.1 Volumenteilen 4 M NaCl auf einen Wert von 0.36 M NaCl eingestellt. Die RNA Lösung wird auf die Säule gegeben und die nicht polyadenylierte RNA [poly (A)- RNA] mit der oben erwähnten Pufferlösung eluiert. Die Absorption der Eluate wird mit Hilfe eines Spectrophotometers (Hitachi Modell 100-40, Hitachi, Tokyo, Japan) sowie eines angeschlossenen W+W Rekorders (Scientific Instruments, Basel, Schweiz) bestimmt. Sobald die Absorption die Basislinie erreicht hat, wird die an die Säule gebundene poly (A)+ RNA mit 1 mM EDTA, 10 mM Tris-HCl pH 7.5 eluiert. Die Eluate werden in eine Lösung aus 0.5 mM EDTA und 0.3 M Natriumacetat pH 5.0 gegeben und durch Zugabe von 2.5 Volumenteilen Ethanol präzipitiert. Die RNA wird anschliessend durch Zentrifugation bei 83'000 x g (30 bis 45 Minuten) gesammelt, unter Stickstoff getrocknet und in 1 mM EDTA, 10 mM Tris, pH 7.5 oder in Wasser resuspendiert.

4.3. Konstruktion und Selektion von cDNA Klonen

Poly(A)+ RNA wird mit Hilfe einer präparativen Ultrazentrifugation (17 Stunden bei 57'000 g) über einen 5-25%igen (w/v) Saccharosegradienten (1 mM EDTA; 10 mM Tris-HCl, pH 7.5) in einzelne Fraktionen zerlegt. Die Fraktionen, welche die Infomation für Chitinase enthalten, lassen sich nach einer *in vitro* Translation durch anti-Chitinase Antikörper identifizieren. Diese werden dann vereinigt und für die weitere Aufarbeitung verwendet.

Das Vorgehen für die Herstellung von anti-Chitinase Antikörpern ist dem Fachmann bekannt und kann beispielsweise gemäss dem bei Shinshi *et al* (1985) bzw. bei Mohnen (1985) beschriebenen Verfahren durchgeführt werden. Dabei werden im wesentlichen Emulsionen mit gereinigten Chitinasepräparationen in komplettem Freunds Adjuvans in drei Monate alte weibliche Kaninchen (New Zealand white rabbit) injiziert. Weitere Injektionen folgen nach einer bzw. zwei Wochen sowie anschliessend in monatlichen Intervallen. Die Blutentnahme erfolgt jeweils 7 bis 10 Tage nach der Injektion, wobei die Serumproben bei -20 °C aufbewahrt werden. Immunglobulin G wird über eine Affinitätschromatographie an einer Protein A-Sepharose C1 4B Säule (Pharmacia) gereinigt, anschliessend lyophilisiert und bei -20 °C aufgehoben.

Die Synthese doppelsträngiger DNA ausgehend von der poly(A)+ RNA Matritze, die Klonierung dieser doppelsträngigen DNA in pBR322, die differentielle Koloniehybridisierung und die Plasmid Isolierung werden im wesentlichen gemäss den Vorschriften und der Beschreibung bei Maniatis *et al* (1982) durchgeführt.

Für die Synthese von ca. 0.8 µg doppelsträngiger DNA werden 3 µg der zuvor isolierten und mit Chitinase mRNA angereicherten poly(A)+ RNA mit reverser Transkriptase (Life Sciences, St. Petersburg, FL) sowie DNA Polymerase I (New England Biolabs, Beverly, MA) inkubiert. Die so gebildete cDNA wird mit Hilfe der ,Homopolymeric dC-dG Tailing 'Methode' die es erlaubt die cDNA und die Vektor DNA mit komplementären kohäsiven Enden zu versehen und die bei Maniatis *et al* (1982) [Seite 217 - 219] im einzelnen beschrieben ist, in die PstI Schnittstelle von pBR322 eingespleisst. Der mit PstI linearisierte umd mit oligo-dC Enden versehene Vektor pBR322 ist kommerziell erhältlich.

Das resultierende rekombinante Plasmid wird dann für die Transformation kompetenter *E.coli* DH1 Zellen verwendet. Die Klonierung in Plasmiden ist bei Maniatis *et al* (1982) auf den Seiten 242-246 und 391 im Detail beschrieben.

Die auf diese Weise konstruierte cDNA Bibliothekt, die in Form von Bakterienkolonien auf Agarplatten vorliegt, wird dann zunächst mit Hilfe der differentiellen Koloniehybridisierung unter Verwendung radioaktiv marikierter cDNA gescreent.

Im Rahmen der differentiellen Koloniehybridisierung werden von den Bakterienkolonien Duplikate auf Nitrocellulosefiltern hergestellt, indem man die Filter auf die Agarplatte auflegt und dann vorsichtig wieder abzieht. Die Original-Agarplatte wird für die spätere Identifizierung positiver Kolonien aufbewahrt. Die mit Bakterienkolonien behafteten Filter werden anschliessend auf ein Nährmedium gelegt und dort belassen, bis die Kolonien zu ca. 2 mm grossen Klonen ausgewachsen sind. Die Filter werden dann mit Natronlauge behandelt, was zu einer Lyse der Bakterienzellen und zur Denaturierung und Fixierung der bakteriellen DNA auf dem Filter führt. Nach pH-Neutralisierung werden die Filter mehrmals gewaschen, getrocknet und schliesslich bei einer Temperatur von 80 ° C unter Vakuum "gebacken", wodurch die DNA kovalent an den Filter gebunden wird.

Die Filter werden 2 mal hintereinander mit radioaktiv markierten (nicht-klonierten) cDNA Sonden hybridisiert. Bei diesen cDNA Sonden handelt es sich um poly(A)+ RNA aus Tabakgewebe das zuvor zur Produktion von Chitinase induziert wurde (siebentägige Inkubation auf Basalmedium ohne Hormonzusätze) sowie um poly(A)+ RNA aus nicht-induziertem Tabakgewebe (sieben tägige Inkubation auf Auxin-Cytokinin Medium). Vielversprechende cDNA Klone, die stärker mit der cDNA aus induziertem Gewebe reagieren als mit der Kontroll-DNA aus nicht-induziertem Gewebe, werden dann mit Hilfe des 'Hybrid-select' Translationsverfahrens gemäss der Beschreibung bei Mohnen *et al* (1985) und Mohnen (1985) weiter analysiert. Die Plasmid DNA wird durch einminütiges Kochen in 0.2 M - 0.3 M NaOH, 3 M NaCl denaturiert und anschliessend auf Eis abgekühlt. Die Hybridisierungsreaktion wird auf quadratischen BA/85 Nitrocellulosefiltern (Schleicher und Schüll, Dassel, FRG) mit 200 µg bis 250 µg Gesamt-RNA pro Filter, durchgeführt. Die auf den Filtern hybridisierte RNA wird eluiert, mit Ethanol präzipitiert und in 10 µl Wasser gelöst und mit Hilfe der *in vitro* Translation (mit Hilfe eines kommerziell erhältlichen Weizenkeimextraktes) analysiert. Die radioaktiv markierten Produkte werden mit Antikörpern gegen das gewünschte Protein gefällt und durch SDS-Polyacrylamid-Gelelektrophorese analysiert.

4.4. cDNA Klon pCHN48

Die cDNA Genbibliothek wird gemäss Maniatis *et al* (1982) mit Hilfe der Kolonie- bzw. Plaque-Hybridisierung gescreent. Als DNA Probe dient der bei Shinshi *et al* (1987) beschriebene cDNA Klon PCHN50, der die gesamte, das reife Protein-kodierende DNA Sequenz enthält, dem aber die komplette N-terminale Signalpeptidsequenz fehlt. Man erhält auf diese Weise verschiedene Klone unterschiedlicher Länge. Der längste dieser Klone wird ausgewählt und einer Nukleotidsequenzanalyse unterzogen. Dieser Klon mit der Bezeichnung pCHN48 besitzt ein 1.14 Kb umfassendes Insert mit 7 Adenosinen am Poly(A) Ende, einen einzigen grossen Leserahmen ('open reading frame') von 987 Nukleotiden Länge, entsprechend einem Polypeptid von 329 Aminosäuren sowie einem Nukleotid aus der 5'-nicht-translatierten Region. Die aus der DNA Sequenz der kodierenden Region ableitbare Aminosäuresequenz stimmt mit der Sequenz für die ersten 20 N-terminalen Aminosäuren bekannter Chitinasen aus Tabak überein.

Beispiel 5: Konstruktion einer genomischen Genbibliothek

5.1. Isolierung chromosomaler Tabak-DNA

Zur Lysierung der Protoplasten werden 100 ml eisgekühlte Protoplastensuspension mit 400 ml eines eisgekühlten TENP-Puffers(vgl. Abschnitt VII) vermischt. Die Kerne werden aus diesem Lysat mit Hilfe einer 10 minütigen Zentrifugation in einer IEC klinischen Zentrifuge bei 2'000 Upm abgetrennt. Das so erhältliche Pellet wird in 500 ml eisgekühltem TENP-Puffer resuspendiert und, wie zuvor beschrieben, erneut pelletiert. Anschliessend werden 8 CsCl Gradientenröhrchen vorbereitet, wobei in jedem der Röhrchen ein Zellpellet aus ca. 12.5 ml Protoplastensuspension zu 26 ml 10fach konzentrierten TE Puffers (vgl. Abschnitt VII) zugegeben wird. Zur Lyse der Zellkerne wird diesem Ansatz 5 ml einer 20%igen (w/v) Natriumlaurylsarkosin Lösung zugesetzt sowie 32.2 g CsCl und 2.89 ml Ethidumbromidlösung (EtBr; 10 mg/ml). Der gesamte Ansatz wird leicht durchmischt um das CsCl zu lösen.

Die so gewonnenen Lysate werden in Polyallomer-Röhrchen (Beckman VTi50, 39 ml Röhrchen) übertragen und bei 45'000 Upm und einer Temperatur von 20°C 16 Stunden lang in einem VTi50 Rotor (Beckman) zentrifugiert. Im UV-Licht fluoreszierende Banden werden mit Hilfe von 3 ml Spritzen mit 16 Gauge Nadeln aus dem Gradienten abgezogen und gesammelt. Die weitere Aufarbeitung der DNA erfolgt durch Wiederholung der zuvor beschriebenen CsCl/EtBr Gleichgewichtszentrifugation. Die fluoreszierenden Banden werden erneut gesammelt und anhaftendes EtBr wird in 6 aufeinanderfolgenden Extraktionsschritten unter Verwendung von Isopropanol, der mit 20 x SSC (vergl. Abschnitt VII) gesättigt ist (gleiches Volumen), entfernt. Aus dieser auf die zuvor beschriebene Weise gereinigten Gradientenlösung wird die DNA ausgefällt, indem man nacheinander 2 Volumenteile destilliertes Wasser, 0.1 Volumenteile 3.0 M Natriumacetat, pH 5.4 und 2 Volumenteile Ethanol zugibt. Die fadenförmige DNA wird mit Hilfe einer Pasteurpipette aus der Lösung heraus aufgewickelt, in 70% Ethanol gewaschen und mit einem gleichen Volumenteil Chloroform weiter extrahiert. Die DNA wird durch Zugabe von 0.1 Volumenteilen 3 M Natriumacetat, pH 5.4 und 2.0 Volumenteilen Ethanol präzipitiert. Der DNA Faden wird erneut aufgewickelt, in 70% Ethanol gewaschen und 5 Minuten an der Luft getrocknet. Anschliessend wird er in einem Gesamtvolumen von 7 ml TE-Puffer (vergl. Abschnitt VII) gelöst und bis zur weiteren Verwendung bei 4°C aufbewahrt.

5.2. Konstruktion einer genomischen λ Genbibliothek

Die zuvor isolierte Tabak DNA wird mit dem Restriktionsenzym Sau3A verdaut und 20 Stunden über einen 10% - 40%igen Saccharosegradienten in einem SW41 Rotor (Beckman) bei 20'000 Upm aufgetrennt. Die aus diesem Gradienten erhältlichen Fraktionen werden mit Hilfe einer Gelelektrophorese (0.5% Agarosegel in TBE Puffer) analysiert. Diejenigen Fraktionen, die Fragmente der richtigen Grösse aufweisen, werden gepoolt. Die DNA wird mit 1/10 Volumenteil 3 M Natriumacetat (pH 4.8) un 2 Volumenteilen Ethanol ausgefällt und mit BamHI-verdauter, Phosphatase-behandelter λ EMBL3 DNA (Stratagen; La Jolla, CA) ligiert. Die Verknüpfungsreaktion wird gemäss den Angaben des Herstellers durchgeführt, wobei jeweils Reaktionsansätze von 5 µl verwendet werden, die 1 µg λ-Vektor-DNA sowie 0.1 µg der einzubauenden Tabak DNA enthalten. Der Einbau der aus dieser Verknüpfungsreaktion resultierenden DNA in die Köpfe von λ-Phagen wird mit Hilfe des Gigapack Plus Kits von Stratagene gemäss den Angaben des Hersteller durchgeführt. Die Phagenausbeute nach Infektion von *E. coli* CES201 (Glover DM,) beträgt ca. 2 x $10^6$ Phagen pro µg inserierter DNA.

5.3. Screening der Genbibliotheken und Isolierung genomischer Klone

Die genomische Genbibliothek wird gemäss Maniatis *et al* (1982) mit Hilfe der Kolonie- bzw. Plaque-Hybridisierung gescreent. Als DNA Probe dient der bei Shinshi *et al* (1987) beschriebene cDNA Klon pCHN50 sowie der in Abschnitt 4.4. isolierte Klon pCHN48.

Die auf diese Weise erhältlichen Rekombinanten werden gereinigt und mit Hilfe der Southern Blot-Analyse teilweise charakterisiert. Einer dieser Klone, der mit den cDNA Probenmolekülen PCHN50 und PCHN48 ein sehr starkes Hybridisierungssignal erkennen lässt und gemäss Southern Blot-Analyse das komplette Chitinasegen enthält, wird für die weiteren Untersuchungen ausgewählt und erhält die Bezeichnung λCHN17.

## II. Charakterisierung des genomischen Klons λCHN17

Beispiel 6 DNA-Sequenzierung und Sequenzanalyse

Nach Verdauung mit dem Restriktionsenzym HindIII erhält man ein 5.4 Kb umfassendes DNA Fragment, welches das komplette Chitinasegen enthält und mit einem Fragment gleicher Grösse aus Tabak DNA korrelliert. Ein Teil dieses Fragments, das 3850 Bp umfasst und die gesamte kodierende Sequenz beinhaltet, wird anschliessend sequenziert.

Restriktions fragmente werden in geeigneten Klonierungsvektoren, wie z.B, M13mp18 oder M13mp19 [Yanisch-Perron *et al* (1985)] kloniert und in beiden Richtungen mit Hilfe der Dideoxynukleotid Methode ['Dideoxynucleotide chaintermination method'; Sanger *et al* (1977)] sequenziert. Die ermittelten Nukleotid- und Aminosäuresequenzen werden mit Hilfe des Computers unter Verwendung einer Genetics Computer Group Software [Devereux *et al* (1984)] weiter analysiert.

Die komplette DNA Sequenz des basischen Chitinasegens aus Tabak, das die Bezeichnung Gen 48 erhält, ist im Sequenzprotokoll [SEQ ID NO: 1] wiedergegeben.

Ein Vergleich dieser Sequenz mit den cDNA Klonen pCHN48 und pCHN50 macht deutlich, dass innerhalb der kodierenden Region von Gen 48 zwei intervenierende Sequenzabschnitte (Intron) zwischengeschaltet sind. Das erste dieser Introns umfasst 274 Bp und befindet sich zwischen dem 1. und 2. Nukleotid des für Glycin-kodierenden Kodons 148. Das zweite Intron ist 269 Bp lang und befindet sich zwischen dem 2. und 3. Nukleotid des Histidin-kodierenden Kodons 199. Beide Introns besitzen in Übereinstimmung mit anderen, bekannten pflanzlichen und tierischen Introns Consensus-Spleissstellen, die eine Donor- und eine Akzeptorsequenz ( GT AAGTC und AC AG ) aufweisen. Darüberhinaus besitzen beide Introns die Sequenz CT(G/A)A(C/T), 33 Nukleotide vom 3′ Border entfernt, die Ähnlichkeiten zu tierischen Consensussequenzen (PyTPuAPy) aufweist. Diese Consensussequenzen sind an der Ausbildung schleifenförmiger Intermediärprodukte im Rahmen der Excission beteiligt.

Die Nukleotidsequenz der Exons von Gen 48 ist identisch mit der DNA Sequenz der kodierenden Region von Klon pCHN48.

Beispiel 7: S1 Kartierung zur Identifizierung der 3′- und -5′-flankierenden Region

7.1. Primer Extension

50 µg der zuvor isolierten Gesamt-RNA werden in einem 500 µl Eppendorfröhrchen lyophilisiert. Die RNA wird dann in 30 µl einer Lösung resuspendiert, die radioaktiv markierte Probenmoleküle enthält und für 10 Minuten auf 70 °C erhitzt. Das Röhrchen wird im Wasserbad langsam auf eine Temperatur von 37 °C abgekühlt und über Nacht inkubiert. Ohne das Röhrchen aus dem 37 °C Wasserbad zu entnehmen, werden die folgenden Lösungen und Reagentien zugesetzt:

| | |
|---|---|
| Reverser Transkriptase Puffer (10fach) | 2 µl |
| (500 mM Tris-HCl, pH 7.5; 400 mM KCl; 30 mM $MgCl_2$) | |
| Rinderserumalbumin | 5 mg/ml |
| Dithiothreitol (100 mM) | 5 µl |
| dNTPs (10fach; 10 mM von jedme dNTP in $H_2O$) | 5 µl |
| $H_2O$ | 3 µl |
| RNasin (80 Einheiten) | 2 µl |
| Reverse Transkriptase (400 Einheiten) | 2 µl |

Dieser Reaktionsansatz wird 30 Minuten bei einer Temperatur von 37 °C inkubiert. Die Reaktion wird durch Zugabe von 5 µl 3 M Natriumacetat (pH 5) und 150 µl absolutem Ethanol abgebrochen. Das Röhrchen wird anschliessend 30 Minuten bei -20 °C belassen, bevor das Präzipitat über eine Zentrifugation gewonnen wird. Es schliessen sich ein Wasch- (80% Ethanol) und Trocknungsschritt (Lufttrocknung) an. Das so gewonnene Präzipitat wird in 10 µl bis 20 µl einer Farbstoff-haltigen Lösung (90% Formamid, 0.05% Bromphenolblau, 0.05% Xylolcyanol, 1 mM EDTA) resuspendiert. Die Extensionsprodukte werden auf einem 6%igen Sequenzierungsgel [Maniatis *et al* (1982)] aufgetrennt und mit Hilfe der Autoradiographie sichtbar gemacht.

7.2. S1 Kartierung

Die Analyse der Transkriptionsstartstelle der mRNA wird mit Hilfe einer S1 Kartierung durchgeführt, die sich im wesentlichen an der Beschreibung bei Maniatis *et al* (1982) orientiert.

Der genomische Klon λCHN17, der die gesamte 5′-nicht-translatierte Region enthält, wird mit Sau3A/PstI geschnitten und ein 0.3 Kb umfassendes Fragment, welches einen Teil der 5′-flankierenden Region sowie einen kurzen Abschnitt der kodierenden Sequenz enthält, wird in einem M13 Vektor subkloniert. Die aus diesem Subklonierungsschritt gewonnene Einzelstrang DNA dient dann als Template für die sich anschliessende Primer-Extension. Die resultierende $^{32}$P-markierte DNA Probe wird mit SstI und PstI geschnitten. Der verlängerte DNA Strang wird anschliessend denaturiert und über eine Polyacrylamid Gelelektrophorese aufgetrennt. Die DNA probe wird dann für 16 Stunden und bei einer Temperatur von 52° C mit 3 μg Poly(A)$^+$ RNA inkubiert und anschliessend bei einer Temperatur von 30° C mit S1 Nuklease (ca. 400 Einheiten/ml) verdaut. Die optimale S1 Nukleasekonzentration kann in Vorversuchen bestimmt werden. Die Verdauungsprodukte werden dann auf einem 6%igen Sequenzierungsgel aufgetrennt.

Poly(A)+ RNA wird gemäss obiger Beschreibung aus kultiviertem Tabakgewebe, dass zuvor zur Produktion hoher Chitinasespiegel induziert wurde, isoliert.

Die Länge der Verdauungsprodukte wird anhand von Grössenvergleichsmarkern bestimmt. Geeignete Grössenvergleichsmarker erhält man, indem man Sequenzierungsreaktionen mit Einzelstrang DNA durchführt und die Reaktionsprodukte vor der Elektrophorese mit PstI verdaut. Man erhält auf diese Weise Fragmente unterschiedlicher Länge mit einem Dideoxy-Ende, die die gleichen 5′-Enden aufweisen wie die zur Kartierung vorgesehenen, geschützten Fragmente.

### 7.3. Nukleotidsequenz der 3′- und 5′-flankierenden Region des Chitinasegens

Mit Hilfe der S1 Nuklease-Kartierung lassen sich insgesamt zwei Banden ermitteln, was die Vermutung nahe legt, das mehrere Startstellen auf dem Chitinasegen existieren. Geht man von der Intensität der betreffenden Banden aus, dann ist die Haupt-Transkriptionsstartstelle identisch mit dem A in Position 1969 innerhalb der Sequenz CT **A**CT. Das erste mögliche Initiationssignal befindet sich in Position 1980, 11 Bp stromabwärts des Transkriptionsstarts, innerhalb einer Basenfolge T **AA** **AATG**AG, die in Übereinstimmung mit anderen Translationsstartstellen pflanzlicher Gene als Consensus-Sequenz bezeichnet wird.

Die 5′-flankierende Region ist durch zahlreiche Basenfolgen charakterisiert, die in Analogie zu anderen eukaryontischen Genen in einen direkten Zusammenhang mit regulatorischen Funktionen gebracht werden können:

(a) eine TAAATA-Sequenz (mutmassliche 'TATA box') stromaufwärts der Transkriptionsstartstelle in Position -28 bis -23 der 5′-flankierenden Region;

(b) eine CCAATT-Sequenz in Position -114, ähnlich der CAAT Box, die zuweilen in tierischen Genen, stromaufwärts der TATA Box, gefunden wird ;

(c) eine 6 Bp umfassende unvollkommen invertierte Wiederholungssequenz ( GCCGAATTCG A GC ) in Position -140, die dem regulatorischen Hitzeschock-Consensuselement tierischer Gene ähnelt;

(d) eine 10 Bp umfassende vollkommene Wiederholungssequenz (ATGTCCAAAC) in Position -152 und -228;

(e) eine 20 Bp umfassende unvollkommene direkte Wiederholungssequenz ( TTTTAA C TAA-ATCTATGTCC ) in Position -166 und -569;

(f) eine 25 Bp umfassende unvollkommene direkte Wiederholungssequenz ( CAACTTT C AAAA A TT A TTTTTTAAA ) in Position -191 und -217;

(g) ein 20 Bp umfassendes Palindrom ( TAAAATATGA | TCAT G TTTTA ) in Position -289;

(h) eine 11 Bp umfassende vollkommene direkte Wiederholungssequenz (TAAGAGCCGCC) in Position -435 und -480;

(i) eine 15 Bp umfassende unvollkommene direkte Wiederholungssequenz ( TAAAATACA C GTCGA ) in Position -514 und -644;

Die 3′-flankierende Region ist durch zwei AATAAA Sequenzen in Position 52 und 120, stomabwärts der Translationsstop Sequenz TAA gekennzeichnet.

## III. KONSTRUKTION CHIMÄRER GENE

### Beispiel 8: Verknüpfung der Chitinase-kodierenden Sequenzaus pCHN48 mit der regulatorischen 5′-Sequenz aus Gen 48

### 8.1. Konstruktion von Plasmid pGY1

Das Plasmid pGY1 ist abgeleitet von dem bei Pietrzak *et al* (1986) beschriebenen pflanzlichen Expressionsvektor pDH51. Im Plasmid pGY1 ist die NcoI Schnittstelle des Ausgangsplasmids pDH51 gegen eine XhoI Schnittstelle ersetzt.

Das Plasmid pDH51 wird mit NcoI geschnitten und die überstehenden Enden werden mit Klenow Polymerase aufgefüllt. Die nunmehr glatten Enden werden dann mit einem XhoI Linker (CCTCGAGG) ligiert.

8.2. Konstruktion von Plasmid pCIB200

Die Konstruktion dieses binären Vektors geht aus von dem bei Schmidhauser und Helinski (1985) beschriebenen Plasmid pTJS75, einem Abkömmling von RK2 (An G *et al*, 1985), das einen weiten Wirtsbereich umfasst und ein Tetrazyklinresistenzgen aufweist. Dieses Plasmid wird mit dem Restriktionsenzym NarI geschnitten und anschliessend mit dem AccI Fragment von pUC4K [Vierra und Messing (1982)-], welches das NptI-Gen trägt, verknüpft. Das auf diese Weise gebildete Plasmid pTJS75kan, das neben dem Tetrazyklinresistenzgen nunmehr auch das NptI-Gen beinhaltet, wird dann mit dem Restriktionsenzym SalI verdaut.

Gleichzeitig wird das Plasmid pCIB7, das bei Rothstein SJ *et al* (1987) beschrieben ist, mit EcoRV geschnitten und das resultierende EcoRV Fragment, welches die linke und rechte T-DNA Grenzsequenz aus dem Ti-Plasmid von *Agrobacterium tumefaciens* sowie ein chimäres Nos/NptII Gen und die pUC Polylinkerregion enthält, wird mit XhoI-Linkern verknüpft.

Das resultierende Konstrukt wird dann mit XhoI verdaut und in die SalI Schnittstelle des Plasmids pTJS75kan einkloniert. Das resultierende Plasmid, dessen Genkarte in Figur 2 wiedergegeben ist, erhält die Bezeichnung pCIB200.

8.3. Konstruktion von Plasmid pSCH10

Plasmid pSCH10 enthält die Chitinase-kodierende Sequenz, die 5′-transkribierte nicht-kodierende Sequenz, 21 stromaufwärts der Transkriptionsstartstelle gelegene Basenpaare des Chitinasegens 48 sowie die 3′-transkribierte, nichtkodierende Sequenz, eingespleisst in die BamHI/PstI Klonierungsstelle des CaMV 35S Pflanzenexpressionsvektors pGY1.

Die zur Herstellung von pSCH10 notwendigen Verfahrensschritte sind in Figur 3 wiedergegeben und im folgenden näher beschrieben:

(1) Der genomische Klon λCHN17, der das Chitinasegen 48 enthält, wird mit HindIII geschnitten. Das resultierende 5.6 Kb umfassende HindIII Fragment wird isoliert und in die HindIII Klonierungsstelle des Plasmids pUC8 eingespleisst, unter Bildung von Plasmid pCHN65.

(2) Plasmid pCHN65 wird mit EcoRI geschnitten und das resultierende 2.5 Kb umfassende Fragment, welches das 5′-Ende der Chitinase-kodierenden Region enthält, wird ebenfalls in pUC8 einkloniert. Das entstehende Plasmid erhält die Bezeichnung pCHN68.

(3) Plasmid pCHN68 wird mit PstI verdaut und unter Verlust eines 0.5 Kb umfassenden PstI/EcoRI Fragmentes religiert (mit sich selbst verknüpft). Das resultierende Plasmid wird mit pCHN74 bezeichnet.

(4) Um die 5′-nicht kodiertende Region des Transkriptes von Gen 48 hinter eine BamHI Schnittstelle zu klonieren, wird das Plasmid pCHN74 zunächst mit HphI verdaut, anschliessend durch Einwirkung von T4 DNA Polymerase mit glatten Enden versehen und schliesslich mit PstI geschnitten. Das HphI/PstI Fragment wird isoliert und in das mit HincII/PstI verdaute Plasmid pUC8 [Vieira & Messing (1982)] eingespleisst, unter Bildung von pCHN87.

Nach Sequenzierung lässt sich anhand der DNA Sequenz zeigen, dass während des letzten Verfahrensschrittes eine Base der 1/2 HincII Schnittstelle verlorengegangen ist.

(5) pCHN87 wird mit EcoRI und HindIII verdaut und in den Klonierungsvektor pUC9 [Vieira & Messing (1982)] eingespleisst. Das resultierende Plasmid erhält die Bezeichnung pCHN88.

(6) Das 1 Kb umfassende PstI Fragment des Tabak cDNA Klones 48 (pCHN 48) wird in die PstI Klonierungsstelle von Plasmid pUC8 eingespleisst unter Bildung von pCHN78.

(7) Das PstI Insert von Plasmid pCHN78 wird durch Schneiden des Plasmids mit dem Restriktionsenzym PstI freigesetzt und in die PstI Stelle von pCHN88 eingespleisst, wodurch die komplette, für Chitinase kodierende DNA Sequenz wiederhergestellt wird. Das resultierende Plasmid erhält die Bezeichnung pCHN89.

(8) Das Plasmid pCHN89 wird anschliessend vollständig mit BamHI und teilweise mit PstI verdaut. Das resultierende 1.5 Kb umfassende Fragment wird in den pflanzlichen Expressionsvektor pGY1, der zuvor ebenfalls mit BamHI und PstI geschnitten wurde, einkloniert, unter Bildung von pSCH10. Durch diesen Klonierungsschritt wird die Chitinasekodierende DNA Sequenz zwischen den CaMV Promotor und die CaMV Terminationssequenz plaziert.

8.4. Konstruktion von Plasmid pSCH12

Bei dem Plasmid pSCH12 handelt es sich um einen sogenannten 'Shuttle Vektor', der aufgrund seiner Konstruktion in der Lage ist sowohl in *E. coli* als auch in *Agrobacterium tumefaciens* stabil zu replizieren.

Zur Konstruktion von pSCH12 wird zunächst das zuvor beschriebene Plasmid pSCH10 mit EcoRI geschnitten und in den binären Vektor pCIB 200 eingespleisst. Es entsteht dann das gewünschte Plasmid pSCH12.

Der binäre Ti-Plasmid Vektor pSCH12 enthält 31 Bp 5′ vom ATG Startkodon, die kodierende Sequenz sowie die 145 Bp umfassende 3′-nicht-kodierende Region des Tabakchitinasegens 48, eingespleisst in die Polylinkerregion zwischen dem CaMV 35 S Promotor und dem Terminator von Plasmid pGY1. Dieser Vektor enthält darüberhinaus ein Tn5 NPTII Gen, das unter der regulatorischen Kontrolle von Nopalinsynthase-Expressionssignalen steht und den transformierten Zellen eine Kanamycin-Resistenz verleiht.

Beispiel 9: Verknüpfung der β-1,3-Glucanase kodierenden-Sequenz mit der regulatorischen 5′-Sequenz aus Gen 48

9.1. Konstruktion von Plasmid pSGL4

(a) Der Tabak cDNA Klon pGL36, der bei Shinshi *et al* (1988) beschrieben ist, wird mit dem Restriktionsenzym PstI geschnitten und in das Plasmid pUC8 inkloniert. Das resultierende Plasmid erhält die Bezeichnung pGLN12.

(b) Ein zweiter cDNA Klon aus Tabak, pGL31 [Shinshi *et al* (1988)], wird mit AccI und PstI verdaut und ein 0.85 Kb umfassendes Fragment, welches den 3′-Anteil der Glucanasekodierenden Sequenz enthält, wird isoliert.

(c) pGLN12 aus Schritt (a) wird mit AccI und PstI verdaut. Ein 0.5 Kb umfassendes Fragment, welches den 5′-Anteil der Glucanase-kodierenden Sequenz enthält, wird isoliert.

(d) Die Fragmente aus Schritt (b) und (c) werden gemeinsam in das zuvor mit PstI geschnittene Plasmid pUC8 einkloniert, wobei die vollständige Glucanase-kodierende Sequenz wiederhergestellt wird. Das resultierende Plasmid wird mit pGLN13 bezeichnet.

(e) Um den 5′G-Anhang der cDNA zu beseitigen und gleichzeitig eine BamHI Schnittstelle an der 5′ Seite der Glucanasekodierenden Sequenz zu installieren, nutzt man die Tatsache, dass der 5′ G-Anhang zusammen mit den ersten beiden Basen der cDNA eine HaeIII Schnittstelle bildet. pGLN12 wird daher mit HaeIII geschnitten und das 0.3 Kb Fragment in die HindII Stelle von pUC9 einkloniert und Bildung von pGLN16.

(f) pGLN16 wird mit ClaI und PstI verdaut und das 0.25 Kb umfassende Fragment wird durch das ClaI/PstI Fragment von Plasmid pGLN13 ersetzt, wodurch die kodierende Sequenz der Glucanase wiederhergestellt wird. Das resultierende Plasmid erhält die Bezeichnung pGLN17.

(g) pGLN17 wird mit BamHI und PstI verdaut. Das so erhältliche BamHI/PstI Fragment wird in den zuvor mit BamHI und PstI geschnittenen Vektor pGY1 eingespleisst, wobei die Glucanasekodierende Sequenz zwischen den 35 S Promotor von CaMV und dessen Terminationssequenz plaziert wird. Das resultierende Plasmid erhält die Bezeichnung pSGL2.

(h) Das EcoRI Fragment von pSGL2 wird in den binären Vektor pCIB200 eingespleisst unter Bildung von pSGL4.

9.2. Oligonucleotid-vermittelte Mutagenese zur Herstellungvon pSGL7

Um die 5′nicht-kodierende Sequenz des Chitinasegens unmittelbar vor die Glucanase-kodierende Sequenz zu plazieren bedient man sich der Oligonucleotid-vermittelten Mutagenese.

Zunächst wird das EcoRI Fragment von Plasmid pSGL2 in den Klonierungsvektor M13mp19 inkloniert unter Bildung von mSGL1. mSGL1 wird dann mit XhoI geschnitten und religiert, wobei ein Teil des Inserts verloren geht. Es entsteht mSGL5.

Einzelstrang DNA, die durch Wachstum von mSGL1 auf einem *E.coli* dam-Stamm [z.. *E.coli* GM1674] erzeugt wird, wird mit XhoI geschnittener mSGL5 DNA aus einem $dam^+$-Stamm [z.. *E. coli* HB101] hybridisiert. Dabei entsteht Doppelstrang-DNA (Duplex), die von einer einzelsträngigen Lücke ('gap') unterbrochen ist. Diese wird mit einem mutierten Oligonukleotid, das die folgende Basenfolge aufweist:

5′TCATTTGGAGAGGACTACTACATTAAAATGGCTGCTATCA3,()

über Basenpaarung verknüpft ('annealing'). Ein mutierter Klon, der sowohl die BamHI als auch die SmaI Schnittstelle verloren hat wird identifiziert und isoliert. Er erhält die Bezeichnung mSGL7.

Das AccI/ClaI Fragment aus mSGL7, welches die mutierte Sequenz enthält, wird anschliessend in die AccI/ClaI Schnittstelle von pSGL2 einkloniert unter Bildung von pSGL2/9.

pSGL2/9 wird mit den Restriktionsenzymen EcoRV und PstI verdaut und das EcoRV/PstI Fragment, das die mutierte Sequenz enthält, wird in die EcoRV/PstI Schnittstelle von pSGL2 eingespleisst unter Bildung von pSGL6.

Somit werden alle Sequenzen, die bei der Mutagenes dabei waren, ersetzt, mit Ausnahme des kurzen Sequenzabschnittes zwischen der EcoRV und ClaI Schnittstelle.

Abschliessend wird PSGL6 mit ECORI verdaut und das EcoRI Fragment in den binären Vektor PCIB200 einkloniert. Das resultierende Plasmid erhält die Bezeichnung pSGL7.

Dabei handelt es sich um ein chimäres Konstrukt bestehend aus:

1. einer β-1,3 Glucanse, die am 5′-Ende trunkiert ist und mit dem Startkodon ATG in Position +34 beginnt;
2. der regulatorischen Sequenz aus dem Klon CHN17, die von Position -14 bis -1 (ATG = 0) reicht und mit dem CaMV 35S Promotor verknüpft ist.

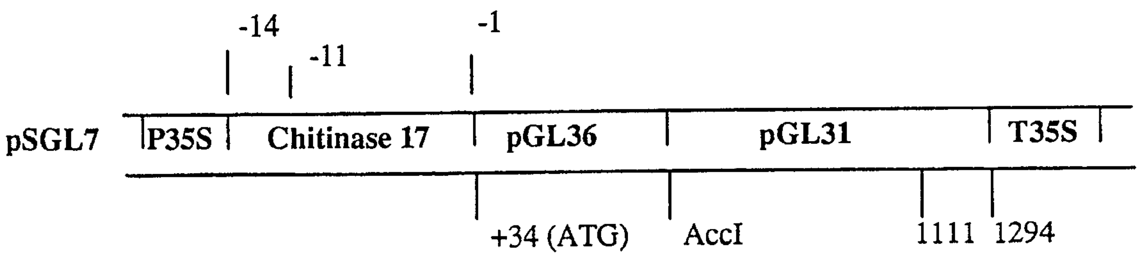


Als Kontrolle fungiert ein entsprechendes Konstrukt ohne die regulatorische Sequenz aus dem Chitinaseklon CHN17 mit der Bezeichnung pSGL5.

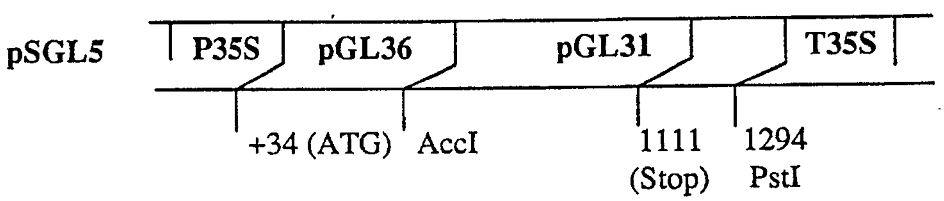


Als weitere Kontrolle wird ein sogenannter Leervekor (pCIB200) eingesetzt, der kein Chitinasekonstrukt enthält.

## IV TRANSFORMATION UND HERSTELLUNG TRANSGENER PFLANZEN

Beispiel 10.1: Transformation von *Agrobacterium tumefaciens*-mit binären Vektoren

Die zuvor in den Beispielen 8 und 9 beschriebenen binären Vektoren werden unter Anwendung des folgenden Verfahrens in den *Agrobacterium tumefaciens* Stamm LB4404 transformiert. *A. tumefaciens* LBA 4404 enthält ein deletiertes Ti Plasmid, dem die T-DNA Region fehlt, das aber nach wie vor eine intakte vir-Region aufweist [Hoekema *et al* (1983)].

*Agrobacterium tumefaciens* Stamm LB4404 wird bei einer Temperatur von 30° C in einer über Nacht Kultur in 5 ml MG/L Medium [vergl. Abschnitt VII] herangezogen. Zu dieser 5 ml über Nacht Kultur werden dann 250 ml MG/L Medium zugegeben und der ganze Ansatz wird gut durchmischt, bis eine optische Dichte von OD=0.6 (bei 600 nm) erreicht ist. Die Zellen werden dann über eine Zentrifugation bei 8'000 g gesammelt und in 5 ml MG/L Medium resuspendiert. 200 µl dieser Zellsuspension werden mit 0.2 µg bis 1 µg binärer Plasmid DNA in MG/L Medium inkubiert, wobei dieser Ansatz nach leichtem Durchmischen sofort in einem Trockeneis/Ethanol Bad tiefgefroren wird. Nach 5 Minuten wird das Röhrchen in ein 37°C Wasserbad gegeben und dort für 5 Minuten belassen. Danach werden 2 ml MG/L Medium zugegeben. Diese Suspension wird dann für 2 bis 3 Stunden in einem 30°C Wasserbad inkubiert. Anschliessend werden die Zellen über eine Zentrifugation gesammelt. Die Zellen werden in einem kleinen Volumen MG/L Medium resuspendiert und dann auf Selektivmedien (MG/L Platten mit 100 µg/ml Gentamycin) ausplattiert. Nach 2 bis 3 Tagen bei 30°C erscheinen die ersten Kolonien.

Beispiel 10.2: Transformation von *Abrobacterium tumefaciens*-mit binären Vektoren

In einer alternativen Ausführungsform werden die zuvor in den Beispielen 8 und 9 beschriebenen binären Vektoren durch eine triparentale Kreuzung [Rogers SG *et al* (1986)], unter Verwendung eines *E. coli* Helferstammes, der ein Plasmid mit einer tra-Funktion besitzt, in den *Agrobacterium tumefaciens* Stamm LBA4404 überführt. Als *E. coli* Helferstamm kann z.B. *E. coli* BHB1011 verwendet werden, der das Plasmid pRK2013 enthält, das die für den Transfer der binären Vektoren notwendigen tra-Funktionen besitzt.

*A. tumefaciens* LBA4404 wird über Nacht bei 28°C in LB Medium mit 20 mg/l Rifampicin und 500mg/l Streptomycin herangezogen. *E. coli* BHB1011 und die *E. coli* stämme mit den binären Vektoren wrden über Nacht bei 37°C in LB Medium mit 25mg/l Kanamycin herangezogen.

Je 1 ml dieser Kulturen wird bei 8000 g abzentrifugiert, in 1 ml sterilem Wasser oder 10mM $MgSO_4$ gewaschen, wieder abzentrifugiert und in 100 ul Wasser oder einer $MgSO_4$ Lösung resuspendiert. Eine Platte mit einem LB Festmedium wird in vier Sektoren unterteilt. In diesen Sektoren werden Tropfen der drei Bakterienkulturen übereinander aufgetragen und zwar so, dass in drei der vier Sektoren die drei möglichen Kombinationen zweier Kulturen gemischt werden. Diese dienen als Kontrollen. Im vierten Sektor dagegen werden all drei Kulturen zusammengemischt. Nach dem Eintrocknen der Tropfen werden diese Platten über Nacht bei 28°C inkubiert. Danach wird von jedem Sektor eine Probe entnommen und in Wasser oder $MgSO_4$ Lösung suspendiert. Von diesen Suspensionen werden Verdünnungen hergestellt und auf LB-Platten mit 20 mg/l Rifampicin, 500 mg/l Streptomycin und 25mg/l Kanamycin ausplattiert und für zwei bis drei Tage bei ca. 28°C inkubiert. Kolonien, die bei hoher Verdünnung der triparentalen Kreuzung wachsen [bei ähnlicher Verdünnung der Kontrollkreuzungen darf nichts wachsen] werden durch wiederholtes ausplattieren einzelner Kolonien von eventuell noch vorhandenen Elternbakterien befreit.

Beispiel 11: Blattscheiben- (,Leaf-disk,) Transformationvon *N. sylvestris* und *N. tabacum*

Die Blattscheiben-Transformation wird im wesentlichen gemäss dem bei Horsch *et al*, (1985) beschriebenen Verfahren durchgeführt.

*A. tumefaciens* LBA 4404 (pSCH12; pSGL7) wird über Nacht in einem Glutamat-Salz Medium, das mit 20 mg/l Rifampicin, 500 mg/l Streptomycin und 25 mg/l Kanamycin angereichert und einen pH-Wert von 5.6 eingestellt ist, bei einer Temperatur von 28°C herangezogen. In dieser über Nacht-Kultur, die ca. 3.3 x $10^8$ Zellen enthält, werden sterile Blattscheiben (5 mm bis 10 mm Druchmesser) von *N. sylvestris* oder *N. tabacum* cv. Havana 425 für 5 Minuten inkubiert.

Diese werden dann aus der Kultur entnommen und aufsterilen Papierhandtüchern trockengetupft, bevor sie in Petrischalen mit einem Durchmesser von 100 mm überführt werden, die eine Nährkultur enthalten. Diese Nährkultur besteht aus einem mit 1% Agar (DIFCO) verfestigten Basismedium (30 ml) nach Linsmeier und Skoog (1965), das als weitere Zusätze einen pH Indikator (Chlorphenolrot; 5 mg/l) sowie die Pflanzenwuchsstoffe Kinetin (0.3 mg/l) und a-Naphthylessigsäure (2 mg/l) enthält. Dieses Agar-Medium (Medium A) wird mit 1 ml bis 2 ml einer 2 Wochen alten Suspensionskultur von S275N Zellen, die sich aus Markgewebe von *N. tabacum* cv. Havana 425 (Eichholz et al, 1983) ableitet, überschichtet und mit einem Filterpapier (#1 Whatman filter paper) überdeckt. Auf dieses Filterpapier werden dann die gemäss obiger Beschreibung vorbehandelten Blattstückchen aufgelegt.

Nach 48 Stunden werden die Explantate zur Sprossinduktion auf ein Selektivmedium der gleichen Zusammensetzung gegeben, das darüberhinaus aber noch 350 mg/l Cefotaxim sowie 100 mg/l Kanamycin enthält (Medium B) und bei 25°C und diffusem Licht (80 bis 100 µEinstein) inkubiert. Ko-kultiviertes Kontrollgewebe wird auf das gleiche Medium ohne Kanamycin überimpft. Die Explantate werden wöchentlich auf frisches Medium B transferriert.

4 bis 8 Wochen nach der Ko-Kultivierung werden die sich aus den Explantaten entwickelnden grünen Sprosse geerntet und in 50 ml Gefässen auf 25 ml Medium C (Festmedium mit 0.6% Phytagar) überführt. Das gesamte Gewebe wird bei einer Temperatur von 24°C bis 28°C bei einer Beleutungsintensität von 80 bis 100 µEinstein kultiviert. Nach 1 bis 2 Wochen erfolgt die Bewurzelung der Sprosse.

## V. ANALYSE TRANSGENER PFLANZEN

### Beispiel 12.1: Selektion der Transformanten

Die Pflänzchen, welche aus den verschiedenen Transformationsereignissen resultieren, können auf zwei verschiedene Weisen auf Transformation untersucht werden.

1) Der Vektor-spezifische Kanamycin-Resistenz Marker dient als Kriterium für eine erfolgreiche Transformation. Blattexplantate (ca. 5 mm Durchmesser) von zuvor transformierten Pflänzchen werden auf einem Auxin/Cytokinin-Medium (Medium A) mit 50 mg/l Kanamycin subkultiviert. Als Kontrolle dienen Explantate der selben Pflanze, die auf einem Auxin/Cytokinin-Medium (Medium A) ohne Selektivmarker kultiviert werden. Die Blattexplantate werden für einen Zeitraum von 21 Tagen gemäss der Beschreibung bei Meins & Lutz (1979) inkubiert. Von den Pflänzchen die sowohl auf dem Kanamycin-Medium als auch auf dem Kontroll-Medium wachsen kann angenommen werden, dass sie Kanamycinresistent sind.

2) Der zweite Test auf Transformation ist auf den Nachweis der Überexpression von Chitinase und β-1,3-Glucanase gerichtet. Eine Überexpression kann durch messung des Chitinase- bzw. Glucansesgehalts im Blattgewebe mit Hilfe einer "Rocket" Immunelektrophorese [Laurell and Mckay (1981)] unter Verwendung gereinigter anti-Tabak Chitinase bzw. Glucanase IgG Antikörper [Shinshi et al, (1987)] ermittelt werden.

Blattgewebe wird zunächst in 2 bis 5 Volumenteilen eines Extraktionspuffers (0.1 mM Ethylendiamintetraessigsäure, 1 mM Dithiothreitol, 1 mM Phenylmethylsulfonylfluorid und 200 mM 2-Amino-2-(hydroxymethyl)-1.3-propandiol, pH 8.0) homogenisiert. Die lösliche Proteinfraktion erhält man durch Zentrifugation (15 min bei 10,000 g) bei 6°C.

Der Gehalt an Chitinase und β-1,3-Glucanase wird mit Hilfe der "Rocket" Immunelektrophorese unter Verwendung von anti-Tabak Chitinase bzw. β-1,3-Glucanase IgG bestimmt. Chitinase bzw. β-1.3-Glucanase haltige Proben werden über ein 1% (w/v) "low $m_r$" Agarosegel (BioRad), das anti-Tabak Chitinase bzw. β-1,3-Glucanase IgG in einer Endkonzentration von 10 µg/ml enthält, bei einer Temperatur von 6°C und einem Maximum von 200 V und 15 mA 12-16 Stunden lang aufgetrennt. Die getrockneten Gele werden mit Coomassie Blau gefärbt und der Gehalt an Chitinase bzw. β-1,3-Glucanase bestimmt.

Als Standards dienen authentische Enzymproben, die direkt aus Havana 425 Tabakgewebe isoliert werden.

(3) Die Chitinaseaktivität wird radiometrisch bestimmt unter Verwendung von $^3$H-markietem Chitin [spez. Aktivität 4.93 x $10^5$ dpm/Mol N-Acetylglucosamin] als Substrat [Boller *et al* (1983)].

### Beispiel 12.2: Extraktion der Interzellulären Flüssigkeit (ICF)

Die Extraktion intercellulärer Flüssigkeit aus pflanzlichem Gewebe kann gemäss der bei Parent und Asselin (1984) beschriebenen Methode durchgeführt werden.

Dabei werden zunächst Blätter von regenerierten, transgenen Pflanzen gesammelt und in Stücke von 4 bis 5 cm$^2$ zerschnitten. Diese werden dann im Vakuum jeweils für 30 Sekunden unter leichem Schütteln mit einem starken Überschuss an kaltem (ca 4.C) Puffer infiltriert. Besagter Puffer weist vorteilhafterweise die folgende Zusammensetzung auf:

| | |
|---|---|
| Tris-HCl [pH 7.8] mit 0.5 M Saccharose | 25.0 mM |
| $MgCl_2$ | 10.0 mM |
| $CaCl_2$ | 10.0 mM |
| Phenylmethylsulfonylfluorid (PMSF) | 0.5 mM |
| 2-Mercaptoethanol | 5.0 mM |

Alternativ dazu kann auch ein 50 mM Citratpuffer (pH 5.5) verwendet werden. Auch ein Arbeiten bei Zimmertemperatur ist möglich.

Beim Entfernen des Vakuums dringt der Puffer in die Blätter ein. Die Blattstückchen werden anschliessend vorsichtigt abgetrocknet und in eine 20 ml Spritze überführt. Diese wird in ein Zentrifugenröhrchen eingehängt und 10 Minuten bei niedrigen Geschwindigkeiten (ca. 1000 x g) und bei niedriger Temperatur (4° C) zentrifugiert.

Beispiel 12.3: Extraktion der Intrazellulären Proteinfraktion

Die gemäss Abschnitt 13.2 behandelten Blattstückchen werden anschliessend im gleichen Puffer homogenisiert. Die groben Partikel werden durch Zentrifugation abgetrennt.

Beispiel 13: Entwicklung transgener, homozygoter S1 Pflanzen

Die Pflänzchen der S0 Generation, die Kanamycin-resistentes Gewebe enthalten und erhöhte Chitinase- bzw. Glucansesspiegel aufweisen, werden in Erde überführt und nach Selbstbestäubung im Gewächshaus zur Samenbildung veranlasst. Die Samen werden Oberflächen-sterilisiert und ohne Sekundärkontaminationen in Petrischalen, die 30 ml Medium D (Medium A ohne Kinetin und a-Naphthylessigsäure unter Zusatz von 400 mg/l Kanamycin) enthalten, zur Keimung gebracht. Die Kanamycin-resistenten Sämlinge werden im Gewächshaus bis zur Reife herangezogen (S1 Generation) und ebenfalls zur Samenbildung veranlasst.

Die Samen von verschiedenen Pflanzen innerhalb der S1 Generation werden auf die zuvor beschriebene Weise auf Kanamycinresistenz hin untersucht. Wenn von 100 getesteten Samen einer S1 Pflanze alle das Merkmal Kanamycinresistenz aufweisen, so kann man davon ausgehen, dass diese Pflanze homozygot ist für diesen Antibiotikaresistenzmarker. Die homozygoten $Km^r/Km^r$ Samen aus transformierten *Nicotiana sylvestris* Pflanzen (SSC2.3) werden dann für die biologischen Tests verwendet.

Als Kontrollen dienen Pflanzen, die (a) aus nicht-transformierten, unbehandelten Blattscheiben regeneriert werden sowie (b) Pflanzen, die ausgehend von Blattscheiben regeneriert werden, die zuvor mit dem Vektor PCIB200 transformiert worden sind (SCIB2).

Beispiel 14: Überexpression wird als ein monogenetischer-Mendel-Faktor weitervererbt.

Samen der S1 Generation von transformierten Pflanzen sowie von Kontrollpflanzen werden zur Keimung gebracht. Die Blattgewebe werden auf Kanmycin-Resistenz, Chitinase- bzw. Glucanasegehalt ("Rocket" Immunelektrophorese) sowie auf Chitinase- bzw. Glucanseaktivität hin untersucht. Die Chitinaseaktivität wird mit Hilfe eines Radiometrischen Assays unter Verwendung tritiierter Chitinase gemäss der Beschreibung bei Boller *et al* (1983) bestimmt. Die Glucanaseaktivität wird anhand der Produktionsrate an nicht-reduzierten Zuckern bestimmt, die mit reduziertem Laminarin als Substrat gebildet werden [Felix G und Meins FJr (1985)].

## VI Ergebnisse

(A) Chitinase-Transformation

Chitinasegehalt, Chitinaseaktivität (Proteinebene)

Die in Tabelle 1 und Figur 5 wiedergegebenen Resultate zeigen, dass

(1) Blattgewebe von S1 SSC2.3 Pflanzen einen Chitinasegehalt aufweisen, der bis zu 400 mal höher liegt als bei den Vektor-transformierten Kontrollen (SCIB2)

(2) Eine absolute Korrelation besteht zwischen der Überexpression von Chitinase und dem Auftreten des Kanamycinresistenzmarkers ($Km^r$).

(3) die Merkmale der Kanamycinresistenz und der Chitinase-Überexpression in einer Weise segregieren, wie dies für einen monogenischen Mendelfaktor zu erwarten ist.

(4) die Chitinaseaktivität und der Gehalt an Chitinaseantigen in den transformierten Pflanzen eindeutig miteinander korrellieren. Dies ist ein deutlicher Anhaltspunkt dafür, dass das eingeschleuste Chitinasegen in ein enzymologisch aktives Protein transkribiert und translatiert wird.

Es ist bekannt, dass das basische Isoenzym der Chitinase in Blättern, die mit dem Stresshormon Ethylen behandelt werden, in erheblichem Umfang induziert wird. Ein Vergleich der Verteilung des Chitinaseproteins in homozygoten SSC2.3 Pflanzen und Kontrollpflanzen mit und ohne Ethyleninduktion ist in Tabelle 2 wiedergegeben. Die Ergebnisse zeigen, dass der Chitinasegehalt in allen getesteten Geweben und Organen dramatisch zugenommen hat, im Vergleich zu den Kontrollen. Obgleich das Chitinasegen unter der Kontrolle eines konstitutiven Promotors steht, lässt sich dennoch in einigen Fällen ein erhöhter Chitinasespiegel nach Ethyleninduktion feststellen.

Trotz der stark erhöhten Chitinasespiegel zeigen die transformierten transgenen Pflanzen ein normales Wachstums-und Entwicklungsverhalten.

(5) Chitinasen aus Blattextrakten von Kontroll- und transformierten Pflanzen werden mit Hilfe einer SDS-PAGE fraktioniert, auf Nitrozellulosepapier übertragen und mit anti-Tabakchitinase IgG als primärem Antikörper gefärbt. Die gereinigte Tabakchitinase liefert als Refernz zwei Banden, die der Chitinase A (ca. 34 KD) und der Chitinase B (ca. 32 KD) entsprechen [Shinshi *et al* (1987)].

Die Blots derjenigen Gele, bei denen gleiche Mengen Chitinaseantigen in jede Reihe geladen werden, ergeben eine Einzelbande von ca. 32 KD in den Extrakten der nicht-transformierten *N. sylvestris* Pflanzen und eine Einzelbande von ca 34 KD in den Extrakten der SSC 2.3 Transformanten. Blots die aus stark überladenen Gelen resultieren lassen auch bei den SSC Tansformanten eine zusätzliche, schwache Bande bei ca. 32 KD erkennen, die mit der *N. sylvestris* Chitinase korrespondiert. Aus Mischungsexperimenten ergibt sich, dass die Chitinase in den Extrakten der SSC 2.3 Pflanzen etwa die gleiche Molekülgrösse aufweist wie die Tabakchitinase A, das Expressionsprodukt von Gen 48, das für die Transformation verwendet wird.

Diese Ergebnisse bestätigen somit, dass das erfindugsgemäss chimäre Tabakchitinasegen in *N. sylvestris* Pflanzen exprimiert und das Genprodukt korrekt zu einem Polypeptid von der Grösse des reifen Chitinasegens prozessiert wird.

Bestimmung des RNA-Gehaltes (RNA-Ebene)

Die Bestimmung des RNA-Gehaltes wird mit Hilfe der Northern Blot Analyse durchgeführt. Dabei wird Gesamt-RNA aus den unterständigen Blättern von drei homozygoten Kontrollpflanzen mit niedrigem Chitinasespiegel [15 ± 3.0 µg/g FW] und von drei homozygoten Chitinasetransformanten mit hohem Chitinasespiegel [827 ± 66 µg/g FW] isoliert und analysiert. Als Probe dient das PstI Insert des Chitinase cDNA Klons pCHN48, der sowohl mit Chitinasesequenzen aus Tabak als auch mit solchen aus *N. sylvestris* hybridisert.

Die Gesamt-RNA aus Kontrollpflanzen liefert eine Bande von ca. 1.2 - 1.3 KB. Vergleichbare Mengen an RNA aus SSC2.3 Pflanzen dagegen liefern zwei Hybridisierungsbanden, die zumindest eine 10-fach höhere Intensitat aufweisen im Vergleich zu den Kontrollen. Die beiden Banden korrespondieren, was ihre Grösse angeht, mit den 1,198 KB und 1,41 KB Transkripten, die man bei der Transkription des chimären Tabakchitinasegens, bestehend aus CaMV 35S Transkriptionsstart, der Tabakchitinase-kodierenden DNA Sequenz sowie dem CaMV Terminator, erwarten kann. Die Intensität dieser Banden ist in etwa gleich, was den Schluss nahelegt, dass das Tabakgen unabhängig vom jeweils verwendeten Terminator mit etwa der gleichen Effektivität transkribiert wird.

Kompartimentgerechte Lokalisation der Expressionsprodukte

Basische Chitinasen sind in der Regel im intrazellulären Kompartiment lokalisiert. Um nun die vom Gen 48 kodierte Chitinase in den transgenen *N. sylvestris* Pflanzen lokalisieren zu können, vergleicht man den Chitinasegehalt von intaktem Gewebe mit dem der interzellulären Waschflüssigkeit (IWF), die durch Vakuuminfiltration von Blättern mit Puffern hoher Salzkonzentration erhältlich ist. Man geht davon aus, dass man unter diesen Bedingungen eine IWF erhält, welche in der Hauptsache die in der Interstititalflüssigkeit befindlichen sowie die lose mit der Zellwand assoziierten Proteine enthält. Als Kontrolle fungiert die α-Mannosidase, die als Marker der Zentralvakuole gilt [Boller und Kende (1979)] sowie die Peroxidase, die sowohl in der Zentralvakuole als auch im extrazellulären Kompartiment zu finden ist.

Der prozentuale Anteil der oben genannten Enzyme in der IWF Fraktion von Blattgewebe aus nicht-transformierten und SSC2.3 Blättern ist in Tabelle 5 wiedergegeben. In der IWF Fraktion findet man weniger als 1% des Markerenzyms α-Mannosidase, was zeigt, dass bei Verwendung des beschriebenen Verfahrens zur Herstellung der IWF keine nennenswerten Mengen an vakuolär vorliegenden Enzymen extrahiert werden. Im Gegensatz dazu findet man ca. 30% der Peroxidaseaktivität in der IWF Fraktion. Die Blätter der SSC 2.3 Pflanzen weisen eine 20-fach höhere Chitinaseantigenkonzentration auf als die Kontrollpflanzen. In beiden Fällen aber beträgt die Chitinasekonzentration in der IWF weniger als 2%.

Diese Ergebnisse zeigen, dass die Chitinase auch bei sehr hohen Chitinasespiegeln, wie sie durch das erfindungsgemässe chimäre Kontrukt erzeugt werden, korrekt im zellulären Kompartiment der *N. sylvestris* Blätter lokalisiert ist

(B) β-1,3-Glucanase Transformation

Die Ergebnisse zweier unabhängig voneinander durchgeführter Transformationen (SSG7.1 und SSG7.2) sind in Tabelle 3 wiedergegeben. Die transformierten Pflanzen besitzten in ihrem Blattgewebe β-1,3-Glucanasekonzentrationen, die bis zum 100 fachen über den Kontrollwerten liegen. Diese Kontrollpflanzen sind mit dem Kontrollplasmid pSGL5 (vergl. Abschnitt 9.2) transformiert, das eine genetische Kontruktion enthält, worin die erfindungsgemässe Sequenz aus der 5,-nicht-translatierten Region des Chitinasegens nicht mit dem Glucanase-Strukturgen verknüpft ist.

(C) Tabbellen

**Tabelle 1:** Chitinaseaktivität [nKat/g] pSCH10 transformierter *N. sylvestris* Pflanzen [$Km^r/Km^r$; $Km^s/Km^r$] im Vergleich zu den Kontrollen [$Km^s/Km^s$].

| Geselbstete S0 Pflanzen | β-1,3-Glucanase | Chitinase [µg/g Antigen] | Chitinase [nKat/g] |
|---|---|---|---|
| $Km^s/Km^s$ | 18.7 ± 0.2 (3) | 45.7 ± 1.64 | 22.4 ± 2.58 |
| $Km^s/Km^r$ | 15.8 ± 2.17(8) | 553.0 ± 78.4 | 149.0 ± 17.3 |
| $Km^r/Km^r$ | 19.9 ± 2.33(3) | 750.0 ± 22.5 | 312.0 ± 46.0 |

**Tabelle 2:** Chitinasekonzentration [µg/g Frischgewicht (FG)] in verschiedenen Geweben pSCH10 transformierter *N. sylvestris* Pflanzen im Vergleich zu den Kontrollen mit [4 Tage bei 20 ppm] und ohne Ethylenbehandlung.

| Transformanten | Gewebe | Chitinase [µg/g FG] -Ethylen | +Ethylen |
|---|---|---|---|
| Vektor (SIB2) | Oberständige Blätter | 10.4 | 53.2 |
| | Unterständige Blätter | | |
| | intakt | 10.0 | 42.8 |
| | Epidermis | 90.8 | 104.0 |
| | Untere Epidermis | | |
| | entfernt | 9.8 | 38.8 |
| | Stengel | 8.4 | 14.0 |
| | Wurzeln | 58.8 | 148.0 |
| Chitinase | | | |
| (SSC2.3) | Oberständige Blätter | 452.0 | 704.0 |
| | Unterständige Blätter | | |
| | intakt | 520.0 | 760.0 |
| | Epidermis | 756.0 | 828.0 |
| | Untere Epidermis | | |
| | entfernt | 652.0 | 1116.0 |
| | Stengel | 161.0 | 1200.0 |
| | Wurzeln | 1200.0 | 1200.0 |

Tabelle 3

| β-1,3-Glucanasekonzentration [μg/g Frischgewicht (FG)] im Blattgewebe von transformierten *N. sylvestris* S1 Pflanzen im Vergleich zu den Kontrollen (SYL3) | | | | |
|---|---|---|---|---|
| Pflanzen | oberständige Blätter | | unterständige Blätter | |
| | Glucanase | Chitinase | Glucanase | Chitinase |
| | [μg/g FG] | | [μg/g FG] | |
| SSG7.1-1 | 128 | 11.4 | 74.1 | 54.6 |
| SSG7.1-4 | 93.9 | 21.9 | 90.3 | 51.0 |
| SSG7.2-23 | 93.9 | 15.0 | 5.4 | 46.5 |
| SSG7.2-24 | 100.8 | 13.5 | 0.0 | 41.7 |
| SYL3 | 1.2 | 6.3 | 3.0 | 15.6 |

Tabelle 4

| β-1,3-Glucanasekonzentration in *N. sylvestris* $Km^R$-Pflanzen der S1-Generation im Vergleich zu den mit einem Leervektor (pCIB200) transformierten Kontrollen. | |
|---|---|
| Pflanzen | Chitinasekonzentration in den oberständigen Blättern [μg/g FG] |
| SCIB (Kontrolle) | 1.5 ± 1.5* (4)# |
| SSG 7.1 | 77.0 ± 6.4 (20) |
| SSG 7.2 | 63.4 ± 1.0 (20) |

*Mittelwert ± SE;
#Anzahl Pflanzen

Tabelle 5

| Verteilung der Chitinase zwischen dem extrazellulären und intrazellulären Komparitment in den Blättern von Kontrol- und transformierten *N. sylvestris* Pflanzen | | | |
|---|---|---|---|
| | % Anteil in der IWF[a] | | |
| Pflanze | Peroxidase | α-Mannosidase | Chitinase |
| Nicht-transf. | 28.2 ± 4.8[b] | 0.65 ± 0.13 | 1,48 ± 0.54 |
| SSC 2.3 | 30.5 ± 6-2 | 0.89 ± 0.16 | 1.78 ± 0.65 |

[a] Prozentualer Anteil des Gesamthaft im Blattgewebe vorliegenden Enzymgehaltes in der IWF, basierend auf der Bestimmung von Chitinaseantigen sowie der Peroxidase- und Mannosidaseaktivität.
[b] Mittelwert ± SEM aus 4 unabhängigen Versuchen.

## VII. MEDIEN UND PUFFERLÖSUNGEN

| Medium A | |
| --- | --- |
| $NH_4NO_3$ | 1650 mg/l |
| $KNO_3$ | 1900 mg/l |
| $CaCl_2 x 2H_2O$ | 440 mg/l |
| $MgSO_4 x 7H_2O$ | 370 mg/l |
| $KH_2PO_4$ | 170 mg/l |
| $Na_2EDTA$ | 37.3 mg/l |
| $FeSO_4 x 7H_2O$ | 27.8 mg/l |
| $H_3BO_3$ | 6.2 mg/l |
| $MnSO_4 x 4H_2O$ | 22.3 mg/l |
| $ZnSO_4 x 7H_2O$ | 8.6 mg/l |
| KI | 0.83 mg/l |
| $Na_2MoO_4 x 2H_2O$ | 0.25 mg/l |
| $CuSO_4 x 5H_2O$ | 0.025 mg/l |
| $CoCl_2 x 6H_2O$ | 0.025 mg/l |
| Saccharose | 30.0 g/l |
| Thiamin•HCl | 0.400 mg/l |
| myo-Inosit | 100.0 mg/l |
| Kinetin | 0.3 mg/l |
| a-Naphthylessigsäure | 2.0 mg/l |
| Chlorphenolrot | 5.0 mg/l |
| Agar | 10.0 g/l |

**Medium B**

gleiche Zusammensetzung wie Medium A, jedoch ohne $\alpha$-Naphthylessigsäure sowie folgenden Zusätzen:

| | |
| --- | --- |
| Cefotaxim | 500 mg/l |
| Kanamycin | 75 mg/l |

**Medium C**

gleiche Zusammensetzung wie Medium B, jedoch ohne Kinetin

**MG/L Medium für *Agrobacterium***

L-Broth 50% Mannit-Glutamat Medium (Holsters *et al* 1978) 50%

| **W5-Salzlösung** | |
|---|---|
| NaCl | 154 mM |
| $CaCl_2 x 2H_2O$ | 125 mM |
| KCl | 5 mM |
| Glucsoe | 5 mM |
| pH 5.6-6.0 | |

| **Rinse I-Lösung** | |
|---|---|
| Saccharose | 154.0 g/l |
| MES | 0.59 g/l |
| $KNO_3$ | 250.0 mg/l |
| $NH_4NO_3$ | 25.0 mg/l |
| $NaH_2PO_4 \cdot H_2O$ | 15.0 mg/l |
| $CaCl_2 \cdot 2H_2O$ | 90.0 mg/l |
| $MgSO_4 \cdot 7H_2O$ | 25.0 mg/l |
| $(NH_4)_2SO_4$ | 13.4 mg/l |

| **TENP Puffer** | |
|---|---|
| Tris-HCl (pH 8.0) | 100 mM |
| EDTA | 10 mM |
| NP-40 (Sigma Chem.) | 1% (v/v) |

| **TBE-Puffer** | |
|---|---|
| Tris-Borat | 89 mM |
| Borsäure | 89 mM |
| EDTA | 2 mM |

| **TE-Puffer** | |
|---|---|
| Tris-HCl (pH 8.0) | 10 mM |
| EDTA | 1 mM |

| **SSC** | |
|---|---|
| NaCl | 1.54 mM |
| Natriumcitrat (pH 7.0) | 0.154 mM |

## VIII LITERATURVERZEICHNIS

An G *et al*, EMBO J., 4: 277-284 (1985).
**Birk** Y *et al*, Biochim. Biophys. Acta, 67: 326-328 (1963).
**Boller** *et al*, Planta, 157: 22-31 (1983).
**Boller** T und Kende H, Plant Physiol., 63: 1123-1132 (1979)
**Cashmore A,** Genetic Engineering of Plants, an Agricultural Perspective, Plenum, New York 1983, Seite 29-38.
**Devereux** *et al*, Nucl. Acids Res., 12: 387-395 (1984).
**Eichholz** R *et al*, Planta, 158: 410-415 (1983).
**Facciotti** und Pilet, Plant Science Letters, 15: 1-7 (1979).
**Felix** G und Meins FJr, Planta, 164: 423-428 (1985).
**Frank** G *et al*, Cell, 21: 285-294 (1980).
**Gardner** RC *et al*, Nucl. Acids Res., 9: 2871-2888 (1981).
**Garfinkel** und Nester, J. Bact., 144: 732-743 (1980).
**Glover** DM, DNA cloning, volume 1: a practical approach ; DM Glover ed., IRL Press, Oxford and Washington DC, p. 33 (198)
**Grimsley** NH *et al*, Nature, 325: 177-179 (1987).
**Haymes** BT *et al*, Nucleic Acid Hybridisation a Practical Approach , IRL Press, Oxford, England (1985).
**Hilder** *et al*, Nature, 330: 160-163 (1987).
**Hoekema** *et al*, Nature, 303: 179-180 (1983).
**Hohn** T *et al*, in: "Molecular Biology of Plant Tumors" , Academic Press, New York, pp. 549-560 (1982).
**Horn** *et al*, Plant Cell Reports, 7: 469-472 (1988).
**Horsch** *et al*, Science, 227: 1229 (1985).
**Howard** *et al*, Planta, 170: 535 (1987).
**Lagrimini** LM *et al*, Proc. Natl. Acad. Sci., USA 84: 7452, (1987).
**Laurell** and McKay, Methods Enzymology, 73: 339-361 (1981).
**Lathe** R *et al*, J. Mol. Bio., 183: 1-12 (1985).
**Linsmeier** und Skoog, Physiol. Plant., 18: 101-127 (1965).
**Logemann J** *et al*, Analyt. Biochem., 163: 16-20 (1987)
**Maniatis** *et al*, Molecular Cloning, A Laboratorv Manual , Cold Spring Harbor Laboratory, 1982.
**Maxam** and Gilbert, 'Sequencing end-labelled DNA with base-specific chemical cleavage' , in: Methods in Enzymology 65: 499-560, Academic Press, New York, London, (1980).
**Meins** & Lutz, Differentiation, 15: 1-6 (1979).
**Mohnen**, "Regulation of Glucanohydrolases in Nicotiana tabacum on the messanger RNA level" , Dissertation University of Illinois at Urbana-Champaign, 1985.
**Mohnen** *et al*, EMBO J., 4: 1631-1635 (1985).
**Morelli** *et al*, Nature, 315: 200 (1985).
**Murashige** und Skoog, Physiol. Plant., 15: 473-497 (1962).
**Negrutiu** I *et al*, Plant Mol. Biol., 8: 363-373 (1987).
**Neuhaus** *et al*, Theor. Appl. Genet., 74: 30 -36 (1987).
**Parent** JG and Asselin A, Can J. Bot., 62: 564-569 (1984).
**Petit** *et al*, Mol. Gen. Genet., 202: 388 (1986).
**Pietrzak** *et al*, Nucl. Acids Res., 14: 5857-5868 (1986).
**Potrykus** I and Shillito RD, Methods in Enzymology, Vol 118 , Plant Molecular Biology, eds.A and H Weissbach, Academic Press, Orlando, 1986. **Rhodes** *et al*, Biotechnology, 6: 56-60 (1988).
**Rogers** SG *et al*, Methods in Enzymology, 118: 630-633 (1986)
**Rothstein** SJ *et al*, Gene, 53: 153-161 (1987).
**Sambrook *et al*, Molecular Cloning, A Laboratory Manual, Second Edition (1989).**
**Sanger** *et al*, Proc. Natl. Acad. Sci., USA 74: 5463-5467 (1977).
**Schmidhauser** und Helinski, J. Bacteriol., 164: 446-455 (1985).
**Schocher** RJ *et al*, Bio/Technology, 4: 1093-1096(1986).
**Shillito** RD *et al*, Biotechnology, 7: 581-587 (1989).
**Shinshi** *et al*, Proc. Natl. Acad. Sci., USA 84: 89-93 (1987).
**Shinshi** *et al*, Planta, 164: 423-428 (1985).
**Shinshi** *et al*, Proc. Natl. Acad. Sci., USA 85: 5541-5545 (1988) .
**Shillito** *et al*, Bio Technology, 3: 1099-1103 (1985);
**Siegel** BZ und Galston AW, Plant Physiol, 42: 221-226 (1967)

**Southern** EM, J. Mol. Biol. 98: 503-517 (1975).
**Spena** *et al*, EMBO J., 4: 2736 (1985).
**Vierra** und Messing, Gene, 19: 259-268 (1982).
**Wang** Y-C *et al*, Plant Mol. Biol., 11: 433-439 (1988).
**Yamada** Y *et al*, Plant Cell Rep , 5: 85-88 (1986) .
**Yanisch-Perron** *et al*, Gene, 33: 103-119 (1985).

## IX SEQUENZPROTOKOLL

**SEQ ID NO:** 1
**ART DER SEQUENZ:** Nukleotid mit entsprechendem Protein
**SEQUENZLÄNGE:** 3850 Basenpaare

**STRANGFORM:** Doppelstrang
**TOPOLOGIE:** linear
**ART DES MOLEKÜLS:** Genom-DNA

**URSPRÜNGLICHE HERKUNFT**
**ORGANISMUS:** *Nicotiana tabacum* L. cv. Havana 425
**UNMITTELBARE EXPERIMENTELLE HERKUNFT**
**NAME DES ZELLKLONS:** λCHN17 [λ Phage]

**EIGENSCHAFTEN:** basiches Chitinasegen

```
GAATTCAATC AAAATGTGTT TTGTATATAG GGTGTCAACT ACTAATATAT    50
TGTTATTTTC TAAAGACATA CATGTATACA TGTAAAATTT ACCGAACTTT   100
ACGGATGTCG ATAACCCCTC TCGATATAGC ATAGGTCCGC CTCTGATTTA   150
CGAAGGGACA CGAGGAAATT CCTCTATGTA ATTAGTTTTA GCAGTTACAC   200
GTTAAAGTAT AAATACATAT TACTTTACCA TAGTTAAGAC CAAACATGTG   250
TATGATTGAC ATACATCTTG CATTCATTAA TTAATTTGAT TTGATGCGAT   300
TAAATTTTTT AAGGATAGAG TTTTTAGTCC AAGTTGAGCT AGTGTAACTC   350
TTATAGTCAA TTGGACTCTC TATTACTAGA TACTATATCA GTTCAAAAGA   400
CACCAATATT GTATTTTAAC AGAAGGAGGC AAATAAGAAA TTGCAAATTC   450
TCAATTCTTT TTAATTATAT CTAATGAACC AAAAGGAGGA AGAGGAGCTA   500
CATATTGGAT TTATAAATAT AAAGCTAGCT GAGGCTCAAA TAATTGTGGA   550
TGCAATACAA GCAATTTACT TAAAACACGA AACAGAAGAG GATTTCGGTC   600
AAATATCGAC ACCTAAGTTT TGAAATACAT TACTGAACAA ATTATGAGAT   650
CATAGACTAG TAATTTAGGA TATTATTCTG TGATTGACTT GATTTTGCAC   700
ATGAAGAAAC GTGAACGGCT TTCTTTTTAG GGCTGCCGTA GAATTGATCA   750
```

```
AAACATATCT CAACATATTA AAATAGGGTC TCAACTAAAC CGGATTCATG    800
CGGAATGAGA CCCATTTAAA AGGAGCAGTG GTTCCTATTC AAAGAATTAG    850
ATACATTTCT ACATATTTTT AATTATGAAA ATTACTCCTA TACTAATTTG    900
TGTGGTTTTA ATCGAATATG TAAATTTTAT TTGAAAATAA AATAAAAAAT    950
CACAGTCCAA CTTTAATCAT AACACTCAAA TTAAATTCAG CTATCTTTCT   1000
AGGACATAGG AAACATTATC AGTGGAAATA TTATATTATA TCCATAAGAC   1050
TTTAGCAAAT CCTATAAGAA GTCTAAACAT GTAATTGACT ACTTTTAGAA   1100
GACGCACTTA TCTAACCCAA GAAACACCTG GCGTAACTCG AATTTGCTTT   1150
TGCCAAAACC AAAAGTCTAG GAATTAAGCT CCAAATTAAA GACATAGATT   1200
TTGGCTTACT TTTTTTCAAA AAAAAAATAA AATTAAAAAT TAAAATATTT   1250
TTTGTTCATG TAATTTAATC AGTTTTTGGG TGAAATTTTT TCTTCCACAC   1300
ACAAGATTTT AACTTTTTTC CAAATAAAAT ACACGTCGAA ACATAAATTC   1350
AAATTTCAGA ACTATTTTTC AACGTAATTT TAAAATTTTT ATTTTCTAGT   1400
TTTAACTAAA TCTATGTCCT GATTAAGTCT CCAGTCTTAA CTCTTAAAGT   1450
ATTGAAAATA CATGTTCGAG AATTGTCTGG GATGAAGCTA AGAGCCGCCA   1500
CTAAGAAAAA AAATCTAAAA ATATATAAAA AGGTAAGAGC CGCCACATAA   1550
TATATGTAAC CTGTCGGCGT AATCTACTGA ATTAATTTTC TGGATAAGAA   1600
AGATATGACT GAGCTCCGGT TTGCTCATAG ATTTTGACTT TACTTTTTTA   1650
ATTTCTTTTT GAAAATATTG TTTGTTTAAT AAAATATGAT CATGTTTTAG   1700
AAAAACAAAT TTCAAAAAAC TTCAAGTTCC CAAAAGTTGT ATGTCCAAAC   1750
ACAACTTTCA AAAATTATTT TTTAAAACAA CTTTAAAAAC TTTTTTTTTA   1800
AATTTTAATT AAATCTATGT CCAAACTAGC CGAAATTCGA GCCTTGGTTA   1850
TTCAACCAAT TGATTTGGTC AGAAAGTCAG TCCTCTCAAC AACTAAAATA   1900
GACATTAATT AAGCCATGTC TCCAGCATCT TCCTTAGCAA TAAATACCTT   1950
GCATTTCACC AGTTTACTAC TACATTAAA ATG AGG CTT TGT AAA TTC   1997
                                Met Arg Leu Cys Lys Phe

ACA GCT CTC TCT TCT CTA CTA TTT TCT CTC CTA CTG CTT TCT   2039
Thr Ala Leu Ser Ser Leu Leu Phe Ser Leu Leu Leu Leu Ser

GCC TCG GCA GAA CAA TGT GGT TCC CAG GCC GGA GGT GCG CGT   2081
Ala Ser Ala Glu Gln Cys Gly Ser Gln Ala Gly Gly Ala Arg
```

```
TGT CCC TCG GGT CTC TGC TGC AGC AAA TTT GGT TGG TGT GGT  2123
Cys Pro Ser Gly Leu Cys Cys Ser Lys Phe Gly Trp Cys Gly

AAC ACC AAT GAC TAC TGT GGC CCT GGC AAT TGC CAG AGC CAG  2165
Asn Thr Asn Asp Tyr Cys Gly Pro Gly Asn Cys Gln Ser Gln

TGC CCT GGT GGT CCC ACA CCT ACA CCC CCC ACC CCA CCC GGT  2207
Cys Pro Gly Gly Pro Thr Pro Thr Pro Pro Thr Pro Pro Gly

GGT GGG GAC CTC GGC AGT ATC ATC TCA AGT TCC ATG TTT GAT  2249
Gly Gly Asp Leu Gly Ser Ile Ile Ser Ser Ser Met Phe Asp

CAG ATG CTT AAG CAT CGC AAC GAT AAT GCA TGC CAA GGA AAG  2291
Gln Met Leu Lys His Arg Asn Asp Asn Ala Cys Gln Gly Lys

GGA TTC TAC AGT TAC AAT GCC TTT ATC AAT GCT GCT CGG TCT  2333
Gly Phe Tyr Ser Tyr Asn Ala Phe Ile Asn Ala Ala Arg Ser

TTT CCT GGC TTT GGT ACC AGT GGC GAT ACC ACT GCC CGT AAA  2375
Phe Pro Gly Phe Gly Thr Ser Gly Asp Thr Thr Ala Arg Lys

AGA GAA ATC GCG GCT TTC TTT GCT CAA ACC TCC CAT GAA ACT  2417
Arg Glu Ile Ala Ala Phe Phe Ala Gln Thr Ser His Glu Thr

ACT GGT AAGTCTAGTT ACGTTGAACT ATATGATCGT CTTATTCAAA      2463
Thr Gly

AGTTTAATCA ATTAGAGAGA TCATACTTTT ATTTAATCAT ACTGGTCTAT   2513
TCTGATTTCA TGAGACAAAC ACATAGAACT TCCTTTTAAA ATGATTGCGC   2563
TGAGACTTGA ATTCAGGACC TCTATCTGCT CATCACTGGA GTATCCAATT   2613
TTGAGATATC ACAATGCTTC TTAAATTTCG AAGTTTTTTA TAAGCTGACG   2663
CGTTCAATAA TTGACCATGT AACCGTTGAC AGGA    GGA TGG GCA ACA  2709
                                         Gly Trp Ala Thr
```

```
GCA CCA GAT GGT CCA TAT GCA TGG GGT TAT TGC TGG CTT AGA  2751
Ala Pro Asp Gly Pro Tyr Ala Trp Gly Tyr Cys Trp Leu Arg

GAA CAA GGT AGC CCC GGC GAC TAC TGT ACC CCA AGT GGT CAG  2793
Glu Gln Gly Ser Pro Gly Asp Tyr Cys Thr Pro Ser Gly Gln

TGG CCT TGT GCT CCT GGT CGA AAA TAT TTC GGA CGA GGC CCC  2835
Trp Pro Cys Ala Pro Gly Arg Lys Tyr Phe Gly Arg Gly Pro

ATC CAA ATT TCA CAG TAAGTTCCTT CTTACCCACA CGGAGTGTTT     2880
Ile Gln Ile Ser His

ACACCAAAGT CGTGGGACGG AATGCTTACT ACCTACTATA TATTTCATTG   2930
TGAGAGTAGG TACACAATAT CATGATATTT CTATGATTAT AAGAGTATGT   2980
GATTAATTTC TATGAGAAGT GTAAAGTTAA ATAGTTTCCA CAACACAAAA   3030
AAAATGTCAT TTTTTTAACA GATTAAAAAA GAAAAAGTAT ATGATGAACT   3080
TGTAGGATCT AATTAAGTGT ATTTTGACAT AAATACAGC  AAC TAT AAC  3128
                                            Asn Tyr Asn

TAC GGG CCT TGT GGA AGA GCC ATA GGA GTG GAC CTG CTA AAC  3170
Tyr Gly Pro Cys Gly Arg Ala Ile Gly Val Asp Leu Leu Asn

AAT CCT GAT TTA GTG GCC ACA GAT CCA GTC ATC TCA TTT AAG  3212
Asn Pro Asp Leu Val Ala Thr Asp Pro Val Ile Ser Phe Lys

TCA GCT CTC TGG TTC TGG ATG ACT CCT CAA TCA CCA AAA CCT  3254
Ser Ala Leu Trp Phe Trp Met Thr Pro Gln Ser Pro Lys Pro

TCT TGC CAC GAT GTC ATC ATC GGA AGA TGG CAG CCA TCA GCT  3296
Ser Cys His Asp Val Ile Ile Gly Arg Trp Gln Pro Ser Ala
```

```
GGT GAT CGC GCA GCC AAT CGC CTC CCT GGA TTT GGC GTC ATC  3338
Gly Asp Arg Ala Ala Asn Arg Leu Pro Gly Phe Gly Val Ile

ACA AAC ATC ATC AAT GGT GGC TTG GAA TGT GGT CGT GGC ACT  3380
Thr Asn Ile Ile Asn Gly Gly Leu Glu Cys Gly Arg Gly Thr

GAC TCA AGG GTC CAG GAT CGC ATT GGG TTT TAC AGG AGG TAT  3422
Asp Ser Arg Val Gln Asp Arg Ile Gly Phe Tyr Arg Arg Tyr

TGC AGT ATT CTT GGA GTT AGT CCT GGT GAC AAT CTG GAT TGC  3464
Cys Ser Ile Leu Gly Val Ser Pro Gly Asp Asn Leu Asp Cys

GGC AAC CAG AGG TCT TTT GGA AAT GGA CTT TTA GTC GAT ACT  3506
Gly Asn Gln Arg Ser Phe Gly Asn Gly Leu Leu Val Asp Thr

ATG  TAATTTCATG ATCTGTTTTG TTGTATTCCC TTGCAATGCA         3549
Met

GGGCCTAGGG CTATGAATAA AGTTAATGTG TGAATGTGAA TGTGTGATTG   3599
TGACCTGAAG GGATCACGAC TATAATCGTT TATAATAAAC AAAGACTTTG   3649
TCCCAATATA TGTGTTAATG AGCATTACTG TAGTTGGTTT AATTCGGCAC   3699
CAGATAAATA GATAACCACC CGCACTATTA TATTTCATTA TTTAGAAAAC   3749
CGAGATCTTT ATTTGAGTGA ATGAAAATCT TCCTAACCAG ATAGTCATAC   3799
TAATCAGTCA AAAAAAAATC TAACCTCAAA ATTTAAGCAT CCGAGCTGCAG  3850
```

**SEQ ID NO:** 2

**ART DER SEQUENZ:** Nukleotid, Teilsequenz aus der 5'-Region von SEQ ID NO: 1

**SEQUENZLÄNGE:** 31 Basenpaare

TTGCATTTCA CCAGTTTACT ACTACATTAA A

**SEQ ID NO:** 3

**ART DER SEQUENZ:** Nukleotid, Teilsequenz aus der 5'-Region von SEQ ID NO: 1

**SEQUENZLÄNGE:** 14 Basenpaare

ACTACTACAT TAAA

**Ansprüche**

1. Regulatorische, aus der 5'- nicht-translatierten Region eines pflanzlichen Chitinasegens erhältliche DNA Sequenz, die, sofern sie mit exprimierbarer DNA sowie in pflanzlichen Zellen aktiven Expressionssignalen operabel verknüpft ist, bei Transformation in einen pflanzlichen Wirt zu einer signifikanten Erhöhung der Expressionsrate der jeweils operabel assoziierten exprimierbaren DNA führt.

2. Regulatorische DNA Sequenz gemäss Anspruch 1, dadurch gekennzeichnet, dass sie aus der 5'- nicht-translatierten Region eines basischen Tabak - Chitinasegens erhältlich ist.

3. Regulatorische DNA Sequenz gemäss Anspruch 2, dadurch gekennzeichnet, dass es sich bei diesem Sequenzabschnitt um eine A/T reiche Region handelt, die zu wenigstens 55% aus A/T besteht.

4. Regulatorische DNA Sequenz gemäss Anspruch 2, dadurch gekennzeichnet, dass es sich bei diesem Sequenzabschnitt um eine A/T reiche Region handelt, die zu wenigstens 70% aus A/T besteht.

5. Regulatorische DNA Sequenz gemäss Anspruch 2, dadurch gekennzeichnet, dass sie aus der 5'- nicht-translatierten Region eines basischen Chitinasegens von *Nicotiana tabacum* L. c.v. Havana 425 Pflanzen erhältlich ist und im wesentlichen die folgende DNA-Sequenz aufweist:

5'- **TTGCATTTCACCAGTTTACTACTACATTAAA** -3'

einschliesslich aller Derivate dieser DNA-Sequenz, die sich auf die Mutation einer oder mehrerer Basen stützen, die aber obiger Sequenz nach wie vor im wesentlichen homolog sind und die noch die spezifischen, regulatorischen, expressionssteigernden Eigenschaften der Ausgangssequenz aufweisen.

6. Regulatorische DNA Sequenz gemäss einem der Ansprüche 1 bis 5, dadurch gekennzeichnet, dass besagte DNA Sequenz in im wesentlichen reiner Form vorliegt.

7. Fragmente oder Teilsequenzen, die aus einer der regulatorischen DNA Sequenzen oder aus Derivaten dieser DNA Sequenzen gemäss einem der Ansprüche 1 bis 6 erhältlich sind und die noch die spezifischen, regulatorischen, expressionssteigernden Eigenschaften der Ausgangssequenz aufweisen.

8. Teilsequenz gemäss Anspruch 7, dadurch gekennzeichnet, dass sie die folgende DNA-Sequenz aufweist:

**5'-ACTACTACATTAAA-3'**

einschliesslich aller Derivate dieser DNA-Sequenz, die sich auf die Mutation einer oder mehrerer Basen stützen, die aber obiger Sequenz nach wie vor im wesentlichen homolog sind und die noch die spezifischen, regulatorischen, expressionssteigernden Eigenschaften der Ausgangssequenz aufweisen.

9. Basisches Chitinasegen erhältlich aus *Nicotiana tabacum* L. c.v. Havana 425 Pflanzen, das in seiner 5'-nichttranslatierten Region die folgenden strukturellen Besonderheiten aufweist:

(a) einen Transkriptionsstart innerhalb der Sequenz CTACT in Position 1967 bis 1971;
(b) ein erstes mögliches Startkodon in Position 1980, 11 Bp stromabwärts der Transkriptionsstartstelle;
(c) eine TAAATA-Sequenz ('TATA box') stromaufwärts der Transkriptionsstartstelle in Position -28 bis -23 der 5′-flankierenden Region;
(d) eine CCAATT-Sequenz in Position -114;
(e) eine 6 Bp umfassende unvollkommen invertierte Wiederholungssequenz (GCCGAATTCGAGC) in Position -140;
(f) eine 10 Bp umfassende vollkommene Wiederholungssequenz (ATGTCCAAAC) in Position -152 und -228;
(g) eine 20 Bp umfassende unvollkommene direkte Wiederholungssequenz (TTTTAACTAAATCTATGTCC) in Position -166 und -569;
(h) eine 25 Bp umfassende unvollkommene direkte Wiederholungssequenz (CAACTTTCAAAAATTATTTTTTAAA) in Position -191 und -217;
(i) ein 20 Bp umfassendes Palindrom (TAAAATATGA | TCATGTTTTA) in Position -289;
(j) eine 11 Bp umfassende vollkommene direkte Wiederholungssequenz (TAAGAGCCGCC) in Position -435 und -480;
(k) eine 15 Bp umfassende unvollkommene direkte Wiederholungssequenz (TAAAATACACGTCGA) in Position -514 und -644; und
(l) zwei AATAAA Sequenzen in Position 52 und 120, stomabwarts der Translationsstop Sequenz TAA in der 3′-flankierenden Region.

10. Basisches Chitinasegen erhältlich aus *Nicotiana tabacum* L. c.v. Havana 425 Pflanzen, das die in Figur 6 wiedergegebene Nukleotidsequenz aufweist.

11. Rekombinantes DNA Molekül, dadurch gekennzeichnet, dass es sich um eine chimäre genetische Konstruktion handelt, worin eine regulatorische DNA-Sequenz gemäss einem der Ansprüche 1 bis 8 mit einer exprimierbaren DNA sowie mit weiteren, in pflanzlichen Zellen aktiven Expressionssignalen, operabel verknüpft ist, sodass es bei Transformation in einen pflanzlichen Wirt zu einer signifikanten Erhöhung der Expressionsrate der operabel assoziierten exprimierbaren DNA kommt.

12. Rekombinantes DNA Molekül gemäss Anspruch 11, dadurch gekennzeichnet, dass zwischen der Promotorsequenz und der sich anschliessenden regulatorischen DNA Segeuenz eine 'Spacer'-Sequenz inseriert ist, wobei die Länge der 'Spacer'-Sequenz so gewählt ist, dass der Abstand zwischen dem Promotor und der regulatorischen DNA-Sequenz für die Expression des jeweils assoziierten Strukturgens optimal ist.

13. Rekombinantes DNA Molekül gemäss Anspruch 12, dadurch gekennzeichnet, dass die 'Spacer'-Sequenz zwischen 1 Bp und 100 Bp umfasst.

14. Rekombinantes DNA Molekül gemäss Anspruch 11, dadurch gekennzeichnet, dass es sich um Expressionssignale handelt, die aus Genen von Pflanzen oder Pflanzenviren stammen.

15. Rekombinantes DNA Molekül gemäss Anspruch 11, dadurch gekennzeichnet, dass es sich um bakterielle Expressionssignale handelt.

16. Rekombinantes DNA Molekül gemäss Anspruch 14, dadurch gekennzeichnet, dass es sich um Promotor- und/oder Terminationssignale von Cauliflower Mosaikvirus Genen (CaMV) handelt.

17. Rekombinantes DNA Molekül gemäss Anspruch 15, dadurch gekennzeichnet, dass es sich um die Expressionssignale der Nopalin-Synthase-Gene (nos) und/oder der Octopin-Synthase-Gene (ocs) aus den Ti-Plasmiden von *Agrobacterium tumefaciens* handelt.

18. Rekombinantes DNA Molekül gemäss Anspruch 11, dadurch gekennzeichnet, dass es zusätzliche regulatorische DNA-Sequenzen enthält, die in der Lage sind die Transkription einer assoziierten DNA-Sequenz in pflanzlichen Geweben im Sinne einer Induktion oder Repression zu regulieren.

19. Rekombinantes DNA Molekül gemäss Anspruch 18, dadurch gekennzeichnet, dass besagte regulatorische DNA-Sequenzen durch verschiedene interne undexterne Faktoren, ausgewählt aus der Gruppe bestehend aus Pflanzenhormonen, Hitzeschock, Chemikalien, Pathogenen, Sauerstoffmangel oder Licht, induziert werden.

20. Rekombinantes DNA Molekül gemäss Anspruch 11, dadurch gekennzeichnet, dass es sich bei der mit der regulatorischen DNA Sequenz assoziierten exprimierbaren DNA um ein Strukturgen handelt, das den transformierten pflanzlichen Zellen sowie den daraus sich entwickelnden Geweben und insbesondere aber den Pflanzen einen Schutzeffekt gegenüber Pathogenen, Chemikalien sowie nachteiligen Umwelteinflüssen verleiht.

21. Rekombinantes DNA Molekül gemäss Anspruch 20, dadurch gekennzeichnet, dass es sich bei besagtem Strukturgen um ein Gen handelt, das Chitinase in pflanzlichen Zellen exprimiert.

22. Rekombinantes DNA Molekül gemäss Anspruch 20, dadurch gekennzeichnet, dass es sich bei besagtem Strukturgen um ein Gen handelt, das Glucanase in pflanzlichen Zellen exprimiert.

23. Rekombinantes DNA Molekül gemäss Anspruch 20, dadurch gekennzeichnet, dass es sich bei besagtem Strukturgen um ein Gen handelt, das einen Proteaseinhibitor in pflanzlichen Zellen exprimiert.

24. Rekombinantes DNA Molekül gemäss Anspruch 20, dadurch gekennzeichnet, dass es sich bei besagtem Strukturgen um ein Herbizidresistenz-Gen handelt.

25. Rekombinantes DNA Molekül gemäss Anspruch 20, dadurch gekennzeichnet, dass es sich bei besagtem Strukturgen um ein Gen handelt, das ein δ-Endotoxin aus *Bacillus thuringiensis* in pflanzlichen Zellen exprimiert.

26. Rekombinantes DNA Molekül gemäss Anspruch 11, dadurch gekennzeichnet, dass die mit der regulatorischen DNA Sequenz assoziierten Strukturgene bei Expression in der transformierten pflanzlichen Zelle als solcher oder als Bestandteil einer höher organisierten Einheit ausgewählt aus der Gruppe bestehend aus einem Gewebe, Organ, Kallus, Embryo oder einer ganzen Pflanze zu einer Produkitonssteigerung gewünschter und nützlicher Verbindungen führt.

27. Rekombinantes DNA Molekül gemäss Anspruch 11, dadurch gekennzeichnet, dass es sich bei besagter exprimierbarer DNA-Sequenz um 'anti-sense' DNA handelt.

28. Rekombinantes DNA Molekül gemäss Anspruch 11, dadurch gekennzeichnet, dass es zusätzlich eine DNA Sequenz enthält, die für einen selektierbaren phaenotypischen Marker kodiert.

29. Rekombinantes DNA Molekül gemäss Anspruch 28, dadurch gekennzeichnet, dass es sich bei besagtem selektierbaren phaenotypischen Marker um eine Resistenz gegenüber Antibiotika, ausgewählt aus der Gruppe bestehend aus Ampicillin, Tetracyclin, Hygromycin, Kanamycin, Methotrexat, G418 und Neomycin, handelt.

30. Rekombinantes DNA Molekül gemäss Anspruch 11, dadurch gekennzeichnet, dass es zusätzlich einen Replikationsursprung enthält, der eine Replikation in Mikroorganismen zulässt.

31. Rekombinantes DNA Molekül gemäss Anspruch 30, dadurch gekennzeichnet, dass besagter Replikationsursprung in *E. coli*, in *Agrobacterium* oder in beiden funktionsfähig ist.

32. Klonierungsvektor enthaltend ein rekombinantes DNA Molekül gemäss einem der Ansprüche 11 bis 31.

33. Transformationsvektor enthaltend ein rekombinantes DNA Molekül gemäss einem der Ansprüche 11 bis 31.

34. Shuttle Vektor enthaltend ein rekombinantes DNA Molekül gemäss Anspruch 31, der in der Lage ist sowohl in *E. coli* als auch in *A. tumefaciens* stabil zu replizieren.

35. Shuttle Vektor gemäss Anspruch 34, dadurch gekennzeichnet, dass die chimäre genetische Konstruktion zwischen der Linken (LB) und Rechten Bordersequenz (RB), erhältlich aus dem Ti-Plasmid von *A. tumefaciens*, einkloniert ist.

36. Wirtsorganismus enthaltend ein rekombinantes DNA Molekül gemäss einem der Ansprüche 11 bis 31.

37. Wirtsorganismus enthaltend einen Vektor gemäss einem der Ansprüche 32 bis 35.

38. Wirtsorganismus gemäss einem der Ansprüche 36 oder 37, dadurch gekennzeichnet, dass es sich bei besagtem Wirtsorganismus um ein Bakterium handelt.

39. Wirtsorganismus gemäss einem der Ansprüche 36 oder 37, dadurch gekennzeichnet, dass es sich bei besagtem Wirtsorganismus um pflanzliches Material handelt, ausgewählt aus der Gruppe bestehend aus Protoplasten, Zellen, Kalli, Geweben, Organen, Samen, Embryonen, Pollen, Eizellen, Zygoten, etc.

40. Transgene Pflanze einschliesslich ihrer sexuellen und asexuellen Nachkommenschaft enthaltend ein rekombinantes DNA Molekül gemäss einem der Ansprüche 11 bis 31.

41. Transgene Pflanze einschliesslich ihrer sexuellen und asexuellen Nachkommenschaft mit einem gegenüber dem Wildtyp signifikant erhöhten Proteingehalt in den transformieten Zellen und/oder Geweben.

42. Transgene Pflanze einschliesslich ihrer sexuellen und asexuellen Nachkommenschaft gemäss Anspruch 41 mit einem gegenüber dem Wildtyp signifikant erhöhten Chitinasegehalt.

43. Transgene Pflanze einschliesslich ihrer sexuellen und asexuellen Nachkommenschaft gemäss Anspruch 41 mit einem gegenüber dem Wildtyp signifikant erhöhten Glucanasegehalt.

44. Transgene Pflanze regeneriert aus transformiertem pflanzlichemmaterial gemäss Anspruch 39.

45. Transgene Pflanze gemäss einem der Ansprüche 40 bis 44 , dadurch gekennzeichnet, dass es sich um eine fertile Pflanze handelt.

46. Vermehrungsgut einer transgenen Pflanze gemäss einem der Ansprüche 40 bis 45.

47. Teile einer transgenen Pflanze gemäss einem der Ansprüche 40 bis 45.

48. Verfahren zur Herstellung der in Anspruch 1 charakterisierten regulatorischen, expressionssteigernden DNA Sequenz, dadurch gekennzeichnet, dass man besagte Sequenz aus der 5'-nicht-translatierten Region eines basischen Chitinasegens unter Anwendung bekannter Massnahmen isoliert.

49. Verfahren gemäss Anspruch 48, dadurch gekennzeichnet, dass es sich bei besagter regulatorischer DNA Sequenz im wesentlichen um die folgende Nukleotid-Sequenz handelt:

5'- TTGCATTTCACCAGTTTACTACTACATTAAA -3'
einschliesslich aller Derivate dieser DNA-Sequenz, die sich auf die Mutation einer oder mehrerer Basen stützen, die aber obiger Sequenz nach wie vor im wesentlichen homolog sind und die noch die spezifischen, regulatorischen, expressionssteigernden Eigenschaften der Ausgangssequenz aufweisen.

50. Verfahren gemäss Anspruch 48, das durch die folgenden Verfahrensmassnahmen charakterisiert ist:

(a) Extrahieren und Reinigen von genomischer DNA aus Geweben, die in der Lage sind Chitinase zu exprimieren;

(b) Zerlegen der extrahierten und gereinigten DNA Präparationen in Fragmente einer für die nachfolgende Insertion in einen Klonierungs-Vektor geeigneten Grösse;

(c) Einklonieren der fragmentierten DNA in einen Klonierungvektor und Herstellen einer Genbibliothek;

(d) Auslesen von Klonen, die das Chitinasegen oder Teile davon enthalten mit Hilfe von Probenmolekülen;

(e) Isolierung derjenigen Klone, die mit dem Probenmolekül ein starkes Hybridisierungssignal zeigen;

(f) Charakterisierung der in (e) isolierten Klone mit Hilfe biochemischer Verfahren; und

(g) Identifizierung und Isolierung der für die Expressionssteigerung verantwortlichen regulatorischen DNA Sequenz.

51. Verfahren gemäss Anspruch 50, dadurch gekennzeichnet, dass man für die Herstellung einer genomischen Genbibliothek Expressionsvektoren verwendet.

52. Verfahren gemäss Anspruch 50, dadurch gekennzeichnet, dass man als Probenmoleküleinen bekannten Chitinaseklon oder Teile davon verwendet.

53. Verfahren zur Herstellung der in Anspruch 1 charakterisierten regulatorischen, expressionssteigernden DNA Sequenz, dadurch gekennzeichnet, dass man besagte regulatorische DNA Sequenz sowie die davon abgeleiteten Derivate gleicher Funktion mit Hilfe chemischer Verfahren synthetisiert.

54. Verfahren zur Herstellung eines rekombinanten DNA Moleküls gemäss Anspruch 11, dadurch gekennzeichnet dass man eine regulatorische DNA Sequenz gemäss Anspruch 1 unmittelbar vor dem Startkodon eines gewünschten Strukturgens inseriert und diese Konstruktion mit in pflanzlichen Zellen aktiven Expressionssignalen operabel verknüpft.

55. Verfahren gemäss Anspruch 54, dadurch gekennzeichnet, dass man zwischen dem Promotor und der regulatorischen DNA Sequenz eine 'Spacer'-Sequenz inseriert.

56. Verfahren gemäss Anspruch 54, dadurch gekennzeichnet, dass es sich bei besagtem Strukturgen um ein Chitinase- oder Glucanasegen handelt.

57. Verfahren gemäss Anspruch 54, dadurch gekennzeichnet, dass es sich bei besagtem Strukturgen um ein einen Proteinaseinhibitor-kodierendes Gen handelt.

58. Verfahren gemäss Anspruch 54, dadurch gekennzeichnet, dass es sich bei besagtem Strukturgen um ein Herbizid resistenzgen handelt.

59. Verfahren gemäss Anspruch 54, dadurch gekennzeichnet, dass es sich bei besagtem Strukturgen um ein $\delta$-Endotoxingen aus *Bacillus thuringiensis* handelt.

60. Verfahren zur Herstellung eines Expressionsvektors, welcher eine in pflanzlichen Zellen exprimierbare DNA in operabler Verknüpfung mit einer regulatorischen DNA Sequenz gemäss Anspruch 1 enthält, dadurch gekennzeichnet, dass man

(a) besagte regulatorische DNA Sequenz unmittelbar vor dem Startkodon einer gewünschten exprimierbaren DNA inseriert; und

(b) dieses Konstrukt in einen bekannten Pflanzenexpressionsvektor, der gegebenenfalls ein für die Transformantenselektion geeignetes Markergen enthalten kann, zwischen in pflanzlichen Zellen aktiven Expressionssignalen operabel einspleisst.

61. Verfahren gemäss Anspruch 60, dadurch gekennzeichnet, dass das Einspleissen in die Polylinkerregion des Pflanzenexpressionsvektors erfolgt, die zwischen dem Promotor und dem Terminator lokalisiert ist.

62. Verfahren zur Herstellung einer transgenen Pflanze mit gegenüber dem Wildtyp erhöhtem Proteingehalt in den transformierten Zellen und/oder Geweben, dadurch gekennzeichnet, dass man besagte Pflanze unter Verwendung bekannter Verfahren mit einem rekombinaten DNA Molekül gemäss einem der Ansprüche 11 bis 31 transformiert.

63. Verfahren zur Herstellung einer transgenen Pflanze gemäss Anspruch 62 mit einem gegenüber dem Wildtyp signifikant erhöhten Chitinasegehalt, dadurch gekennzeichnet, dass man besagte Pflanze mit einem rekombinanten DNA Molekül gemäss Anspruch 21 transformiert und das eingeschleuste Chitinasegen exprimiert.

64. Verfahren zur Herstellung einer transgenen Pflanze gemäss Anspruch 62 mit einem gegenüber dem Wildtyp signifikant erhöhten Glucanasegehalt, dadurch gekennzeichnet, dass man besagte Pflanze mit einem rekombinanten DNA Molekül gemäss Anspruch 22 transformiert und das eingeschleuste Glucanasegen exprimiert.

65. Verfahren zum Schutz von Pflanzen vor chitinhaltigen Pathogenen, dadurch gekennzeichnet, dass man besagte Pflanzen mit einem rekombinanten DNA Molekül gemäss Anspruch 21 transformiert und das eingeschleuste Chitinasegen in einer Menge exprimiert, die ausreicht, die Pathogene abzutöten oder aber diese unter Kontrolle zu halten.

66. Verfahren zum Schutz von Pflanzen vor chitinhaltigen Pathogenen, dadurch gekennzeichnet, dass man besagte Pflanzen mit einem rekombinanten DNA Molekül gemäss Anspruch 22 transformiert und das eingeschleuste Glucanasegen in einer Menge exprimiert, die ausreicht, die Pathogene abzutöten oder aber diese unter Kontrolle zu halten.

67. Verfahren zur Bekämpfung chitinhaltiger Pathogene, dadurch gekennzeichnet, dass man besagte Pathogene mit einer transgenen Pflanze oder Teilen dieser Pflanze in Kontakt bringt, die mit einem rekombinanten DNA Molekül gemäss einem der Ansprüche 21 oder 22 transformiert sind und die das eingeschleuste Chitinase- bzw. Glucansasegen in einer Menge exprimieren, die ausreicht, die Pathogene abzutöten oder aber diese unter Kontrolle zu halten.

68. Verfahren zur Herstellung eines basischen Chitinasegens gemäss Anspruch 9, das durch die folgenden Verfahrensmassnahmen charakterisiert ist:

(1) Extrahieren und Reinigen von genomischer DNA aus Geweben, die in der Lage sind Chitinase zu exprimieren;

(2) Zerlegen der extrahierten und gereinigten DNA Präparationen in Fragmente einer für die nachfolgende Insertion in einen Klonierungs-Vektor geeigneten Grösse;

(3) Einklonieren der fragmentierten DNA in einen Klonierungsvektor und Herstellen einer Genbibliothek;

(4) Auslesen von Klonen, die das Chitinasegen oder Teile davon enthalten mit Hilfe von Probenmolekülen;

(5) Isolierung derjenigen Klone, die mit dem Probenmolekül ein starkes Hybridisierungssignal zeigen;

(6) Charakterisierung der in (5) isolierten Klone mit Hilfe biochemischer Verfahren.

69. Verfahren gemäss Anspruch 68, dadurch gekennzeichnet, dass man für die Herstellung einer genomischen Genbibliothek Expressionsvektoren verwendet.

70. Verfahren gemäss einem der Ansprüche 68 oder 69, dadurch gekennzeichnet, dass man die genomische Genbibliothek mit Hilfe der Plaquehybridisierung oder der differentiellen Koloniehybridisierung, unter Verwendung eines zuvor isolierten cDNA Klons als Probenmolekul oder unter Verwendung immunologischer Nachweismethoden, die auf einer Identifizierung der spezifischen Translationsprodukte beruhen, screent.

71. Verfahren gemäss Anspruch 70, dadurch gekennzeichnet, dass es sich bei besagtem cDNA Klon um pCHN48 bzw. pCHN50 oder um funktionelle Äquivalente davon handelt.

72. Verfahren zum Aufspüren neuer regulatorischer DNA Sequenzen, dadurch gekennzeichnet, dass man eine regulatorische DNA Sequenz oder Teilsequenzen davon gemäss einem der Ansprüche 1 bis 8 dazu verwendet um homologe DNA Sequenzen gleicher Funktion aufzuspüren, indem man zunächst genomische oder cDNA Genbibliotheken herstellt und diese unter Verwendung der erfindungsgemässen, regulatorischen DNA Sequenz als Probenmolekül, auf das Vorhandensein homologer DNA Sequenzen untersucht, die in der Lage sind mit dieser zu hybridisieren.

73. Verwendung der in Anspruch 1 charakterisierten regulatorischen DNA Sequenz zur Steigerung der Genexpression in pflanzlichem Material.

74. Verwendung der in Anspruch 1 charakterisierten regulatorischen DNA Sequenz zum Aufspüren homologer DNA Sequenzen gleicher Funktion, dadurch gekennzeichnet, dass man genomische Genbibliotheken herstellt und diese unter Verwendung besagter regulatorischer DNA Sequenz als Probenmolekül auf das Vorhandensein homologer DNA Sequenzen untersucht, die in der Lage sind mit dem Probenmolekül zu hybridisieren.

Patentansprüche für folgenden Vertragsstaat: ES

1. Verfahren zur Herstellung einer regulatorischen DNA Sequenz, die, sofern sie mit exprimierbarer DNA sowie in pflanzlichen Zellen aktiven Expressionssignalen operabel verknüpft ist, bei Transformation in einen pflanzlichen Wirt zu einer signifikanten Erhöhung der Expressionsrate der jeweils operabel assoziierten exprimierbaren DNA führt und die im wesentlichen die folgende Nukleotid-Sequenz aufweist:

5′- **TTGCATTTCACCAGTTTACTACTACATTAAA** -3′

sowie von Derivaten dieser DNA-Sequenz, die obiger Sequenz nach wie vor im wesentlichen homolog sind und noch die spezifischen, regulatorischen, expressionssteigernden Eigenschaften der Ausgangssequenz aufweisen, dadurch gekennzeichnet, dass man besagte Sequenz oder eines ihrer Derivate

(a) aus der 5′-nicht-translatierten Region eines pflanzlichen Chitinasegens unter Anwendung bekannter Massnahmen isoliert; oder

(b) mit Hilfe chemischer Verfahren synthetisiert.

2. Verfahren gemäss Anspruch 1, dadurch gekennzeichnet, dass man besagte Sequenz oder eines ihrer Derivate aus der 5′-nicht-translatierten Region eines basischen Tabak-Chitinasegens isoliert.

3. Verfahren gemäss Anspruch 1, das durch die folgenden Verfahrensmassnahmen charakterisiert ist:

(a) Extrahieren und Reinigen von genomischer DNA aus Geweben, die in der Lage sind Chitinase zu exprimieren;

(b) Zerlegen der extrahierten und gereinigten DNA Präparationen in Fragmente einer für die nachfolgende Insertion in einen Klonierungs-Vektor geeigneten Grösse;

(c) Einklonieren der fragmentierten DNA in einen Klonierungsvektor und Herstellen einer Genbibliothek;

(d) Auslesen von Klonen, die das Chitinasegen oder Teile davon enthalten mit Hilfe von Probenmolekülen;

(e) Isolierung derjenigen Klone, die mit dem Probenmolekül ein starkes Hybridisierungssignal zeigen;

(f) Charakterisierung der in (e) isolierten Klone mit Hilfe biochemischer Verfahren; und

(g) Identifizierung und Isolierung der für die Expressionssteigerung verantwortlichen regulatorischen DNA Sequenz.

4. Verfahren gemäss Anspruch 3, dadurch gekennzeichnet, dass man für die Herstellung einer genomischen Genbibliothek Expressionsvektoren verwendet.

5. Verfahren gemäss Anspruch 3, dadurch gekennzeichnet, dass man als Probenmoleküleinen einen bekannten Chitinaseklon oder Teile davon verwendet.

6. Verfahren gemäss Anspruch 3, dadurch gekennzeichnet, dass man als Probenmoleküleinen ein synthetisch hergestelltes Oligonucleotid verwendet, das einem Teil eines bekannten Chitinasegens homolog ist.

7. Verfahren gemäss Anspruch 3, dadurch gekennzeichnet, dass man die genomische Genbibliothek mit Hilfe der Plaquehybridisierung oder der differentiellen Koloniehybridisierung, unter Verwendung eines geeigneten cDNA Klons als Probenmolekül oder mit Hilfe immunologischer Nachweismethoden, die auf einer Identifizierung der spezifischen Translationsprodukte beruhen, screent.

8. Verfahren gemäss Anspruch 5, dadurch gekennzeichnet, dass es sich bei besagtem cDNA Klon um pCHN48 bzw. pCHN50 oder um funktionelle Äquivalente davon handelt.

9. Verfahren gemäss Anspruch 2, dadurch gekennzeichnet, dass man besagte regulatorische DNA Sequenz sowie die davon abgeleiteten Derivate gleicher Funktion unterverwendung der Phosphotriester- oder der Phosphitmethode synthetisiert.

10. Verfahren gemäss Anspruch 1, dadurch gekennzeichnet, dass es sich bei besagten Derivaten um Fragmente oder Teilsequenzen der regulatorischen DNA Sequenz handelt, die noch die spezifischen, regulatorischen, expressionssteigernden Eigenschaften der Ausgangssequenz aufweisen.

11. Verfahren gemäss Anspruch 10, dadurch gekennzeichnet, dass besagte Teilsequenz die folgende Nukleotid-Sequenz aufweist:

**5′-ACTACTACATTAAA-3′**

12. Verfahren zur Herstellung eines rekombinanten DNA Moleküls, welches eine chimäre genetische Konstruktion bestehend aus einer regulatorische DNA Sequenz gemäss Anspruch 1 und einer exprimierbaren DNA enthält, dadurch gekennzeichnet dass man besagte regulatorische DNA Sequenz unmittelbar vor dem Startkodon einer gewünschten exprimierbaren DNA inseriert und diese Konstruktion mit in pflanzlichen Zellen aktiven Expressionssignalen operabel verknüpft.

13. Verfahren gemäss Anspruch 12, dadurch gekennzeichnet, dass man zwischen dem Promotor und der regulatorischen DNA Sequenz eine 'Spacer'-Sequenz inseriert.

14. Verfahren gemäss Anspruch 13, dadurch gekennzeichnet, dass die 'Spacer'-Sequenz zwischen 1 Bp und 100 Bp umfasst.

15. Verfahren gemäss Anspruch 12, dadurch gekennzeichnet, dass es sich bei der mit der regulatorischen DNA Sequenz assoziierten exprimierbaren DNA um ein Strukturgen handelt, das den transformierten pflanzlichen Zellen sowie den daraus sich entwickelnden Geweben und insbesondere aber den Pflanzen einen Schutzeffekt gegenüber Pathogenen, Chemikalien sowie nachteiligen Umwelteinflüssen verleiht.

16. Verfahren gemäss Anspruch 15, dadurch gekennzeichnet, dass es sich bei besagtem Strukturgen um ein Chitinase- oder Glucanasegen handelt.

17. Verfahren gemäss Anspruch 15, dadurch gekennzeichnet, dass es sich bei besagtem Strukturgen um ein einen Proteinaseinhibitor-kodierendes Gen handelt.

18. Verfahren gemäss Anspruch 15, dadurch gekennzeichnet, dass es sich bei besagtem Strukturgen um ein Herbizide resistenzgen handelt.

19. Verfahren gemäss Anspruch 15, dadurch gekennzeichnet, dass es sich bei besagtem Strukturgen um ein δ-Endotoxingen aus *Bacillus thuringiensis* handelt.

20. Verfahren gemäss Anspruch 12, dadurch gekennzeichnet, dass es sich bei der mit einer regulatorischen DNA Sequenz assoziierten, exprimierbaren DNA-Sequenz um 'anti-sense' DNA handelt.

21. Verfahren gemäss Anspruch 12, dadurch gekennzeichnet, dass besagtes rekombinantes DNA Molekül zusätzlich eine DNA Sequenz enthält, die für einen selektierbaren phaenotypischen Marker kodiert.

22. Verfahren gemäss Anspruch 21, dadurch gekennzeichnet, dass es sich bei besagtem selektierbaren phaenotypischen Marker um eine Resistenz gegenüber Antibiotika, ausgewählt aus der Gruppe bestehend aus Ampicillin, Tetracyclin, Hygromycin, Kanamycin, Methotrexat, G418 und Neomycin, handelt.

23. Verfahren gemäss Anspruch 12, dadurch gekennzeichnet, dass besagtes rekombinantes DNA Molekül zusätzlich einen Replikationsursprung enthält, der eine Replikation in Mikroorganismen zulässt.

24. Verfahren gemäss Anspruch 23, dadurch gekennzeichnet, dass besagter Replikationsursprung in *E. coli*, in *Agrobacterium* oder in beiden funktionsfähig ist.

25. Verfahren gemäss Anspruch 12, dadurch gekennzeichnet, dass es sich um Expressionssignale handelt, die aus Genen von Pflanzen oder Pflanzenviren stammen.

26. Verfahren gemäss Anspruch 12, dadurch gekennzeichnet, dass es sich um bakterielle Expressionssignale handelt.

27. Verfahren gemäss Anspruch 25, dadurch gekennzeichnet, dass es sich um Promotor- und/oder Terminationssignale von Cauliflower Mosaik Virus Genen (CaMV) handelt.

28. Verfahren gemäss Anspruch 26, dadurch gekennzeichnet, dass es sich um die Expressionssignale der Nopalin-Synthase-Gene (nos) und/oder der Octopin-Synthase-Gene (ocs) aus den Ti-Plasmiden von *Agrobacterium tumefaciens* handelt.

29. Verfahren gemäss Anspruch 12, dadurch gekennzeichnet, dass besagtes rekombinantes DNA Molekül zusätzliche regulatorische DNA-Sequenzen enthält, die in der Lage sind die Transkription einer assoziierten DNA-Sequenz in pflanzlichen Geweben im Sinne einer Induktion oder Repression zu regulieren.

30. Verfahren gemäss Anspruch 29, dadurch gekennzeichnet, dass besagte regulatorische DNA-Sequenzen durch verschiedene interne und externe Faktoren, ausgewählt aus der Gruppe bestehend aus Pflanzenhormonen, Hitzeschock, Chemikalien, Pathogenen, Sauerstoffmangel oder Licht, induziert werden.

31. Verfahren zur Herstellung eines Expressionsvektors, welcher eine in pflanzlichen Zellen exprimierbare DNA in operabler Verknupfung mit einer regulatorischen DNA gemäss Anspruch 1 enthält, dadurch gekennzeichnet, dass man

(a) besagte regulatorische DNA Sequenz unmittelbar vor dem Startkodon einer gewünschten exprimierbaren DNA inseriert; und

(b) dieses Konstrukt in einen bekannten Pflanzenexpressionsvektor, der gegebenenfalls ein für die Transformantenselektion geeignetes Markergen enthalten kann, zwischen in pflanzlichen Zellen aktiven Expressionssignalen operabel einspleisst.

32. Verfahren gemäss Anspruch 31, dadurch gekennzeichnet, dass es sich bei besagtem Pflanzenexpressionsvektor um pGY1 handelt.

33. Verfahren gemäss Anspruch 31, dadurch gekennzeichnet, dass das Einspleissen in die Polylinkerregion des Pflanzenexpressionsvektors erfolgt, die zwischen dem Promotor und dem Terminator lokalisiert ist.

34. Verfahren zur Herstellung von pflanzlichem Material ausgewählt aus der Gruppe bestehend aus Protoplasten, Zellen, Kalli, Geweben, Organen, Samen, Embryonen, Pollen, Eizellen, Zygoten, etc., dadurch gekennzeichnet, dass man besagtes pflanzliches Material unter Verwendung bekannter Verfahren mit einem gemäss einem der Ansprüche 12 bis 30 hergestellten rekombinaten DNA Molekül transformiert.

35. Verfahren zur Herstellung einer transgenen Pflanze mit gegenüber dem Wildtyp erhöhtem Proteingehalt in den transformierten Zellen und/oder Geweben, dadurch gekennzeichnet, dass man besagte Pflanze unter Verwendung bekannter Verfahren mit einem gemäss einem der Ansprüche 12 bis 30 hergestellten rekombinaten DNA Molekül transformiert.

36. Verfahren zur Herstellung einer transgenen Pflanze gemäss Anspruch 35 mit einem gegenüber dem Wildtyp signifikant erhöhten Chitinasegehalt, dadurch gekennzeichnet, dass man besagte Pflanze mit einem gemäss Anspruch 16 hergestellten rekombinanten DNA Molekül transformiert und das einge schleuste Chitinasegen exprimiert.

37. Verfahren zur Herstellung einer transgenen Pflanze gemäss Anspruch 35 mit einem gegenüber dem Wildtyp signifikant erhöhten Glucanasegehalt, dadurch gekennzeichnet, dass man besagte Pflanze mit einem gemäss Anspruch 16 hergestellten rekombinanten DNA Molekül transformiert und das eingeschleuste Glucanasegen exprimiert.

38. Verfahren gemäss einem der Ansprüche 34 oder 35, dadurch gekennzeichnet, dass die Transformation mit Hilfe bekannter Verfahren, ausgewählt aus der Gruppe bestehend aus Polyethylenglykolbehandlung, Hitzeschockbehandlung und Elektroporation pflanzlicher Protoplasten; Mikroinjektion in pflanzliche Protoplasten, Zellen oder Embryonen; Beschuss von Zellen oder Geweben mit Mikroprojktilen; *Agrobacterium*-

vermittelter Transformation pflanzlicher Protoplasten, Zellen, Gewebe oder ganzer Pflanzen; sowie CaMV-vermittelter Transformation pflanzlicher Protoplasten, Zellen, Gewebe oder ganzer Pflanzen.

39. Verfahren zur Herstellung transgener Pflanzen, die einer Regeneration ausgehend von pflanzlichem Material zugänglich sind, dadurch gekennzeichnet, dass man

(a) pflanzliches Material ausgewählt aus der Gruppe bestehend aus Protoplasten, Zellen, Kalli, Geweben, Organen, Samen, Embryonen, Pollen, Eizellen, Zygoten, etc. unter Verwendung bekannter Verfahren mit einem gemäss einem der Ansprüche 12 bis 30 hergestellten rekombinaten DNA Molekül transformiert; und

(b) ausgehend von diesem transformierten Material in vitro ganze Pflanzen regeneriert.

40. Verfahren zur Herstellung von transgenem Saatgut von Pflanzen, die nach einem Verfahren gemäss Anspruch 35 hergestellt worden sind, dadurch gekennzeichnet, dass man besagte Pflanzen sexuell vermehrt und die sich entwickelnden Samen, die noch das eingeschleuste genetische Material enthalten, mit Hilfe bekannter Verfahren gewinnt.

41. Verfahren zur Herstellung transformierter, lebensfähiger Teile von Pflanzen, die gemäss einem Verfahren nach Anspruch 35 hergestellt worden sind, dadurch gekennzeichnet, dass man ausgehend von besagten Pflanzen mit Hilfe bekannter Methoden transformierte Pflanzenteile ausgewählt aus der Gruppe bestehend aus Protoplasten, Zellen, Zellklonen, Zellagglomeraten, Kallus- und/oder Gewebekulturen, Samen, Pollen, Eizellen, Zygoten oder Embryonen gewinnt und diejenigen selektioniert, die noch das eingeschleuste genetische Material enthalten.

42. Verfahren zur Herstellung von Kreuzungs- und Fusionsprodukten unter Verwendung des in Anspruch 34 definierten transformierten Pflanzenmaterial, die noch das eingeschleuste genetische Material enthalten, dadurch gekennzeichnet dass man besagtes Pflanzenmaterial untereinander oder mit Fremdmaterial kreuzt oder fusioniert und diejenigen Kreuzungs- und Fusionsprodukte selektioniert, die noch das eingeschleuste genetische Material enthalten und nach wie vor die erfindungswesentlichen Eigenschaften aufweisen.

43. Verfahren zur Herstellung von Varianten und Mutanten von transgenen Pflanzen, die gemäss einem Verfahren nach Anspruch 1 transformiert worden sind, dadurch gekennzeichnet, dass man besagte Pflanzen oder lebensfähige Teile davon sowie deren sexuelle und asexuelle Nachkommenschaft mit Hilfe bekannter Methoden mutiert und anschliessend diejenigen Individuen selektioniert, die noch das eingeschleuste genetische Material enthalten und die erfindugswesentlichen Eigenschaften aufweisen.

44. Verfahren zur Herstellung transgener Pflanzenteile aus Pflanzen, die gemäss einem Verfahren nach Anspruch 35 hergestellt worden sind, ausgewählt aus der Gruppe bestehend aus Blüten, Stengeln, Früchten, Blättern und Wurzeln, dadurch gekennzeichnet, dass man besagte Pflanzenteile isoliert und diejenigen selektioniert, die noch in einem Grossteil ihrer Zellen das eingeschleuste genetische Material enthalten.

45. Verfahren zum Schutz von Pflanzen vor chitinhaltigen Pathogenen, dadurch gekennzeichnet, dass man besagte Pflanzen mit einem gemäss Anspruch 15 hergestellten rekombinanten DNA Molekül transformiert und das eingeschleuste Chitinasegen in einer Menge exprimiert, die ausreicht, die Pathogene abzutöten oder aber diese unter Kontrolle zu halten.

46. Verfahren zum Schutz von Pflanzen vor chitinhaltigen Pathogenen, dadurch gekennzeichnet, dass man besagte Pflanzen mit einem gemäss Anspruch 15 hergestellten rekombinanten DNA Molekül transformiert und das eingeschleuste Glucanasegen in einer Menge exprimiert, die ausreicht, die Pathogene abzutöten oder aber diese unter Kontrolle zu halten.

47. Verfahren zur Bekämpfung chitinhaltiger Pathogene, dadurch gekennzeichnet, dass man besagte Pathogene mit einer transgenen Pflanze oder Teilen dieser Pflanze in Kontakt bringt, die mit einem gemäss Anspruch 15 hergestellten rekombinanten DNA Molekül transformiert sind und die das eingeschleuste Chitinase- bzw. Glucansegen in einer Menge exprimieren, die ausreicht, die Pathogene abzutöten oder aber diese unter Kontrolle zu halten.

48. Verfahren zur Herstellung eines basischen Chitinasegens das durch die folgenden Verfahrensmassnahmen charakterisiert ist:

(1) Extrahieren und Reinigen von genomischer DNA aus Geweben, die in der Lage sind Chitinase zu exprimieren;

(2) Zerlegen der extrahierten und gereinigten DNA Präparationen in Fragmente einer für die nachfolgende Insertion in einen Klonierungs-Vektor geeigneten Grösse;

(3) Einklonieren der fragmentierten DNA in einen Klonierungsvektor und Herstellen einer Genbibliothek;

(4) Auslesen von Klonen, die das Chitinasegen oder Teile davon enthalten mit Hilfe von Probenmolekülen;

(5) Isolierung derjenigen Klone, die mit dem Probenmolekül ein starkes Hybridisierungssignal zeigen;

(6) Charakterisierung der in (5) isolierten Klone mit Hilfe biochemischer Verfahren.

49. Verfahren gemäss Anspruch 48, dadurch gekennzeichnet, dass man für die Herstellung einer genomischen Genbibliothek Expressionsvektoren verwendet.

50. Verfahren gemäss Anspruch 48, dadurch gekennzeichnet, dass man als Probenmoleküleinen einen bekannten Chitinaseklon oder Teile davon verwendet.

51. Verfahren gemäss Anspruch 48, dadurch gekennzeichnet, dass man als Probenmoleküleinen ein synthetisch hergestelltes Oligonucleotid verwendet, das einem Teil eines bekannten Chitinasegens homolog ist.

52. Verfahren gemäss einem der Ansprüche 48 bis 51, dadurch gekennzeichnet, dass man die genomische Genbibliothek mit Hilfe der Plaquehybridisierung oder der differentiellen Koloniehybridisierung, unter Verwendung eines geeigneten Probenmoleküld oder mit Hilfe immunologischer Nachweismethoden, die auf einer Identifizierung der spezifischen Translationsprodukte beruhen, screent.

53. Verfahren gemäss Anspruch 50, dadurch gekennzeichnet, dass es sich bei besagtem cDNA Klon um pCHN48 bzw. pCHN50 oder um funktionelle Äquivalente davon handelt.

54. Verfahren zum Aufspüren neuer regulatorischer DNA Sequenzen, dadurch gekennzeichnet, dass man eine regulatorische DNA Sequenz oder Teilsequenzen davon gemäss einem der Ansprüche 1 bis 11 dazu verwendet um homologe DNA Sequenzen gleicher Funktion aufzuspüren, indem man zunächst genomische oder cDNA Genbibliotheken herstellt und diese unter Verwendung der erfindungsgemässen, regulatorischen DNA Sequenz als Probenmolekül, auf das Vorhandensein homologer DNA Sequenzen untersucht, die in der Lage sind mit dieser zu hybridisieren.

55. Verwendung der in Anspruch 1 charakterisierten regulatorischen DNA Sequenz zur Steigerung der Genexpression in pflanzlichem Material.

56. Verwendung der in Anspruch 1 charakterisierten regulatorischen DNA Sequenz zum Aufspüren homologer DNA Sequenzen gleicher Funktion, dadurch gekennzeichnet, dass man genomische Genbibliotheken herstellt und diese unter Verwendung besagter regulatorischer DNA Sequenz als Probenmolekül auf das Vorhandensein homologer DNA Sequenzen untersucht, die in der Lage sind mit dem Probenmolekül zu hybridisieren.

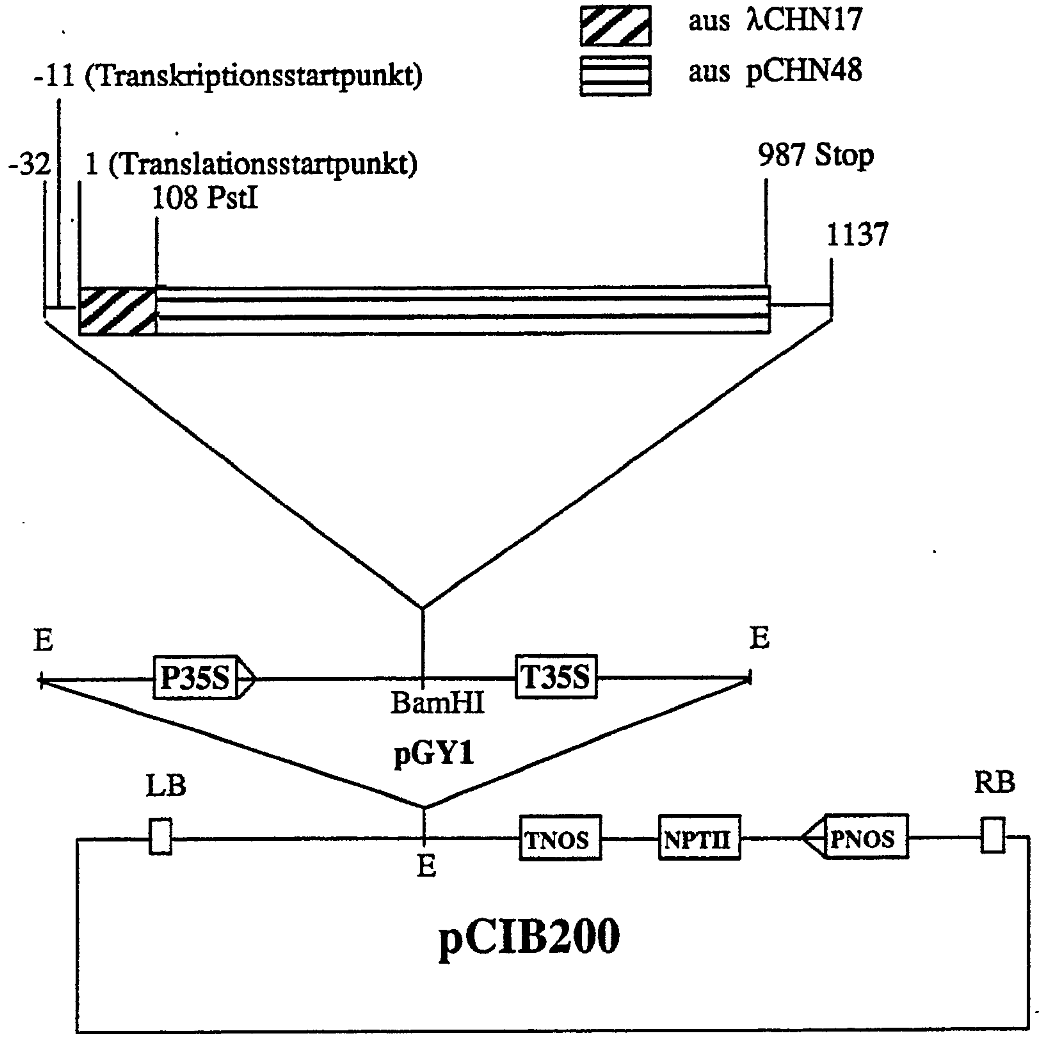


## 5'-Ende der relevanten Sequenz

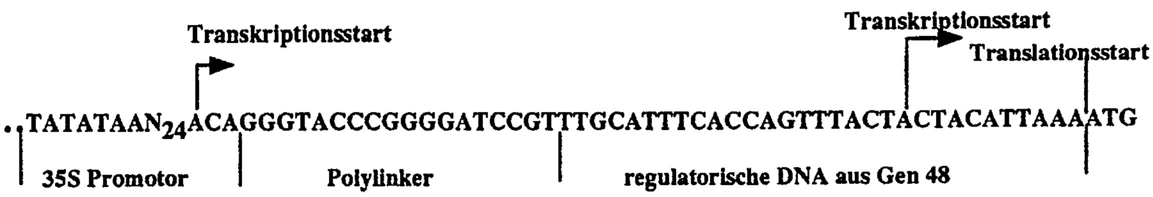


# FIG. 1 Genkarte von pSCH12

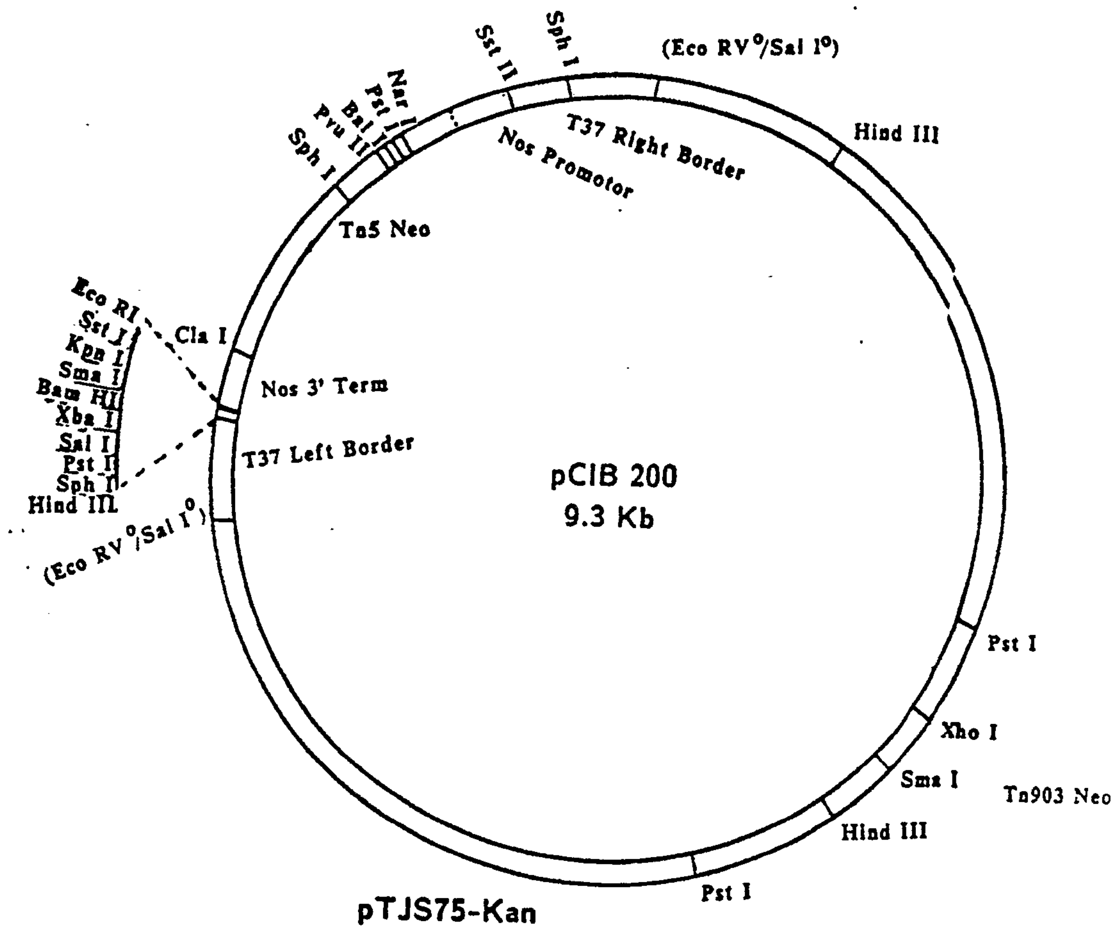


# FIG. 2 Restriktionskarte von pCIB200

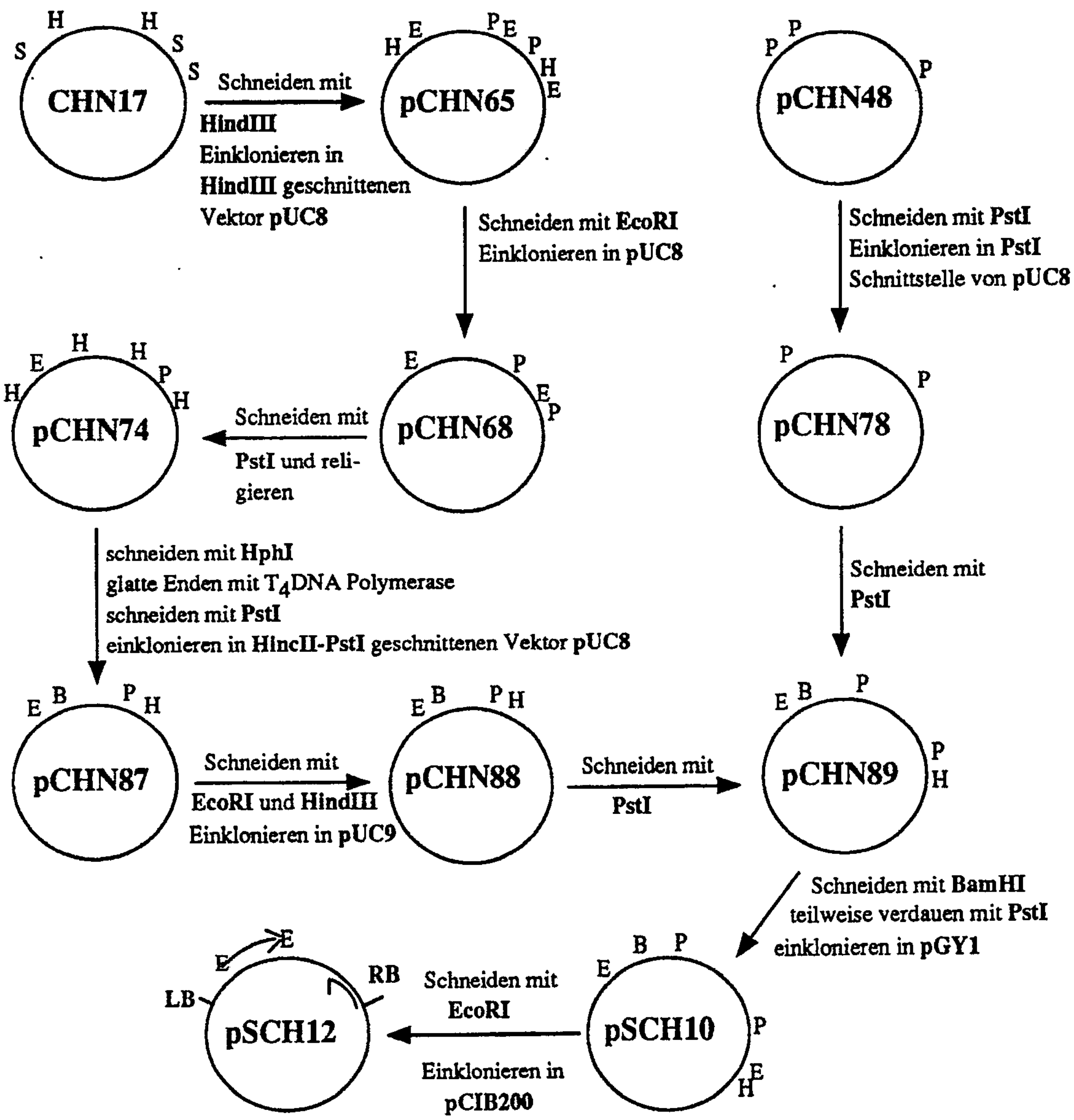


# FIG. 3 Konstruktion von pSCH12

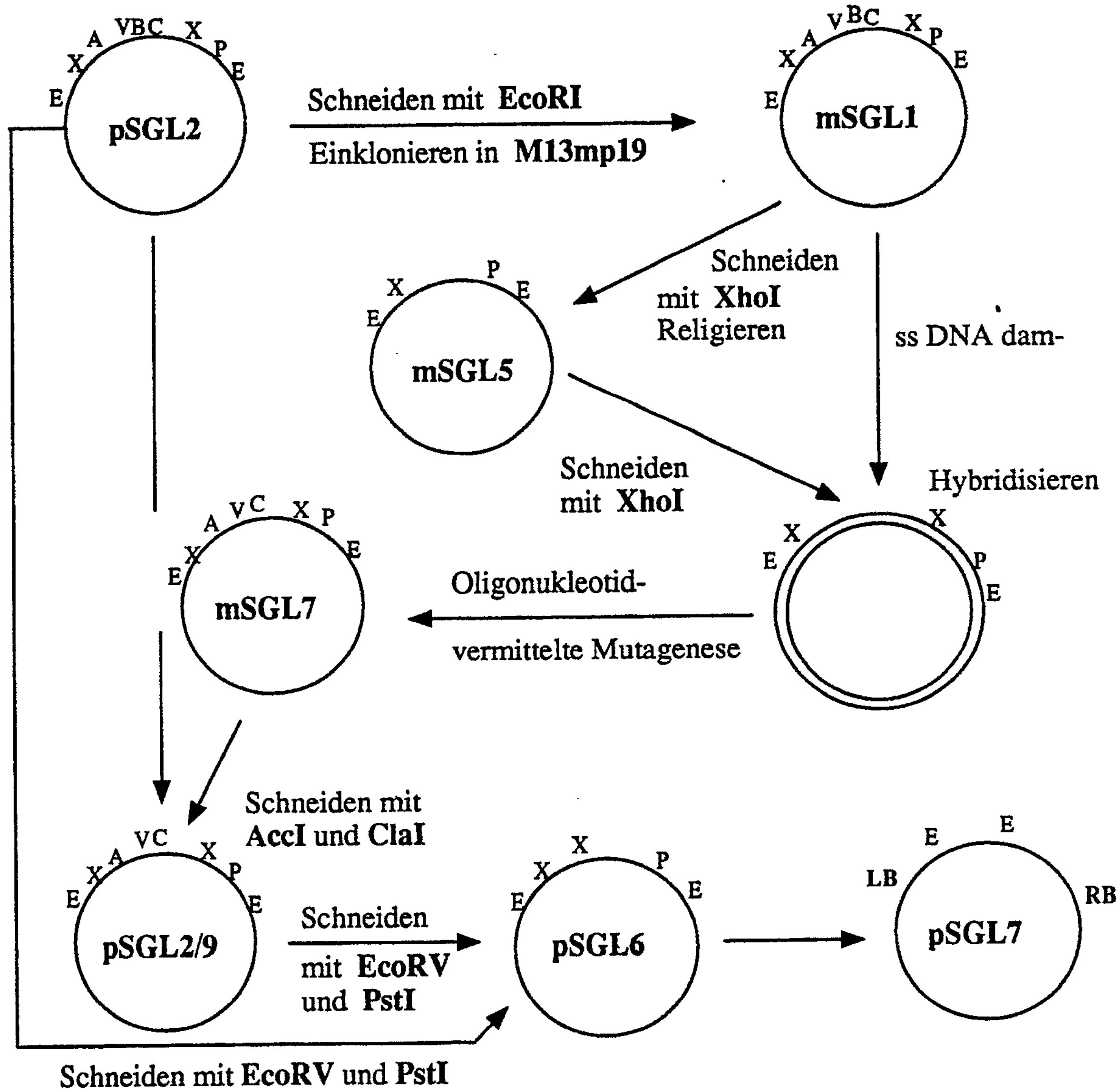


**FIG. 4 Konstruktion von pSGL7**

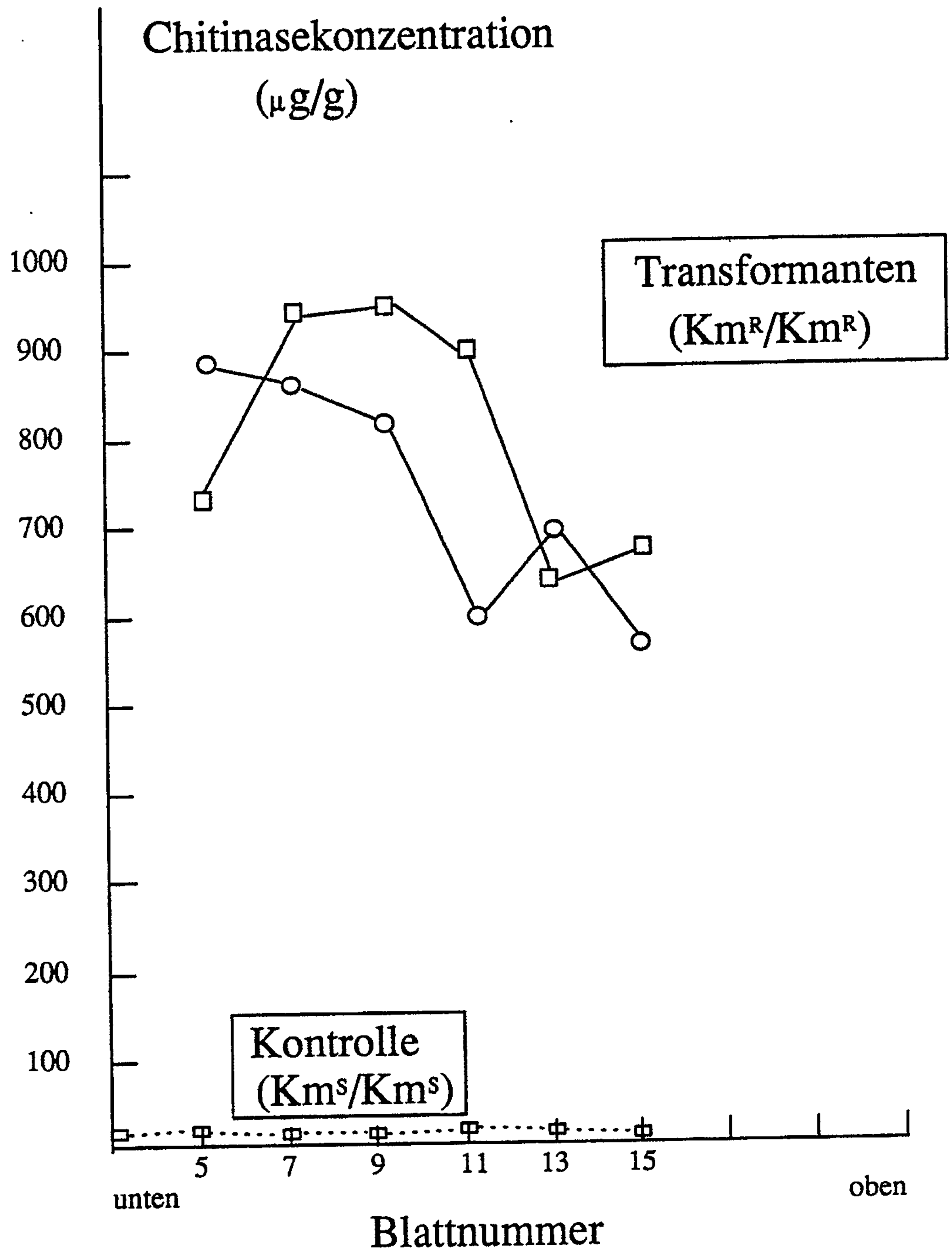


**FIG. 5 Chitinasekonzentration in den Blättern transformierter N. sylvestris Pflanzen**

Europäisches Patentamt

# EUROPÄISCHER RECHERCHENBERICHT

Nummer der Anmeldung

**EP 90 11 7389**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CELL, Band 57, 16. Juni 1989, Seiten 997-1007, Cell Press; H. OHKURA et al.: "The fission yeast dis2+ gene required for chromosome disjoining encodes one of two putative type 1 protein phosphatases" * Abbildung 3A, position -341 bis -328 * | 5,8 | C 12 N 15/82<br>C 12 N 15/56<br>C 12 N 1/21<br>C 12 N 15/10<br>A 01 H 5/00<br>C 12 Q 1/68<br>A 01 N 65/00 |
| X | WO-A-8 707 644 (DIATECH) * Seiten 8,39 * | 3,4,11,16, 20,32,36, 39 | |
| X | PLANT PHYSIOL, Band 85, 1987, Seiten 1103-1109; K.A. BARTON et al.: "Bacillus thuringiensis delta-endotoxin expressed in transgenic Nicotiana tabacum provides resistance to lepidopteran insects" * Seite 1103, rechte Spalte, Zeilen 30-50; Abbildung 1 * | 3,4,13,16, 20,25,29, 31-41,44, 45-47 | |
| X | THE PLANT CELL, Band 1, Nr. 6, Juni 1989, Seiten 599-607, American Society of Plant Physiologists; K.E. BROGLIE et al.: "Functional analysis of DNA sequences responsible for ethylene regulation of a bean chitinase gene in transgenic tobacco" * Ganzes Dokument * | 1,11,26, 30-47,48, 50-52,54, 60,62,68, 69,70 | **RECHERCHIERTE SACHGEBIETE (Int. Cl.5)** |
| X | IDEM | 73 | C 12 N<br>A 01 H<br>A 01 N |
| X | MOL. GEN. GENET., Band 212, 1988, Seiten 536-542, Springer-Verlag; J.D.G. JONES et al.: "Expression of bacterial chitinase protein in tobacco leaves using two photosynthetic gene promoters" * Ganzes Dokument, besonders Seite 541, letzter Absatz - Seite 542, linke Spalte * | 41,42 | |

-/-

Der vorliegende Recherchenbericht wurde für alle Patentansprüche erstellt

| Recherchenort | Abschlußdatum der Recherche | Prüfer |
|---|---|---|
| Den Haag | 11 Dezember 90 | MADDOX A.D. |

KATEGORIE DER GENANNTEN DOKUMENTE
- X: von besonderer Bedeutung allein betrachtet
- Y: von besonderer Bedeutung in Verbindung mit einer anderen Veröffentlichung derselben Kategorie
- A: technologischer Hintergrund
- O: nichtschriftliche Offenbarung
- P: Zwischenliteratur
- T: der Erfindung zugrunde liegende Theorien oder Grundsätze
- E: älteres Patentdokument, das jedoch erst am oder nach dem Anmeldedatum veröffentlicht worden ist
- D: in der Anmeldung angeführtes Dokument
- L: aus anderen Gründen angeführtes Dokument
- &: Mitglied der gleichen Patentfamilie, übereinstimmendes Dokument

Europäisches Patentamt

# EUROPÄISCHER RECHERCHENBERICHT

Nummer der Anmeldung

EP 90 11 7389

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | IDEM | 63,65 | |
| X | EP-A-0 270 248 (ICI)<br>* Seite 3, Zeilen 4-6; Ansprüche 9,10 * | 43 | |
| A<br>P,X | PLANT MOL. BIOL., Band 14, 1990, Seiten 357-368, Kluwer Academic Publishers, BE; H. SHINSHI et al.: "Structure of a tobacco endochitinase gene: evidence that different chitinase genes can arise by transposition of sequences encoding a cysteine-rich domain"<br>* Ganzes Dokument * | 64,66<br>1-10 | |
| P,X | EP-A-0 337 532 (MOGEN)<br>* Abbildung 1B; Seiten 610-597; Spalten 8-9 * | 8,11, 30-47 | |
| Y | EP-A-0 292 435 (CIBA-GEIGY)<br>* Seiten 36-37; Beispiel 16 * | 1-10,48, 49,68,69, 70,71 | |
| Y | PROC. NATL. ACAD. SCI. USA, Band 84, Januar 1987, Seiten 89-93; H. SHINSHI et al.: "Regulation of a plant pathogenesis-related enzyme: Inhibition of chitinase and chitinase mRNA accumulation in cultured tobacco tissues by auxin and cytokinin"<br>* Ganzes Dokument * | 1-10,48, 49,68,69, 70,71 | RECHERCHIERTE SACHGEBIETE (Int. Cl.5) |
| O,A | EXPERIENTIA, Band 43, Nr. 6, 1987, Seite 662, Birkhäuser Verlag, Basel, CH; J.-M. NEUHAUS et al.: "Characterization of tobacco chitinase genes"<br>* Ganze Zusammenfassung *<br>-/- | 1-10 | |

Der vorliegende Recherchenbericht wurde für alle Patentansprüche erstellt

| Recherchenort | Abschlußdatum der Recherche | Prüfer |
|---|---|---|
| Den Haag | 11 Dezember 90 | MADDOX A.D. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument

Europäisches Patentamt

# EUROPÄISCHER RECHERCHENBERICHT

Nummer der Anmeldung

**EP 90 11 7389**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | WO-A-8 703 619 (MIT)<br>* Patentansprüche *<br>– – – | 3,4 | |
| E | EP-A-0 392 225 (CIBA-GEIGY)<br>* Beispiel 33 *<br>– – – – – | 1-8,<br>11-16,<br>19-21,<br>29-47 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| Den Haag | 11 Dezember 90 | MADDOX A.D. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument